# EUROPEAN PATENT APPLICATION

(11) **EP 4 707 304 A1**
(43) Date of publication of application: **11.03.2026**
(21) Application number: 24799882.6
(22) Date of filing: 29.04.2024
(51) Int. Cl.: C07K 16/30, C12N 15/13, A61K 39/395, A61P 35/00

(54) **ANTIGEN-BINDING PROTEIN FOR HCLDN6 AND USE THEREOF**

(30) Priority: 30.04.2023 CN 202310491221; 05.09.2023 CN 202311138622; 07.02.2024 CN 202410173800
(71) Applicant: Axcynsis Therapeutics Pte. Ltd., Singapore 117610 (SG)
(72) Inventor: ZOU, Bin, Shanghai 200131 (CN); ZHONG, Chen, Shanghai 200131 (CN); LIU, Shaojun, Shanghai 200131 (CN); DU, Ping, Shanghai 200131 (CN)
(74) Representative: J A Kemp LLP
(86) International application number: PCT/CN2024/090568
(87) International publication number: WO 2024/227424

(57) **Abstract**

Provided are an antigen-binding protein for hCLDN6 and the use thereof. Said antigen-binding protein comprises a heavy chain variable region. The heavy chain variable region has HCDR1 having an amino acid sequence of X1YTMS; HCDR2 having an amino acid sequence of TISSGGGX2TYYPDSVKG in which X2 = R, N, Q, D or E; and HCDR3 having an amino acid sequence of GDX4RYDX3FAY in which X4 = Y, N or Q, wherein (1) X1 = Y, M, Q, D or E, and X3 = G; or (2) X1 = S or D, and X3 = A. Provided are novel humanized antibodies targeting CLDN6, and different compositions based on these antibodies, which have better cell proliferation inhibitory activity and tumor inhibitory activity, thus providing novel means for cancer diagnosis and treatment.

## Description

### TECHNICAL FIELD

The present invention relates to an antigen-binding protein for hCLDN6, a nucleic acid molecule encoding the antigen-binding protein, a vector, a cell, a pharmaceutical molecule, and uses thereof.

### BACKGROUND ART

The claudin (CLDN) family comprises 27 members, all of which are multi-transmembrane proteins. Some of these members participate in the formation of tight junctions. Tight junctions are one of the important connection forms of cell adhesion structures. CLDN proteins, together with other extracellular and peripheral membrane proteins, form barriers between adjacent polarized epithelial cells or endothelial cells, preventing the transport of large molecules. CLDN proteins also bind to signal proteins and cytoskeletal proteins, participating in cell signaling. Among the members involved in tight junctions, CLDN6 is unique in that it is only expressed in embryos and is barely expressed in normal adult tissues. CLDN6, on the other hand, is a tumor antigen specifically expressed in various cancers, such as ovarian cancer, testicular cancer, non-small cell lung cancer, endometrial cancer, liver cancer, pancreatic cancer, or choriocarcinoma. CLDN6 may also participate in stem cell differentiation and is associated with proliferation, apoptosis, migration and metastasis, and drug resistance of cancer cells. Inhibition of CLDN6 slows tumor growth in mouse models.

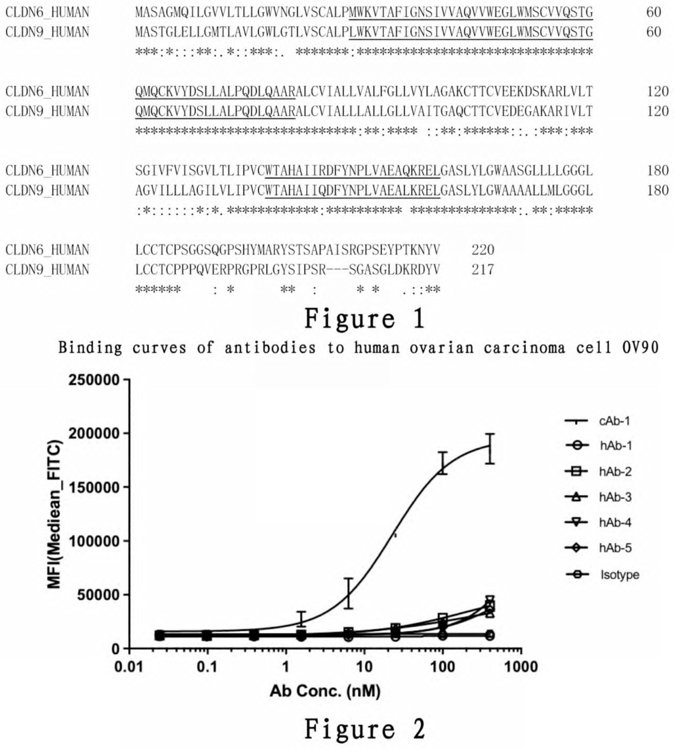

Other members of the CLDN6 family are expressed to a certain extent in normal tissues. Therefore, antibodies with high selectivity for CLDN6 can improve the safety of the antibodies and compositions thereof. CLDN9 has the highest similarity to CLDN6, differing in sequence at only three amino acid residue sites in the extracellular region (as shown in Figure 1, the extracellular loop regions of human CLDN6 and human CLDN9 are underlined. Two extracellular loop regions, EL1 and EL2 are comprised. EL1 spans 29-81 AA, and EL2 spans 138-160 AA, respectively, for human CLDN6 and CLDN9). CLDN9 is expressed in tissues such as the pituitary gland, nasal mucosa, and inner ear, and mutations in CLDN9 are associated with hereditary deafness. Therefore, antibodies with high selectivity for CLDN6, recognizing CLDN6 but not binding or weakly binding to CLDN9 or other CLDN family members, can improve the safety of the antibodies and compositions thereof.

Furthermore, murine antibodies have high immunogenicity, and humanization can reduce the immunogenicity of murine antibodies, making them more suitable for clinical applications. However, humanization is complex, and humanized antibodies obtained after engineering often exhibit reduced affinity, expression, and/or stability. Currently, there is no method that guarantees that a specific method will yield high-affinity humanized antibodies after humanizing murine antibodies.

### SUMMARY OF THE INVENTION

The main purpose of the application is to provide an antigen-binding protein that can specifically bind to CLDN6 and has good antigen-binding activity.

The first aspect of the present invention relates to an isolated antigen-binding protein capable of binding to hCLDN6, comprising a heavy chain variable region, wherein the heavy chain variable region has:
HCDR1 with an amino acid sequence of X₁YTMS,
HCDR2 with an amino acid sequence of TISSGGGX₂TYYPDSVKG, X₂=R, N, Q, D or E,
HCDR3 with an amino acid sequence of GDX₄RYDX₃FAY, X₄=Y, N or Q,
wherein,
   (1) X₁=Y, M, Q, D or E, X₃=G, or (2) X₁=S or D, X₃=A.

In some embodiments of the first aspect of the present invention, the heavy chain variable region has a human heavy chain framework region, wherein the human heavy chain framework region has back mutation(s) at one or more sites relative to a murine heavy chain framework region. The back mutation sites in the human heavy chain variable region include R44, and the position numbering is determined by the Kabat numbering scheme.

In some embodiments of the first aspect of the present invention, the heavy chain variable region: has 92.59% or more identity with HFR1 to HCDR3; has said back mutation sites; and has a sequence comprising said X₁, X₂, X₃ and/or X₄ sites.

In some embodiments of the first aspect of the present invention, the heavy chain variable region has a sequence shown in any one of SEQ ID Nos. 22, 23, 24, 59, 60, 61, 62, 63, 66, 67, 133 to 153, or has a sequence having 92.59% or more identity with HFR1 to HCDR3 of said heavy chain variable region.

In some embodiments of the first aspect of the present invention, the heavy chain framework region is a human IgG heavy chain framework region.

In some embodiments of the first aspect of the present invention, the heavy chain variable region has a murine heavy chain framework region.

In some embodiments of the first aspect of the present invention, the heavy chain variable region has the sequence shown in any one of SEQ ID Nos. 29, 30 or 45.

In some embodiments of the first aspect of the present invention, further comprises a light chain variable region, wherein the light chain variable region has the amino acid sequence shown in SEQ ID NO. 5.

In some embodiments of the first aspect of the present invention, further comprises a light chain variable region, wherein the light chain variable region has:
LCDR1 with an amino acid sequence of RASENIDSX₅LA, X₅=Y, Q, or R,
LCDR2 with an amino acid sequence of ASTLLVD,
LCDR3 with an amino acid sequence of QHYYSX₆PYT, X₆=I or E.

In some embodiments of the first aspect of the present invention, the light chain variable region has a human light chain framework region, and the light chain framework region has back mutation(s) at one or more sites relative to the murine light chain framework region, wherein the back mutation sites in the light chain framework region include one or more of R66, Q70, and R85, and the position numbering is determined by the Kabat numbering scheme.

In some embodiments of the first aspect of the present invention, the sequence having 95.88% or more identity with HFR1 to HCDR3 of the light chain variable region has said back mutation site and has said X₅ and/or X₆ site.

In some embodiments of the first aspect of the present invention, the light chain variable region has a sequence shown in any one of SEQ ID Nos. 25, 28, 154, 155, and 156, or has a sequence having 95.88% or more identity with HFR1 to HCDR3 of said light chain variable region.

In some embodiments of the first aspect of the present invention, the light chain framework region is a human IgG light chain framework region.

In some embodiments of the first aspect of the present invention, comprises a human heavy chain constant region and a human light chain constant region.

In some embodiments of the first aspect of the present invention, comprises a human heavy chain constant region.

In some embodiments of the first aspect of the present invention, the constant region contains a mutation at one or more sites.

In some embodiments of the first aspect of the present invention, the heavy chain constant region contains a LALA mutation.

In some embodiments of the first aspect of the present invention, the heavy chain constant region is a human IgG1 heavy chain constant region, and comprises one or more mutations selected from the group consisting of C220S, A121C, V205C, K149C, D265C, S239C, A330C, and S442C.

In some embodiments of the first aspect of the present invention, the amino acid sequence of the heavy chain constant region is as shown in SEQ ID No. 9.

In some embodiments of the first aspect of the present invention, the amino acid sequence of the light chain constant region is as shown in SEQ ID No. 10.

In some embodiments of the first aspect of the present invention, the CDR sequences are determined according to the Kabat definition scheme.

In some embodiments of the first aspect of the present invention, the antigen-binding protein comprises an antibody or an antigen binding fragment thereof.

In some embodiments of the first aspect of the present invention, the antibody is selected from a monoclonal antibody, a polyclonal antibody, a chimeric antibody, a humanized antibody or a full human antibody.

In some embodiments of the first aspect of the present invention, the antigen-binding fragment is selected from a Fab antibody, a single-chain antibody or a single-domain antibody.

In some embodiments of the first aspect of the present invention, the antigen-binding protein has a heavy chain with a sequence as shown in any one of SEQ ID Nos. 157, 159 to 167, or has a sequence having 92.59% or more identity with HFR1 to HCDR3 of said heavy chain.

In some embodiments of the first aspect of the present invention, the antigen-binding protein has a heavy chain with a sequence as shown in any one of SEQ ID Nos. 158, 171, or has a sequence having 92.59% or more identity with HFR1 to HCDR3 of said heavy chain.

In some embodiments of the first aspect of the present invention, the antigen-binding protein has a light chain with a sequence as shown in any one of SEQ ID Nos. 169, 170, or has a sequence having 95.88% or more identity with HFR1 to HCDR3 of said light chain.

In some embodiments of the first aspect of the present invention, the antigen-binding protein has a light chain with a sequence as shown in SEQ ID No. 168, or has a sequence having 95.88% or more identity with HFR1 to HCDR3 of said light chain.

In some embodiments of the first aspect of the present invention, the antigen-binding protein is antibody hAb-13: with the heavy chain variable region sequence as shown in SEQ ID No. 18 and the light chain variable region as shown in SEQ ID No. 28.

The second aspect of the present invention relates to a nucleic acid molecule, characterized in that the nucleic acid molecule is used to encode any of the antigen-binding proteins described above.

The third aspect of the present invention relates to a vector, characterized in that the vector comprises the nucleic acid molecule.

The fourth aspect of the present invention relates to a cell comprising the nucleic acid molecule, and/or the vector.

The fifth aspect of the present invention relates to a pharmaceutical molecule, characterized by comprising any one of the above-described antigen-binding proteins.

In some embodiments of the fifth aspect of the present invention, the pharmaceutical molecule further comprises a therapeutic agent or a detectable marker linked to the antigen-binding protein.

In some embodiments of the fifth aspect of the present invention, the therapeutic agent is selected from a cytotoxic agent, a cytostatic agent, a radioisotope, an anti-angiogenic agent or a liposome.

In some embodiments of the fifth aspect of the present invention, the therapeutic agent is an immunomodulator.

In some embodiments of the fifth aspect of the present invention, the cytotoxic agent is a molecule derived from a plant, fungus or bacterium, or a derivative thereof.

In some embodiments of the fifth aspect of the present invention, the cytotoxic agent is selected from a peptide toxin, a protein toxin, or an alkylating toxin.

In some embodiments of the fifth aspect of the present invention, the cytotoxic agent is selected from one or more of maytansine alkaloids, auristatins, eribulin, taxane, calicheamicin, cemadotin, pyrrolobenzodiazepine, anthracyclines, camptothecin derivatives, α - amanitin and derivatives thereof, trabectedin and derivatives thereof, and lurbinectidin and derivatives thereof.

In some embodiments of the fifth aspect of the present invention, the therapeutic agent or the detectable marker is conjugated to the antigen-binding protein via a cleavable or a non-cleavable linker.

In some embodiments of the fifth aspect of the present invention, the linker has one or more connectors.

In some embodiments of the fifth aspect of the present invention, the connector is selected from one or more of an oligopeptide connector, a hydrazine connector, a thiourea connector, a triggered self-immolative connector, a succinimidyl trans-4-(maleimidomethyl) cyclohexane-1-carboxylate connector, a maleimide connector, a disulphide connector, a thioether connector, and an olefin connector.

In some embodiments of the fifth aspect of the present invention, the structure is as shown in Formula I:

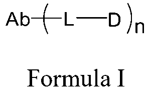

Ab is the antigen-binding protein, L is a linker comprising one or more connectors, D is a therapeutic agent or a detectable marker, and n is an integer selected from 1-20.

In some embodiments of the fifth aspect of the present invention, n is an integer selected from 2-8.

The sixth aspect of the present invention relates to a pharmaceutical composition, characterized in that it comprises any of the antigen-binding proteins, the nucleic acid molecules, the vectors, the cells described above, and/or any of the pharmaceutical molecules described above, as well as pharmaceutically acceptable carriers and/or additives.

The seventh aspect of the present invention relates to a use of any of the antigen-binding proteins, the nucleic acid molecules, the vectors, the cells, and the pharmaceutical molecules described above in the manufacture of a medicament for diagnosing, preventing and/or treating hCLDN6-related diseases and/or disorders.

In some embodiments of the seventh aspect of the present invention, the disease and/or disorder comprises a tumor.

In some embodiments of the seventh aspect of the present invention, the tumor is ovarian cancer, testicular cancer, non-small cell lung cancer, liver cancer, pancreatic cancer, choriocarcinoma or endometrial cancer.

The present invention has obtained highly selective humanized anti-CLDN6 antibodies with comparable or higher affinity than murine parental antibodies. Compositions such as ADC conjugates based on these antibodies have also been prepared, by combining with different linkers and toxins. These antibodies and compositions provide new approaches for the diagnosis and treatment of tumors. In particular, anti-CLDN6 ADCs exhibit excellent anti-tumor activity and have potential for tumor treatment. The invention provides novel humanized antibodies targeting CLDN6, as well as various compositions based on these antibodies, which have better cell proliferation inhibitory activity and tumor inhibitory activity, providing a new means for cancer diagnosis and treatment.

### BRIEF DESCRIPTION OF THE DRAWINGS

Figure 1 shows the sequence alignment of human CLDN6 (hCLDN6) and human CLDN9 (hCLDN9). The extracellular EL1 and EL2 sequences are underlined.
Figure 2 shows the binding activity of an anti-CLDN6 murine/human chimeric monoclonal antibody and CDR-implanted humanized antibodies to human ovarian cancer cells OV90.
Figure 3 shows sequence alignments of the heavy chain variable regions (A) and light chain variable regions (B) of a chimeric antibody, a CDR-implanted humanized antibody, and a representative humanized antibody with back mutations introduced after CDR implantation.
Figure 4 shows the endocytic activity of selected humanized antibodies with back mutations introduced after CDR implantation on JEG-3 cells.
Figure 5 shows the binding activity of cAbs-2~31 to VLP-hCLDN6 (A-B) and the endocytic activity on JEG -3 cells (C).
Figure 6 shows the binding activity detection of hAbs-26~49 with VLP-hCLDN6 (A-C, E) or VLP-hCLDN9 (D, F).
Figure 7 schematically illustrates a process for producing antibody-drug conjugates (ADCs) using antibody interchain disulfide bonds and/or cysteine mutation sites.
Figure 8 shows the in vitro cytotoxicity of selected ADCs against human ovarian cancer cell OV90.
Figure 9 shows the in vitro cytotoxicity of selected ADCs against human choriocarcinoma cell JEG3.
Figure 10 shows the efficacy of ADC-2, DAR2 (A) and ADC-46, DAR4 (B) in a xenograft model in female BALB/C nude mice established with hCLDN6-positive OV90 cells.

### DETAILED DESCRIPTION

Unless otherwise defined, all technical and scientific terms used herein have the same meaning as commonly understood by one of ordinary skill in the art. In the event of a conflict, the descriptions and definitions herein shall prevail.

In view of the problems existing in the prior art, the present invention modifies and humanizes murine antibodies to obtain antigen-binding proteins with higher hCLDN6 binding activity. The heavy chain variable region of the antigen-binding protein involved in the present invention has:
HCDR1 with an amino acid sequence of X₁YTMS,
HCDR2 with an amino acid sequence of TISSGGGX₂TYYPDSVKG, X₂=R, N, Q, D or E,
HCDR3 with an amino acid sequence of GDX₄RYDX₃FAY, X₄=Y, N or Q,
wherein,
   (1) X₁=Y, M, Q, D or E, X₃=G, or (2) X₁=S or D, X₃=A.

The above-mentioned heavy chain variable region of the present invention relates to specific mutations at one or more sites such as S31 and G100 in the HCDR region shown in SEQ ID No. 2 to 4. The antigen-binding protein obtained through mutations has improved binding activity to hCLDN6.

Types of the aforementioned antigen-binding proteins include, but are not limited to, full-length antibodies, antibody fragments, immunoconjugates, antibody analogues, antibody derivatives, and fusion proteins. Antibodies include, but are not limited to, chimeric antibodies and humanized antibodies. Antibody fragments include, but are not limited to, Fab, Fab', F(ab)2, Fv, scFv, dsFv, VHH, and the like.

Chimeric antibodies generally refer to antibodies that have the V region (variable region) of a mouse monoclonal antibody and the C region (constant region) of a human immunoglobulin.

Humanized antibodies generally refer to antibodies formed by introducing the CDR sequences of mouse antibodies into the framework sequences of human VH and VL domains, respectively.

The chimeric antibodies and the humanized antibodies can be produced using known methods. For example, a V region gene consisting of three CDRs concatenated with four FRs can be amplified in its entirety by annealing at the 5' and 3' ends and have primers of appropriate restriction enzyme recognition sequences attached. The DNA obtained as described above is assembled into an expression vector with DNA encoding a human antibody C region for in-frame fusion. This recombinant vector is introduced into a host to establish recombinant cells, which are then cultured to express the DNA encoding the antibody to produce the antibody. For details, see Example 2.

The antibody forms include, but are not limited to, IgG, IgM, IgA, IgE, and IgD, with IgG being preferred. Furthermore, the IgG includes IgG1, IgG2, IgG3, IgG4, etc. The constant regions of the chimeric antibodies and the humanized antibodies may include Cγ1, Cγ2, Cγ3, Cγ4, Cµ, Cδ, Cα1, Cα2, Cε, and the like for the heavy chain, and Cκ, Cλ, and the like for the light chain. LALA mutations may be introduced into the Fc region of the antibody to reduce binding of the antibody to Fcy receptors and complement. Mutations in the heavy chain constant region include, but are not limited to, one or more of C220S, A121C, V205C, K149C, D265C, S239C, A330C, and S442C.

In the present invention, CDR is used to represent the complementarity determining region (or hypervariable loop) of an antibody. The amino acid sequence within the CDR determines the chemical structure and properties of the antibody's antigen-binding site. The CDRs in the heavy chain variable region (VH) are denoted as HCDRs, with the three heavy chain CDR regions designated HCDR1, HCDR2, and HCDR3, respectively. The CDRs in the light chain variable region (VL) are denoted as LCDRs, with the three light chain CDR regions designated LCDR1, LCDR2, and LCDR3, respectively. It should be noted that CDR regions in antibodies are generally predicted and identified experimentally. Unless otherwise specified, the CDR sequences in the present invention are defined according to the Kabat definition scheme, and the associated amino acid numbering is determined by the Kabat numbering scheme. It should be understood that when different CDR definition schemes are used, the predicted CDR region sequences may only partially overlap, or may be shortened or lengthened. During antibody production, the CDR and FR gene sequences defined according to different definition schemes can be concatenated and amplified to full length using known methods to further produce antibodies or antibody fragments.

The antibody fragment Fab refers to the fragment consisting of the VH region of the heavy chain variable region, the first constant region CH1 functional region of the heavy chain, and the entire light chain connected by disulfide bonds, which mainly plays the antigen binding function of the antibody.

scFv is single-chain antibody. Specifically, it involves linking the light and heavy chain variable region genes of an antibody to a suitable oligonucleotide chain (linker) at the DNA level, so that it is expressed in the form of a single peptide chain in a suitable organism and folded into a new antibody consisting only of the heavy chain and light chain variable regions. The linker is not particularly limited. For example, any single-chain peptide comprising approximately 3 to 25 residues can be used as the linker.

dsFv is a disulfide-stabilized antibody. Specifically, it involves an antibody formed by mutating an amino acid residue in the heavy chain variable region (VH) and the light chain variable region (VL) of the antibody to cysteine, and then linking VH and VL through interchain disulfide bonds.

VHH is a heavy chain single-domain antibody, Specifically, it involves an antibody comprising only the VH functional domain but not the VL functional domain.

Bispecific antibodies refer to antibodies that have the binding properties for two antigens.

Fusion proteins refer to products created by fusing antibody molecule fragments to other proteins (such as antibodies, enzymes, immunotoxins, immunocytokines, immunoadhesins, etc.) using genetic engineering technology.

Methods for preparing the various antibody fragments described above are known. One method involves enzymatic treatment of antibodies to produce antibody fragments. Enzymes used to produce antibody fragments include, but are not limited to, papain, pepsin, or plasmin. Another method involves constructing a gene encoding the abovementioned antibody fragment, introducing the gene into an expression vector, and expressing the gene in an appropriate host cell to produce the antibody fragment.

In the present invention, CH is used to represent the heavy chain constant region of an antibody, and CL is used to represent the light chain constant region of an antibody. The heavy chain constant region generally further includes the CH1, CH2, and CH3 domains. FR is used to represent the framework of an antibody variable region, wherein the framework region of the heavy chain variable region is represented by HFR, and the four heavy chain framework regions are named HFR1, HFR2, HFR3, and HFR4, respectively; the framework region of the light chain variable region is represented by LFR, and the four light chain framework regions are named LFR1, LFR2, LFR3, and LFR4, respectively.

The antibody derivatives in the present invention refer to products obtained by substituting, mutating, modifying, replacing, deleting and/or adding one or more amino acid residues in the antibody while retaining the endogenous function.

In the present invention, the binding activity of the antigen-binding protein is detected by existing methods, such as ELISA and flow cytometry, and can be detected by referring to the methods of Example 23 and Comparative Example 7.

The present invention employs existing methods for detecting the endocytic activity of the prepared antigen-binding proteins. For example, a test antigen-binding protein comprising a detectable marker is co-incubated with cells expressing CLDN6, and the fluorescence intensity excited by the protein that has entered the cells is detected by flow cytometry to analyze the endocytic activity. See Examples 24 for details.

The framework regions of the heavy chain variable regions can be derived from murine or human framework regions. The human heavy chain framework region has a back mutation at one or more sites relative to a murine heavy chain framework region. Preferred back mutation sites include R44, and the position numbering is determined by the Kabat numbering scheme.

In the antigen-binding protein, other heavy chain variable region sequences having 92.59% or more identity with HFR1 to HCDR3 of the heavy chain variable region described above, having said back mutation sites, and having said X₁, X₂, X₃ and/or X₄ sites can also be selected.

Preferably, the above antigen-binding protein further comprises a light chain variable region, wherein the light chain variable region has:
LCDR1 with an amino acid sequence of RASENIDSX₅LA, X₅=Y, Q, or R,
LCDR2 with an amino acid sequence of ASTLLVD,
LCDR3 with an amino acid sequence of QHYYSX₆PYT, X₆=I or E.

The framework regions of the light chain variable region can be derived from murine or human framework regions, and can be combined with the above corresponding heavy chain variable regions to form various types of antigen-binding proteins. The human light chain framework region has back mutation(s) at one or more sites relative to a murine light chain framework region. Preferred back mutation sites include one or more of R66, Q70, and R85, and the position numbering is determined by the Kabat numbering scheme.

In the antigen-binding protein, other light chain variable region sequences having sequences with 95.88% or more identity with HFR1 to HCDR3 of the above-mentioned light chain variable region can also be selected.

The vectors of the present invention are used for replication, transcription and/or translation of DNA or RNA. By cultivating cells comprising the vectors, desired expression products can be produced. The selected vectors are known, such as plasmids.

Furthermore, the present invention also relates to pharmaceutical molecules comprising the aforementioned antigen-binding proteins. The pharmaceutical molecules may comprise any known pharmaceutical form, including but not limited to proteins, peptides, small molecule drugs, antibody-drug conjugates, or combinations of various pharmaceutical forms.

An antibody-drug conjugate refers to an antigen-binding protein (usually an antibody or antibody fragment) conjugated to a therapeutic agent via a linker. Its structure can be shown in Formula I:

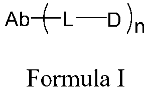

Ab is the antigen-binding protein, L is a linker comprising one or more connectors, D is a therapeutic agent or a detectable marker, and n is an integer selected from 1-20, and further, can be an integer selected from 2-8.

The connection between the linker and the drug is typically produced through known chemical synthesis methods to form a linker-therapeutic agent structure capable of conjugating to the antibody. During the preparation of the antibody-drug conjugate, the antibody is conjugated to one or more linker-therapeutic agent structures. The conjugation between the antibody and the linker-therapeutic agent structure is typically established at the side chains of lysine or cysteine residues in the antibody. For lysine conjugation, the linker typically contains an N-hydroxysuccinimide group that reacts with the ε-amino group of the lysine to form a stable amide bond, thereby forming a conjugate of the antibody and the therapeutic agent. For cysteine conjugation, the linker typically contains a functional group that can mediate the conjugation reaction, such as a maleimide group or allenamide. The cysteine is subjected to disulfide reduction using known methods to generate a reactive thiol, which is then conjugated to the maleimide group to form a conjugate of the antibody and the therapeutic agent. For example, reference may be made to the methods exemplified in Examples 92~163 of the present invention.

Applicable linkers include non-cleavable and cleavable ones. The linker may have one or more connectors, including but not limited to an oligopeptide connector (including cleavable and/or non-cleavable oligopeptide connector), a hydrazine connector, a thiourea connector, a triggered self-immolative connector, a succinimidyl trans-4-(maleimidomethyl) cyclohexane-1-carboxylate (SMCC) connector, a maleimide connector, a disulfide connector, a thioether connector and/or a olefin connector.

Applicable therapeutic agents can be cytotoxic agents, cytostatic agents, etc. The cytotoxic agent can be a molecule from a plant, fungus or bacterium, or a derivative thereof, including but not limited to a peptide toxin, a protein toxin, an alkylating toxin or a toxin of other types. Pharmacutical toxic agent can be selected from one or more of maytansine alkaloids (for example, maytansinol or DM1 maytansine), auristatins (for example, monomethyl auristatin E or monomethyl auristatin F), eribulin, taxane, calicheamicin, cemadotin, pyrrolobenzodiazepine, anthracyclines, camptothecin derivatives, α-amanitin and derivatives thereof, trabectedin and derivatives thereof, and lurbinectidin and derivatives thereof. The cytotoxic substance used in the present invention can be one, or two or more cytotoxic substances can be combined for use.

Applicable therapeutic agents can also be metabolites (e.g., an antifolate such as methotrexate, a fluoropyrimidine such as 5-fluorouracil, cytarabine, or a purine or adenosine analogue); an intercalating agent (e.g., an anthracycline such as doxorubicin, nemorubicin, or preferably a derivative of PNU-159682), daunorubicin, epirubicin, idarubicin, mitomycin-C, actinomycin D, or mithramycin, or other intercalating agents such as pyrrolobenzodiazepine; DNA-reactive agents such as calicheamicin, tiancimycins, and other enediynes; a platinum derivative (e.g., cisplatin or carboplatin); an alkylating agent (e.g., chlormethine, melphalan, chlorambucil, busulfan, cyclophosphamide, ifosfamide nitrosourea, or thiotepa); RNA polymerase inhibitors such as α-amanitin; antimitotic agents (e.g., vinca alkaloids such as vincristine, or taxanes such as paclitaxel or docetaxel); topoisomerase inhibitors (e.g., etoposide, teniposide, amsacrine, topotecan, exitecan); cell cycle inhibitors (e.g., flavopyridol); or antimicrotubule agents (e.g., epothilones, tubulysine, pre-tubulysine, discodermolide analogues, or eleutherobin analogues). The therapeutic agent can be a proteasome inhibitor or a topoisomerase inhibitor such as bortezomib, amsacrine, etoposide, etoposide phosphate, teniposide, or doxorubicin. Therapeutic radioisotopes include iodine (131I), yttrium (90Y), lutetium (177Lu), actinium (225Ac), praseodymium, astatine (At), rhenium (Re), bismuth (Bi or Bi), and rhodium (Rh), etc. Antiangiogenic agents include linoamide, bevacizumab, angiostatin, and razoxane.

Applicable therapeutic agents can be immunomodulators, such as Sting agonists, TLR7, TLR8 and/or TLR9 agonists, PD-1 inhibitors, etc.

Pharmaceutical molecules comprising detectable markers can be used for diagnosis. Examples may include contrast agents. Contrast agents can be radioisotope labels such as iodine (131I or 125I), indium (111In), technetium (99Tc), phosphorus (32P), carbon (14C), tritium (3H), other radioisotopes (such as radioactive ions), or therapeutic radioisotopes such as one of the therapeutic radioisotopes listed above. In addition, contrast agents can include radiopaque materials, magnetic resonance imaging (MRI) agents, ultrasonic imaging agents and any other contrast agents suitable for detection by the device of imaging animal body. The detectable marker can also be a fluorescent marker, a biologically active enzyme marker, a luminescent marker or a chromophore marker.

The present invention also relates to a pharmaceutical composition made from the above-mentioned antigen-binding proteins, nucleic acid molecules, vectors, cells, and pharmaceutically acceptable carriers and/or additives.

The aforementioned pharmaceutical composition can be formulated according to conventional methods and can also contain pharmaceutically acceptable carriers and/or additives. The additives include, but are not limited to, surfactants, excipients, colorants, flavorings, preservatives, stabilizers, buffers, suspending agents, isotonic agents, binders, disintegrants, lubricants, flow enhancers, flavoring agents, etc. Carriers for the pharmaceutical composition include, but are not limited to, light anhydrous silicic acid, lactose, crystalline cellulose, mannitol, starch, carboxymethylcellulose calcium, carboxymethylcellulose sodium, hydroxypropyl cellulose, hydroxypropyl methylcellulose, polyvinyl acetal diethylamino acetate, polyvinyl pyrrolidone, gelatin, medium-chain fatty acid triglycerides, polyoxyethylene hydrogenated castor oil 60, white sugar, carboxymethyl cellulose, corn starch, inorganic salts, etc.

Multiple antibodies may be combined as needed, for formulating in the aforementioned pharmaceutical compositions. For example, creating a cocktail of multiple anti-CLDN6 antibodies may enhance cytotoxicity against CLDN6-expressing cells. Alternatively, in addition to anti-CLDN6 antibodies, the therapeutic effect may also be enhanced by incorporating antibodies that recognize other tumor-associated antigens.

Administration routes of the pharmaceutical composition may be oral or non-oral. Non-oral administration routes include, for example, injection, nasal administration, pulmonary administration, transdermal administration, and the like. Injections may further include intravenous, intramuscular, intraperitoneal, and subcutaneous injections. Furthermore, the dosage may be specifically adjusted based on the patient's age and symptoms. For example, the dosage may be 0.0001 mg to 1000 mg per kg of body weight per administration. For another example, the dosage may be 0.001 mg to 100,000 mg per administration for each patient. It should be noted that the dosages described above are for illustration purposes only and are not intended to limit the scope of the dosage.

The type of cancer treated and diagnosed by the present invention is not particularly limited, and is typically a cancer expressing CLDN6 protein, preferably testicular cancer, ovarian cancer, endometrial cancer, liver cancer, pancreatic cancer, choriocarcinoma, or non-small cell lung cancer.

The present invention is further described below with reference to Examples.

### Example 1. Cell Lines

Human ovarian cancer cells PA-1 and OV90, human triple-negative breast cancer cell line MCF-7, and human choriocarcinoma cell line JEG-3 were purchased from ATCC.

The OV90 cell line was grown in a 1:1 MCDB 105 Medium: Medium 199 (Thermo Scientific; Logan, UT) supplemented with 15% (v/v) heat-inactivated FBS (Thermo Scientific; Logan, UT).

PA-1, JEG-3, and MCF-7 cell lines were grown in MEM (Thermo Scientific; Logan, UT) supplemented with 10% (v/v) heat-inactivated FBS (Thermo Scientific; Logan, UT), 1% (v/v) nonessential amino acids, and 1 mM sodium pyruvate to support adherent culture.

### Example 2. Expression and Purification of Antibody

The variable region coding region of the antibody was prepared by whole gene synthesis using MHSSALLCCLVLLTGVRA as a leading signal peptide and assembled with the human IgH-γ1 and IgL-κ coding regions through NotI/XbaI restriction sites into the expression vector pcDNA3.4, which has a CMV promoter that can enhance the high-level expression of multiple genes in mammalian cells, and contains an ampicillin resistance gene as a selective marker.

CHO-K1 cells were selected as the host for antibody expression. Cells were passaged three times under appropriate conditions (120 rpm, 8% CO₂, 37°C). Cells were harvested and mixed thoroughly with electroporation buffer. Plasmids were then added, mixed thoroughly, and transferred to electroporation tubes. The pcDNA3.4 expression vector was transfected into CHO-K1 cells using an electroporator. After culturing for 24 hours at 37°C, 270 rpm, and 8% CO₂, supplement feed/sodium butyrate/penicillin-streptomycin were added and incubated for 3-7 days. The harvested supernatant was filtered (0.22 µm) to remove cells and used for subsequent purification.

The treated supernatant was purified using a Protein A affinity chromatography column and then eluted with citrate buffer (pH 3.4). The eluate was placed in a dialysis bag for buffer exchange, ultimately yielding the purified AT03-2A antibody. The concentration and quantity of the antibody were determined by measuring absorbance at 280 nM using a NanoDrop instrument. Purity was assessed by SDS-PAGE and SEC-HPLC, revealing a purity of>95%.

Each culture medium was supplemented with 100U/mL penicillin and 100mg/mL streptomycin (Sigma Aldrich).

### Comparative Example 1. Murine/Human Chimeric Antibody cAb-1

According to the method of Example 2, murine/human chimeric antibody cAb-1 was prepared.

| Antibody | Form | SEQ ID No. | Buffer | Concentration (mg/mL) |
|---|---|---|---|---|
| cAb-1 | IgG1 | VH0: SEQ ID NO.1 VL0: SEQ ID NO.5 | PBS (pH7.4) | 0.87 |

The sequences of the complementarity determining regions (CDRs) in the heavy chain variable region and light chain variable region of the above-mentioned antibody are as follows:

| | | Amino acid sequence | SEQ ID NO |
|---|---|---|---|
| VH | HCDR1 | SYTMS | 2 |
| | HCDR2 | TISSGGGRTYYPDSVKG | 3 |
| | HCDR3 | GDYRYDGFAY | 4 |
| VL | LCDR1 | RASENIDSYLA | 6 |
| | LCDR2 | ASTLLVD | 7 |
| | LCDR3 | QHYYSIPYT | 8 |

Under different definition schemes, the CDR region sequences are as follows:

| | CDR1 | CDR2 | CDR3 |
|---|---|---|---|
| Variable region | VH | | |
| IMGT | GFTFNDYT | ISSGGGRT | IRGDYRYDAFAY |
| Kabat | DYTMS | TISSGGGRTYYPDSVKG | GDYRYDAFAY |
| Chothia | GFTFNDY | SSGGGR | GDYRYDAFAY |

| Variable region | VL | | |
|---|---|---|---|
| IMGT | ENIDSY | AST | QHYYSIPYT |
| Kabat | RASENIDSYLA | ASTLLVD | QHYYSIPYT |
| Chothia | RASENIDSYLA | ASTLLVD | QHYYSIPYT |

Antibody DTDH4 (INFECTION AND IMMUNITY, June 2006, p.3682-3683) was used as an isotype control (Isotype). This antibody is an anti-human DT antibody.

### Comparative Examples 2~6. Humanized Antibodies hAbs-1~5

According to the method of Example 2, the CDRs of Comparative Example 1 were implanted into the humanized framework by CDR transplantation to obtain humanized antibodies hAbs-1~5. The variable region sequences of hAbs-1~5 were shown in the following table.

| Antibody | Form | SEQ ID No. | Buffer | Concentration (mg/mL) |
|---|---|---|---|---|
| hAb-1 | IgG1 | VH: SEQ ID NO.11 | PBS (pH7.4) | 0.78 |
| | | VL: SEQ ID NO. 14 | | |
| hAb-2 | IgG1 | VH: SEQ ID NO.12 | PBS (pH7.4) | 0.74 |
| | | VL: SEQ ID NO.15 | | |
| hAb-3 | IgG1 | VH: SEQ ID NO.12 | PBS (pH7.4) | 0.9 |
| | | VL: SEQ ID NO.16 | | |
| hAb-4 | IgG1 | VH: SEQ ID NO.13 | PBS (pH7.4) | 0.09 |
| | | VL: SEQ ID NO.15 | | |
| hAb-5 | IgG1 | VH: SEQ ID NO.13 | PBS (pH7.4) | 0.1 |
| | | VL: SEQ ID NO.16 | | |

### Comparative Example 7. Detection of the Binding Activity of hAbs-1~5 and cAb1 to the hCLDN6-positive Human Ovarian Cancer Cell Line OV90

Flow cytometry: Cells were stained using standard flow cytometry methods. Briefly, for the antibodies hAbs-1~5 and cAb1 to be tested, 4×10⁵ cells of hCLDN6-positive human ovarian cancer cell line OV90 were stained with serial dilutions (4-fold dilutions from 100 nM to 0.006 nM) of the test antibodies for 1 hour on ice. After washing twice with ice-cold PBS comprising 1% (v/v) FBS, the cells were incubated with FITC (Abcam)-conjugated goat anti-human IgG diluted in 100 µL PBS (1:200) comprising 1% (v/v) FBS on ice for 1 hour. The staining after cell binding was analyzed using a Flow cytometer (Agilent).

As shown in Figure 2, the CLDN6 murine/human chimeric monoclonal antibody cAb-1 had strong binding activity to OV90 cells, whereas the CDR-implanted humanized antibodies hAbs-1~5 almost completely lost their binding activity to OV90 cells.

### Examples 3~22. Humanized Antibodies hAbs-6~25 with Back Mutations Introduced after CDR Implantation

Humanization design was performed by selecting appropriate human germline sequences and introducing back mutations. The heavy chain variable region VH and light chain variable region VL sequences with back mutations introduced included:

| Variable region | SEQ ID No. |
|---|---|
| VH1 | 17 |
| VH2 | 18 |
| VH3 | 19 |
| VH4 | 20 |
| VH5 | 21 |
| VH6 | 22 |
| VH7 | 23 |
| VH8 | 24 |
| VL1 | 25 |
| VL2 | 26 |
| VL3 | 27 |
| VL4 | 28 |

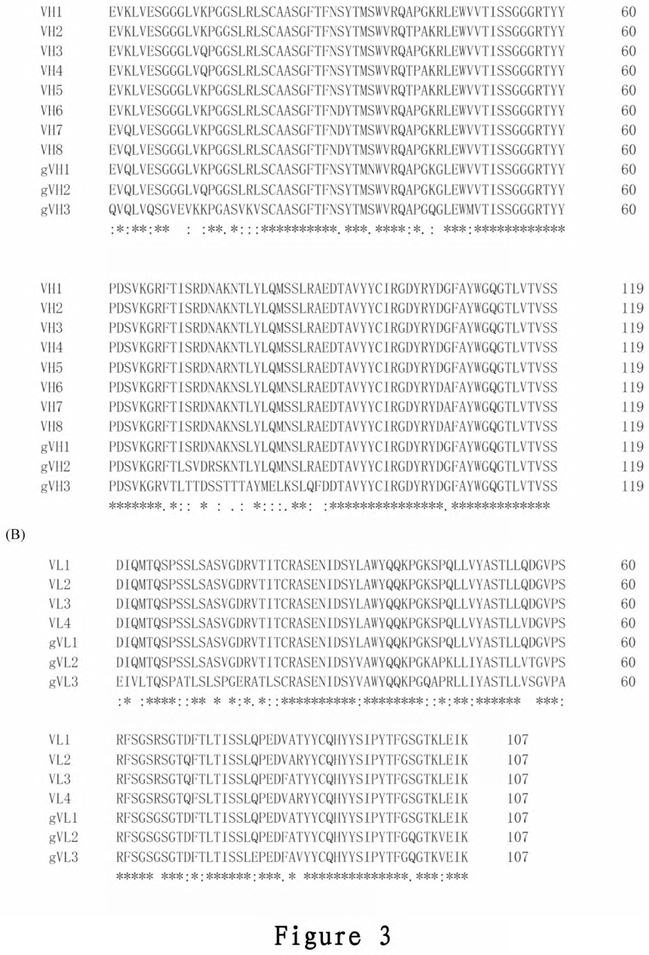

Figure 3A showed the sequence alignment among the heavy chain variable regions VH, and Figure 3B showed the sequence alignment among the light chain variable regions VL.

The method for small-scale expression of humanized antibodies with back mutations introduced was as follows: LVTransm transfection reagent and expression vector of the antibody taken from a refrigerator were thawed at room temperature, and mixed thoroughly by pipetting up and down. PBS was warmed to room temperature. 2 mL of PBS was added to one well of a 6-well plate and 2 µg of pcDNA3.4-IgG1+2 µg of pcDNA3.4-IgKc were added respectively and mixed thoroughly by pipetting up and down. Then 12 µL of LVTransm was added and immediately mixed by pipetting up and down. Let stand at room temperature for 10 minutes. The above DNA/LVTransm complex was added to 3 mL of 293F cells and gently shaked to mix thoroughly. The cells were further cultured at 37°C, 5% CO₂ incubator. After 48 hours of continuous culture, the supernatant was collected by centrifugation and used for ELISA to detect antigen binding. Antibodies were listed in the table below.

| **Antibody** | **Combination of variable regions** | **Form** |
|---|---|---|
| hAb-6 | VH1VL1 | IgG1 |
| hAb-7 | VH1VL2 | IgG1 |
| hAb-8 | VH1VL3 | IgG1 |
| hAb-9 | VH1VL4 | IgG1 |
| hAb-10 | VH2VL1 | IgG1 |
| hAb-11 | VH2VL2 | IgG1 |
| hAb-12 | VH2VL3 | IgG1 |
| hAb-13 | VH2VL4 | IgG1 |
| hAb-14 | VH3VL1 | IgG1 |
| hAb-15 | VH3VL2 | IgG1 |
| hAb-16 | VH3VL3 | IgG1 |
| hAb-17 | VH3VL4 | IgG1 |
| hAb-18 | VH4VL1 | IgG1 |
| hAb-19 | VH4VL2 | IgG1 |
| hAb-20 | VH4VL3 | IgG1 |
| hAb-21 | VH4VL4 | IgG1 |
| hAb-22 | VH5VL1 | IgG1 |
| hAb-23 | VH5VL2 | IgG1 |
| hAb-24 | VH5VL3 | IgG1 |
| hAb-25 | VH5VL4 | IgG1 |

### Example 23. Detection of Binding Activity of cAb-1 and hAbs-6~25 to VLP-hCLDN6

AT-003-ag1 and Control Protein were diluted to 4 µg /mL in sterile CBS (0.035 mol/L NaHCO₃, 0.015 mol/L Na₂CO₃). 100 µL of the dilution was added to each well of a new 96-well plate and coated overnight at 4 °C. The plate was allowed to stand at room temperature for 10 minutes, the antigen coating solution was removed, and the plate was washed once with PBS (pH 7.4). 300 µL/well of blocking buffer (4% skim milk powder in PBS) was added and the plate was blocked at 37°C for 2 hours. After removing the blocking buffer, the plate was washed once with PBS (pH 7.4). Various volumes of the aforementioned cAb-1 and hAbs-6~25 (100 µL of total volume) were added and incubated at 37°C for 1 hour. Control wells are filled with PBS. The wells were drained and washed three times with PBST (0.1% Tween-20 in PBST, pH7.4), followed by three washes with PBS. 100 µL of HRP-Protein A (1:10,000 dilution) was added and incubated at 37°C for 1 hour. The wells were drained and washed three times with PBST (0.1% Tween-20 in PBST, pH7.4), followed by three washes with PBS. 100 µL/well of TMB developing solution was added and incubated at room temperature in the dark for 10 minutes. 50 µL/well of stop solution (2M HCl) was added and the OD450 values of wells were read using a microplate reader. The results were shown in the tables below:

| Antibody | cAb-1 | hAb-6 | hAb-7 | hAb-8 | hAb-9 | hAb-10 | hAb-11 | hAb-12 |
|---|---|---|---|---|---|---|---|---|
| 100 ul | 1.68 | 1.55 | 1.73 | 1.84 | 1.84 | 1.50 | 1.81 | 1.76 |
| | 1.75 | 1.61 | 1.91 | 1.88 | 1.94 | 1.51 | 1.80 | 1.67 |
| 10 ul | 1.47 | 0.89 | 1.37 | 1.27 | 1.71 | 1.18 | 1.41 | 1.36 |
| | 1.46 | 1.04 | 1.46 | 1.22 | 1.57 | 1.13 | 1.54 | 1.40 |

| Antibody | hAb-13 | hAb-14 | hAb-15 | hAb-16 | hAb-17 | hAb-18 | hAb-19 |
|---|---|---|---|---|---|---|---|
| 100 ul | 1.95 | 0.97 | 1.78 | 1.39 | 1.93 | 1.68 | 1.70 |
| | 2.26 | 0.99 | 1.80 | 1.71 | 1.78 | 1.52 | 1.79 |
| 10 ul | 1.68 | 0.22 | 1.39 | 1.26 | 1.60 | 1.17 | 1.62 |
| | 1.70 | 0.23 | 1.47 | 1.24 | 1.72 | 1.13 | 1.57 |

| Antibody | hAb-20 | hAb-21 | hAb-22 | hAb-23 | hAb-24 | hAb-25 | PBS | Blank |
|---|---|---|---|---|---|---|---|---|
| 100 ul | 1.81 | 2.23 | 1.63 | 1.82 | 1.94 | 2.05 | 0.20 | 0.06 |
| | 1.68 | 2.45 | 1.65 | 2.05 | 1.87 | 2.24 | 0.22 | 0.08 |
| 10 ul | 1.38 | 2.05 | 1.12 | 1.42 | 1.45 | 1.69 | | |
| | 1.41 | 1.90 | 0.96 | 1.46 | 1.34 | 1.70 | | |

As shown in the tables above, these humanized antibodies all maintained good VLP-hCLDN6 activity. Compared to hAbs-1~5 (Comparative Examples 2~6), these humanized antibodies retained the R44 site of the murine heavy chain framework region VH0 and the R66 site of the light chain framework region VL0 (Kabat numbering system), indicating that the back mutations at these two sites may be very important.

### Example 24. Detection of Endocytic Activity of Humanized Antibodies with Back Mutations Introduced on Human Choriocarcinoma Cell JEG3 Cells

The humanized antibodies hAbs-6-25 with back mutations introduced were expressed and purified according to the method of Example 2. Humanized antibodies with a culture volume exceeding 2 ml were selected. The selection results were shown in the following table.

| Antibody ID | Combination of variable regions | Form | Buffer | Concentration (mg/ml) |
|---|---|---|---|---|
| hAb-7 | VH1VL2 | IgG1 | PBS (pH7.4) | 1.06 |
| hAb-8 | VH1VL3 | IgG1 | PBS (pH7.4) | 0.90 |
| hAb-13 | VH2VL4 | IgG1 | PBS (pH7.4) | 0.37 |
| hAb-16 | VH3VL3 | IgG1 | PBS (pH7.4) | 1.01 |
| hAb-17 | VH3VL4 | IgG1 | PBS (pH7.4) | 0.54 |
| hAb-21 | VH4VL4 | IgG1 | PBS (pH7.4) | 0.51 |
| hAb-24 | VH5VL3 | IgG1 | PBS (pH7.4) | 0.50 |

Endocytic activity in human choriocarcinoma cell line JEG3 was detected using selected humanized antibodies. Human choriocarcinoma cell JEG3 was cultured overnight in a thermostatic incubator (37°C, 5% CO₂). The selected antibodies were labeled with pHrodo iFL Green human IgG reagent respectively, and the antibody-pHrodo iFL Green human IgG conjugates were incubated with human ovarian cancer cell PA-1 and human choriocarcinoma cell JEG3 for 24 hours respectively. The maximum antibody concentration was set at 10 µg/ml, and a 4-fold serial dilution was performed. After incubation, the cells were washed with PBS and resuspended in culture medium. Fluorescence intensity, which was excited after pHrodo iFL Green entered the cells, was measured using a NovoCyte flow cytometer to analyze endocytic activity for the antibody.

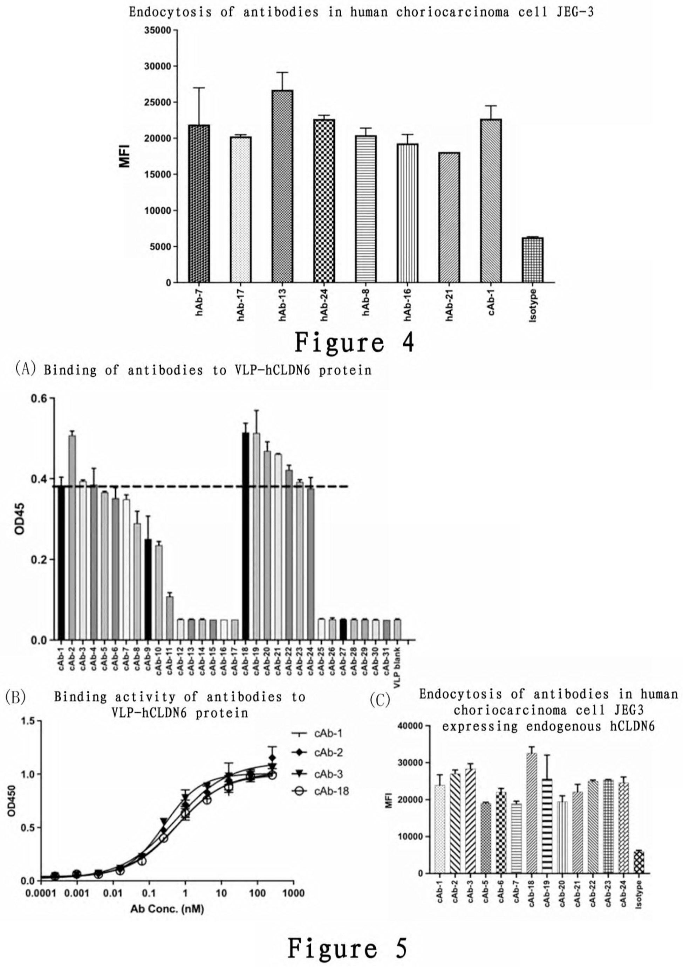

As shown in Figure 4, these humanized antibodies all retained good endocytic activity. Among them, hAb-13, whose variable region combination was VH2VL4, showed higher endocytic activity than the murine/human chimeric antibody cAb-1.

### Examples 25~54. Murine/Human Chimeric Antibodies cAbs-2~31 Comprising Heavy Chain CDR Region Mutations

Based on cAb-1, different sites in the heavy chain CDR region were selected for mutation design. The designed antibodies, along with their final concentrations and buffers, were shown in the table below.

| **Antibody ID** | **Heavy chain CDR region Mutation site** | **Form** | **Antibody SEQ ID HC/LC** | **Buffer** | **Concentration (mg/ml)** |
|---|---|---|---|---|---|
| cAb-2 | S31E | IgG1 | HC: SEQ ID NO. 29 | PBS (pH7.4) | 0.68 |
| | | | LC: SEQ ID NO. 5 | | |
| cAb-3 | S31D | IgG1 | HC: SEQ ID NO. 30 | PBS (pH7.4) | 1.34 |
| | | | LC: SEQ ID NO. 5 | | |
| cAb-4 | S31M | IgG1 | HC: SEQ ID NO.31 | PBS (pH7.4) | 0.76 |
| | | | LC: SEQ ID NO. 5 | | |
| cAb-5 | S31Y | IgG1 | HC: SEQ ID NO. 32 | PBS (pH7.4) | 1.27 |
| | | | LC: SEQ ID NO. 5 | | |
| cAb-6 | S31P | IgG1 | HC: SEQ ID NO. 33 | PBS (pH7.4) | 1.06 |
| | | | LC: SEQ ID NO. 5 | | |
| cAb-7 | S31Q | IgG1 | HC: SEQ ID NO. 34 | PBS (pH7.4) | 1.01 |
| | | | LC: SEQ ID NO. 5 | | |
| cAb-8 | S31N | IgG1 | HC: SEQ ID NO. 35 | PBS (pH7.4) | 0.61 |
| | | | LC: SEQ ID NO. 5 | | |
| cAb-9 | S31W | IgG1 | HC: SEQ ID NO. 36 | PBS (pH7.4) | 0.88 |
| | | | LC: SEQ ID NO. 5 | | |
| cAb-10 | S31A | IgG1 | HC: SEQ ID NO. 37 | PBS (pH7.4) | 0.52 |
| | | | LC: SEQ ID NO. 5 | | |
| cAb-11 | S31V | IgG1 | HC: SEQ ID NO. 38 | PBS (pH7.4) | 1.16 |
| | | | LC: SEQ ID NO. 5 | | |
| cAb-12 | S31H | IgG1 | HC: SEQ ID NO. 39 | PBS (pH7.4) | 1.49 |
| | | | LC: SEQ ID NO. 5 | | |
| cAb-13 | S31I | IgG1 | HC: SEQ ID NO. 40 | PBS (pH7.4) | 0.67 |
| | | | LC: SEQ ID NO. 5 | | |
| cAb-14 | S31R | IgG1 | HC: SEQ ID NO. 41 | PBS (pH7.4) | 0.69 |
| | | | LC: SEQ ID NO. 5 | | |
| cAb-15 | S31K | IgG1 | HC: SEQ ID NO. 42 | PBS (pH7.4) | 1.31 |
| | | | LC: SEQ ID NO. 5 | | |
| cAb-16 | S31F | IgG1 | HC: SEQ ID NO. 43 | PBS (pH7.4) | 1.28 |
| | | | LC: SEQ ID NO. 5 | | |
| cAb-17 | S31L | IgG1 | HC: SEQ ID NO. 44 | PBS (pH7.4) | 0.69 |
| | | | LC: SEQ ID NO. 5 | | |
| cAb-18 | G100K | IgG1 | HC: SEQ ID NO. 45 | PBS (pH7.4) | 0.58 |
| | | | LC: SEQ ID NO. 5 | | |
| cAb-19 | G100L | IgG1 | HC: SEQ ID NO. 46 | PBS (pH7.4) | 1.37 |
| | | | LC: SEQ ID NO. 5 | | |
| cAb-20 | G100F | IgG1 | HC: SEQ ID NO. 47 | PBS (pH7.4) | 1.21 |
| | | | LC: SEQ ID NO. 5 | | |
| cAb-21 | G100R | IgG1 | HC: SEQ ID NO. 48 | PBS (pH7.4) | 0.41 |
| | | | LC: SEQ ID NO. 5 | | |
| cAb-22 | G100W | IgG1 | HC: SEQ ID NO. 49 | PBS (pH7.4) | 1.14 |
| | | | LC: SEQ ID NO. 5 | | |
| cAb-23 | G100I | IgG1 | HC: SEQ ID NO. 50 | PBS (pH7.4) | 0.92 |
| | | | LC: SEQ ID NO. 5 | | |
| cAb-24 | G100A | IgG1 | HC: SEQ ID NO. 51 | PBS (pH7.4) | 0.99 |
| | | | LC: SEQ ID NO. 5 | | |
| cAb-25 | G100Y | IgG1 | HC: SEQ ID NO. 52 | PBS (pH7.4) | 0.43 |
| | | | LC: SEQ ID NO. 5 | | |
| cAb-26 | G100E | IgG1 | HC: SEQ ID NO. 53 | PBS (pH7.4) | 0.56 |
| | | | LC: SEQ ID NO. 5 | | |
| cAb-27 | G100V | IgG1 | HC: SEQ ID NO. 54 | PBS (pH7.4) | 0.70 |
| | | | LC: SEQ ID NO. 5 | | |
| cAb-28 | G100M | IgG1 | HC: SEQ ID NO. 55 | PBS (pH7.4) | 0.74 |
| | | | LC: SEQ ID NO. 5 | | |
| cAb-29 | G100N | IgG1 | HC: SEQ ID NO. 56 | PBS (pH7.4) | 0.69 |
| | | | LC: SEQ ID NO. 5 | | |
| cAb-30 | G100P | IgG1 | HC: SEQ ID NO. 57 | PBS (pH7.4) | 0.66 |
| | | | LC: SEQ ID NO. 5 | | |
| cAb-31 | G100Q | IgG1 | HC: SEQ ID NO. 58 | PBS (pH7.4) | 0.49 |
| | | | LC: SEQ ID NO. 5 | | |

### Example 55. Detection of Binding Activity of cAbs-2~31 to VLP-hCLDN6

ELISA: Each well of a 96-well 3590 plate (Corning, NY) was coated with 100 µL of coating buffer (0.015M Na₂CO₃, 0.035M NaHCO₃, pH 9.5) comprising 400 ng of VLP-CLDN6 at 4°C overnight. After blocking with 150 µL per well of PBS buffer containing 4 % (w/v) skim milk powder at 37°C for 2 h, the supernatant was discarded and the wells were patted dry on a clean paper towel. Then, 100 µL of 4-fold serially diluted antibodies to be tested (cAb-1, cAb-2, cAb-3, cAB-18) was added to each well at 37° C as the primary antibody. After incubation at 37°C for 1 hour, the plate was washed three times with 1% PBST and the supernatant was discarded. After draining on a clean paper towel, 100 µL of PBS buffer containing 1.25 ng/mL horseradish peroxidase (HRP)-conjugated goat anti-human IgG Fc (Invitrogen, California, CA) and 4% (w/v) skim milk powder was added to each well and incubated at 37°C for 45 minutes. After washing three times with 1% PBST and draining on a clean paper towel, the color development reaction was performed using TMB chromogenic solution (Beyotime, Shanghai, CN) according to the manufacturer's instructions. The plate was incubated at room temperature in the dark for 15 minutes until the color developed to the expected intensity, followed by a TMB chromogenic stop solution. The absorbance was measured at 450 nm using a SpectraMax i3X microplate reader (Molecular Devices; Sunnyvale, CA). The results were shown in Figure 5. Some specific EC50 values were presented in the table below.

| Antibody ID | Mutation site in heavy chain CDR region | EC50 (nM) |
|---|---|---|
| cAb-1 | none | 0.59 |
| cAb-2 | S31E | 0.52 |
| cAb-3 | S31D | 0.26 |
| cAb-18 | G100K | 0.58 |

As shown in Figure 5 and the table above, cAbs 2, 3, and 18 all maintained good binding activity to VLP-hCLDN6, among which cAb-3 with the S31D mutation site significantly improved the binding activity to VLP-hCLDN6.

### Examples 56~62. Antibodies hAbs-26~30, 33-34, 44-49 comprising heavy chain CDR region mutations with back mutations introduced

The heavy chain was introduced with mutations at S31 and G100, and implanted into various humanized frameworks comprising back mutations to obtain different humanized antibodies comprising single mutations, double mutations, or multiple mutations, as shown in the table below.

| Antibody | CDR mutation site | Combination of variable region | Form | Antibody SEQ ID HC/LC | Buffer | Concentra tion (mg/ml) |
|---|---|---|---|---|---|---|
| hAb-26 | HC: S31Y | VH2VL4 | IgG1 | HC: SEQ ID NO.59 | PBS (pH7.4) | 2.02 |
| | | | | LC: VL4 | | |
| hAb-27 | HC: S31M | VH2VL4 | IgG1 | HC: SEQ ID NO.60 | PBS (pH7.4) | 2.1 |
| | | | | LC: VL4 | | |
| hAb-28 | HC: S31Q | VH2VL4 | IgG1 | HC: SEQ ID NO.61 | PBS (pH7.4) | 2.33 |
| | | | | LC: VL4 | | |
| hAb-29 | HC: S31D | VH2VL4 | IgG1 | HC: SEQ ID NO.62 | PBS (pH7.4) | 2.37 |
| | | | | LC: VL4 | | |
| hAb-30 | HC: S31E | VH2VL4 | IgG1 | HC: SEQ ID NO.63 | PBS (pH7.4) | 1.8 |
| | | | | LC: VL4 | | |
| hAb-33 | HC: G100A | VH2VL4 | IgG1 | HC: SEQ ID NO.66 | PBS (pH7.4) | 2.3 |
| | | | | LC: VL4 | | |
| hAb-34 | HC: S31D/G100A | VH2VL4 | IgG1 | HC: SEQ ID NO.67 | PBS (pH7.4) | 2.41 |
| | | | | LC: VL4 | | |
| hAb-44 | HC: S31D/G100A | VH6VL5 | IgG1 | HC: SEQ ID NO.22 | PBS (pH7.4) | 0.74 |
| | | | | LC: SEQ ID NO.25 | | |
| hAb-45 | HC: S31D/G100A | VH7VL5 | IgG1 | HC: SEQ ID NO.23 | PBS (pH7.4) | 0.80 |
| | | | | LC: SEQ ID NO.25 | | |
| hAb-46 | HC: S31D/G100A | VH8VL5 | IgG1 | HC: SEQ ID NO.24 | PBS (pH7.4) | 0.81 |
| | | | | LC: SEQ ID NO.25 | | |
| hAb-47 | HC: S31D/G100A | VH6VL4 | IgG1 | HC: SEQ ID NO.22 | PBS (pH7.4) | 0.76 |
| | | | | LC: SEQ ID NO.28 | | |
| hAb-48 | HC: S31D/G100A | VH7VL4 | IgG1 | HC: SEQ ID NO.23 | PBS (pH7.4) | 0.98 |
| | | | | LC: SEQ ID NO. 28 | | |
| hAb-49 | HC: S31D/G100A | VH8VL4 | IgG1 | HC: SEQ ID NO.24 | PBS (pH7.4) | 0.96 |
| | | | | LC: SEQ ID NO.28 | | |

### Comparative Examples 8~18. Antibodies comprising other heavy chain CDR region mutations and introduced back mutations

The CDR region of the heavy chain was introduced with mutations different from those in Examples 56~62, and implanted into a humanized VH2VL4 framework comprising back mutations. Alternatively, S31D/G100A mutations were introduced into the CDR region of the heavy chain, and additional humanizing mutations were introduced into the framework region of VH2VL4 to obtain different humanized antibodies comprising single mutations, double mutations, or multiple mutations, as shown in the table below.

| Antibody | CDR mutation site | Mutation in framework region | Form | Antibody SEQ ID HC/LC | Buffer | Concentratio n (mg/ml) |
|---|---|---|---|---|---|---|
| hAb-31 | HC: G100L | none | IgG1 | HC: SEQ ID NO.64 | PBS (pH7.4) | 2.03 |
| | | | | LC: SEQ ID NO.28 | | |
| hAb-32 | HC: G100I | none | IgG1 | HC: SEQ ID NO.65 | PBS (pH7.4) | 2.31 |
| | | | | LC: SEQ ID NO.28 | | |
| hAb-35 | HC: S31D/G100A | HC: R44G | IgG1 | HC: SEQ ID NO.68 | PBS (pH7.4) | 0.8 |
| | | | | LC: SEQ ID NO.28 | | |
| hAb-36 | HC: S31D/G100A | none | IgG1 | HC: SEQ ID NO.67 | PBS (pH7.4) | 1.02 |
| | LC: V55Q | | | LC: SEQ ID NO.69 | | |
| hAb-37 | HC: S31D/G100A | LC: R66G | IgG1 | HC: SEQ ID NO.67 | PBS (pH7.4) | 0.64 |
| | | | | LC: SEQ ID NO.70 | | |
| hAb-38 | HC: S31D/G100A | LC: Q70D | IgG1 | HC: SEQ ID NO.67 | PBS (pH7.4) | 0.93 |
| | | | | LC: SEQ ID NO.71 | | |
| hAb-39 | HC: S31D/G100A | LC: R85T | IgG1 | HC: SEQ ID NO.67 | PBS (pH7.4) | 0.92 |
| | | | | LC: SEQ ID NO.72 | | |

### Example 63. Detection of Binding Activity of hAbs-26~39, 44~49 to VLP-hCLDN6 or VLP-hCLDN9

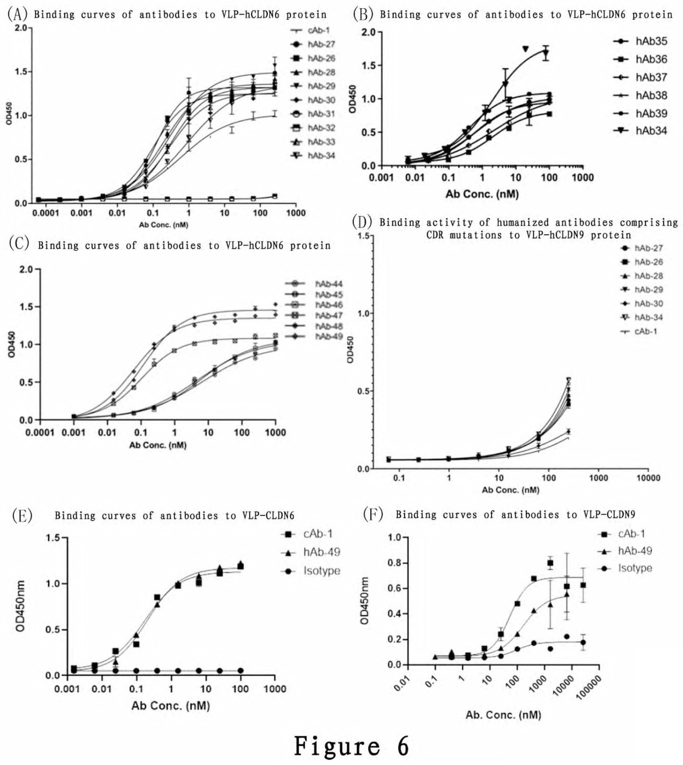

The binding activities of cAb -1, hAbs-26~39, and 44~49 to VLP-hCLDN6 or VLP-hCLDN 9 were detected and compared using the detection method of Example 28. The results were shown in Figure 6.

| Antibody ID | CDR mutation site | EC50 (nM) |
|---|---|---|
| cAb-1 | none | 0.59 |
| hAb-26 | HC: S31Y | 0.10 |
| hAb-27 | HC: S31M | 0.12 |
| hAb-28 | HC: S31Q | 0.19 |
| hAb-29 | HC: S31D | 0.29 |
| hAb-30 | HC: S31E | 0.29 |
| hAb-33 | HC: G100A | 0.31 |
| hAb-34 | HC: S31D/G100A | 0.89 |
| hAb-44 | HC: S31D/G100A | 6.67 |
| hAb-45 | HC: S31D/G100A | 4.84 |
| hAb-46 | HC: S31D/G100A | 6.55 |
| hAb-47 | HC: S31D/G100A | 0.11 |
| hAb-48 | HC: S31D/G100A | 0.13 |
| hAb-49 | HC: S31D/G100A | 0.07 |

As shown in Figures 6A~C, 6E and the table above, when CDRs comprising single mutations of S31Y, S31M, S31Q, S31D, S31E, or G100A were combined with the human VH2VL4 framework region, the binding activity of the antibodies with VLP - hCLDN6 was improved to varying degrees compared with the murine/human chimeric antibody. The affinity of the humanized antibody comprising the S31D/G100A double mutation was also comparable to that of the murine/human chimeric antibody. On the other hand, although CDRs comprising G100L and G100I mutations can produce antibodies with comparable or better affinity or endocytosis when combined with the original murine framework region, CDRs comprising these mutations almost lost their binding activity to VLP - hCLDN6 when combined with the human VH2VL4 framework region, indicating that the G100 site is very important in humanized antibodies based on the human VH2VL4 framework region and that the G100A mutation helps maintaining or improving the activity of humanized antibodies.

As shown in Figures 6D and 6F, the humanized antibodies based on the human VH2VL4 framework region had very low binding activity to VLP - hCLDN9, indicating that these antibodies maintained or improved their selectivity for CLDN6.

### Examples 64~91. Antibodies hAbs-50~84 Comprising Heavy Chain CDR Region Mutations and Introduced Back Mutations

The heavy chain was introduced with mutations at S31 and G100, and implanted into a humanized VH2VL4 framework comprising back mutations. On this basis, new mutations were introduced to obtain different humanized antibodies comprising single mutations, double mutations or multiple mutations, as shown in the table below.

| Antibody | CDR mutation site | Form | Antibody SEQ ID HC/LC | Buffer |
|---|---|---|---|---|
| hAb-50 | HC: N30Q | IgG1 | HC: SEQ ID NO.133 | PBS (pH7.4) |
| | | | LC: SEQ ID NO.28 | |
| hAb-51 | HC: N30E | IgG1 | HC: SEQ ID NO.134 | PBS (pH7.4) |
| | | | LC: SEQ ID NO. 28 | |
| hAb-52 | HC: N30H | IgG1 | HC: SEQ ID NO.135 | PBS (pH7.4) |
| | | | LC: SEQ ID NO. 28 | |
| hAb-53 | HC: N30Y | IgG1 | HC: SEQ ID NO.136 | PBS (pH7.4) |
| | | | LC: SEQ ID NO. 28 | |
| hAb-54 | HC: N30K | IgG1 | HC: SEQ ID NO.137 | PBS (pH7.4) |
| | | | LC: SEQ ID NO. 28 | |
| hAb-55 | HC: N73Q | IgG1 | HC: SEQ ID NO.138 | PBS (pH7.4) |
| | | | LC: SEQ ID NO. 28 | |
| hAb-56 | HC: N73E | IgG1 | HC: SEQ ID NO.139 | PBS (pH7.4) |
| | | | LC: SEQ ID NO. 28 | |
| hAb-57 | HC: N73H | IgG1 | HC: SEQ ID NO.140 | PBS (pH7.4) |
| | | | LC: SEQ ID NO. 28 | |
| hAb-58 | HC: N73Y | IgG1 | HC: SEQ ID NO.141 | PBS (pH7.4) |
| | | | LC: SEQ ID NO.28 | |
| hAb-59 | HC: N73K | IgG1 | HC: SEQ ID NO.142 | PBS (pH7.4) |
| | | | LC: SEQ ID NO. 28 | |
| hAb-60 | HC: N30Q, N73Q | IgG1 | HC: SEQ ID NO.143 | PBS (pH7.4) |
| | | | LC: SEQ ID NO. 28 | |
| hAb-61 | HC: N30E, N73E | IgG1 | HC: SEQ ID NO.144 | PBS (pH7.4) |
| | | | LC: SEQ ID NO. 28 | |
| hAb-62 | HC: N30H, N73H | IgG1 | HC: SEQ ID NO.145 | PBS (pH7.4) |
| | | | LC: SEQ ID NO. 28 | |
| hAb-63 | HC: N30Y, N73Y | IgG1 | HC: SEQ ID NO.146 | PBS (pH7.4) |
| | | | LC: SEQ ID NO. 28 | |
| hAb-64 | HC: N30K, N73K | IgG1 | HC: SEQ ID NO.147 | PBS (pH7.4) |
| | | | LC: SEQ ID NO. 28 | |
| hAb-65 | HC: R56N | IgG1 | HC: SEQ ID NO.148 | PBS (pH7.4) |
| | | | LC: SEQ ID NO. 28 | |
| hAb-66 | HC: R56Q | IgG1 | HC: SEQ ID NO.149 | PBS (pH7.4) |
| | | | LC: SEQ ID NO. 28 | |
| hAb-67 | HC: R56D | IgG1 | HC: SEQ ID NO.150 | PBS (pH7.4) |
| | | | LC: SEQ ID NO. 28 | |
| hAb-68 | HC: R56E | IgG1 | HC: SEQ ID NO.151 | PBS (pH7.4) |
| | | | LC: SEQ ID NO. 28 | |
| hAb-69 | HC: Y97N | IgG1 | HC: SEQ ID NO.152 | PBS (pH7.4) |
| | | | LC: SEQ ID NO. 28 | |
| hAb-70 | HC: Y97Q | IgG1 | HC: SEQ ID NO.153 | PBS (pH7.4) |
| | | | LC: SEQ ID NO. 28 | |
| hAb-71 | LC: Y32Q | IgG1 | HC: SEQ ID NO.24 | PBS (pH7.4) |
| | | | LC: SEQ ID NO.154 | |
| hAb-72 | LC: Y32R | IgG1 | HC: SEQ ID NO.24 | PBS (pH7.4) |
| | | | LC: SEQ ID NO.155 | |
| hAb-73 | LC: I94E | IgG1 | HC: SEQ ID NO.24 | PBS (pH7.4) |
| | | | LC: SEQ ID NO.156 | |
| hAb-74 | LC: K149C | IgG1 | HC: SEQ ID NO.157 | PBS (pH7.4) |
| | | | LC: SEQ ID NO.168 | |
| hAb-75 | HC: L234A/L235A/D26 5C/A121C | IgG1 | HC: SEQ ID NO.158 | PBS (pH7.4) |
| | | | LC: SEQ ID NO.169 | |
| hAb-76 | LC: V205C | IgG1 | HC: SEQ ID NO.159 | PBS (pH7.4) |
| | | | LC: SEQ ID NO.170 | |
| hAb-77 | HC: S239C_S442C | IgG1 | HC: SEQ ID NO.160 | PBS (pH7.4) |
| | | | LC: SEQ ID NO.169 | |
| hAb-78 | HC: A121C | IgG1 | HC: SEQ ID NO.161 | PBS (pH7.4) |
| | | | LC: SEQ ID NO.169 | |
| hAb-79 | HC: S239C | IgG1 | HC: SEQ ID NO.162 | PBS (pH7.4) |
| | | | LC: SEQ ID NO.169 | |
| hAb-80 | HC: D265C | IgG1 | HC: SEQ ID NO.163 | PBS (pH7.4) |
| | | | LC: SEQ ID NO.169 | |
| hAb-81 | HC: C220S/S442C | IgG1 | HC: SEQ ID NO.164 | PBS (pH7.4) |
| | | | LC: SEQ ID NO.169 | |
| hAb-82 | HC: L234A/L235A/S23 9C/A121C | IgG1 | HC: SEQ ID NO.165 | PBS (pH7.4) |
| | | | LC: SEQ ID NO.169 | |
| hAb-83 | HC: S442C | IgG1 | HC: SEQ ID NO.166 | PBS (pH7.4) |
| | | | LC: SEQ ID NO.169 | |
| hAb-84 | HC: C220S | IgG1 | HC: SEQ ID NO.167 | PBS (pH7.4) |
| | | | LC: SEQ ID NO.169 | |
| hAb-85 | HC:L234A/L235A/ S239C/442C | IgG1 | HC: SEQ ID NO.171 | PBS (pH7.4) |
| | | | LC: SEQ ID NO.169 | |

### Examples 92~163. Generation of ADCs-1~72 Using Antibody Interchain Disulfide Bonds and/or Cysteine Mutation Sites

The following ADCs-1~15 were prepared by conjugating 1-20 anti-tumor toxin molecules via the interchain disulfide bond sites of the antibody:

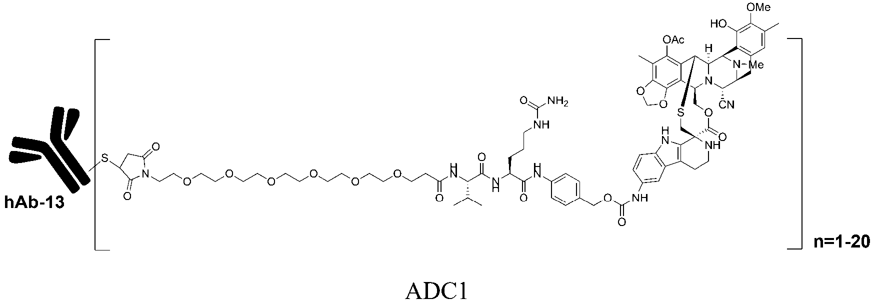

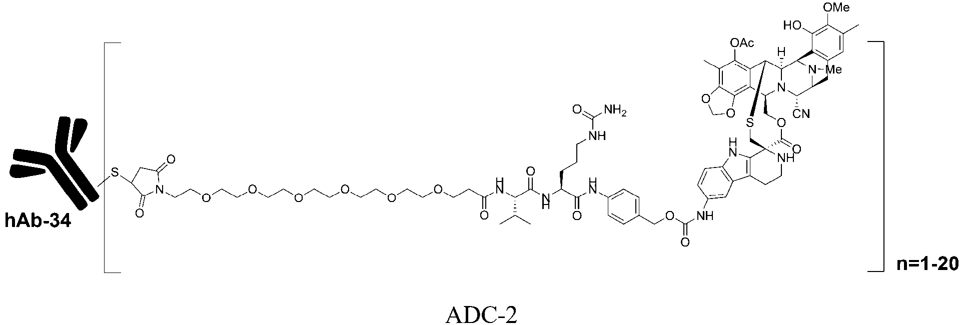

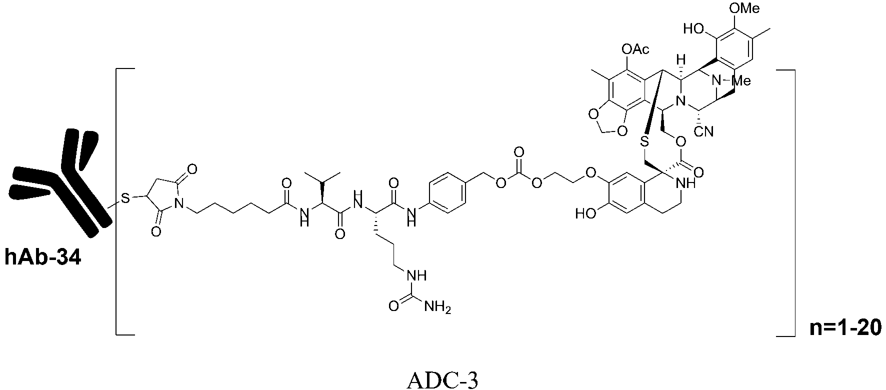

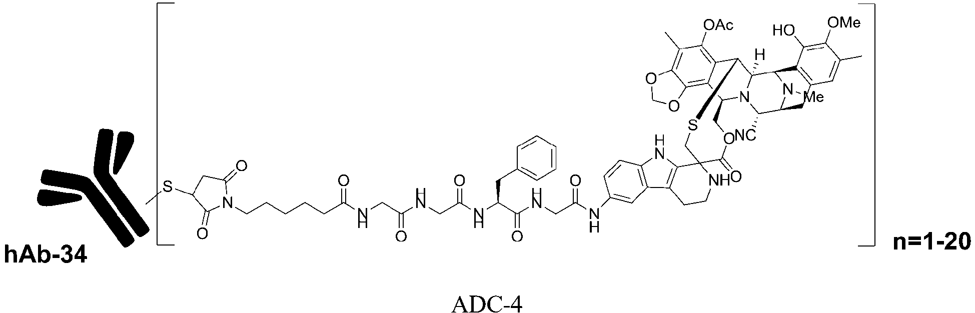

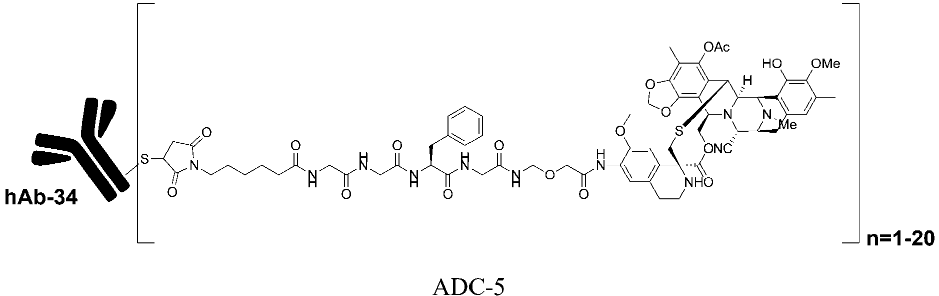

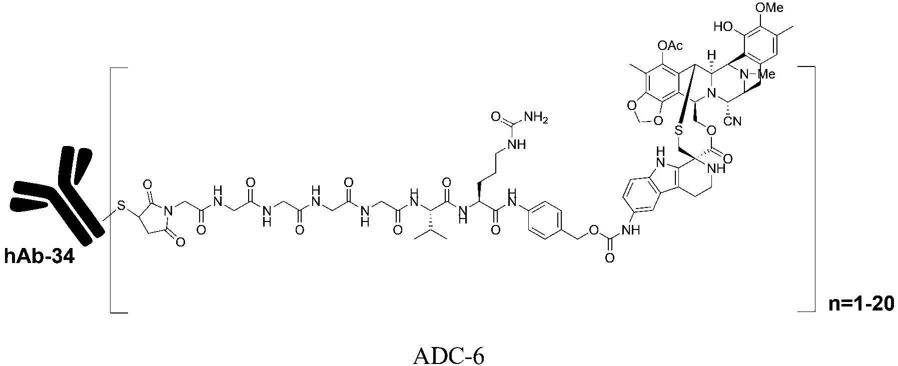

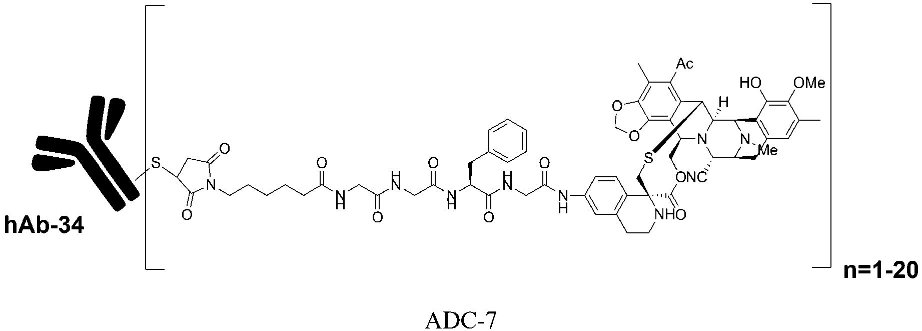

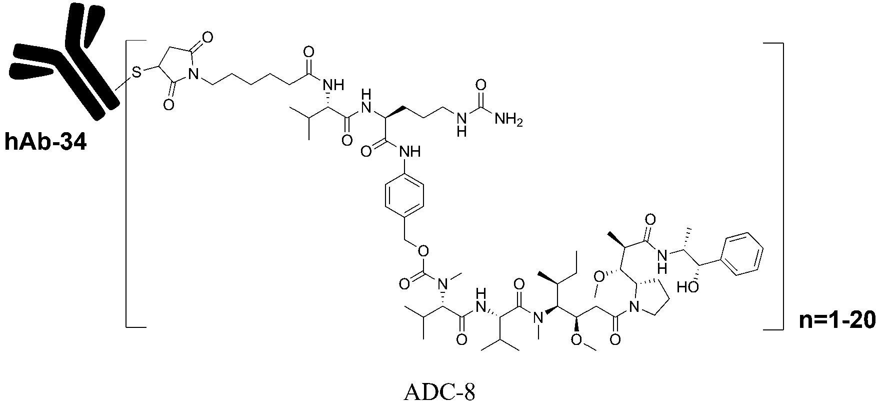

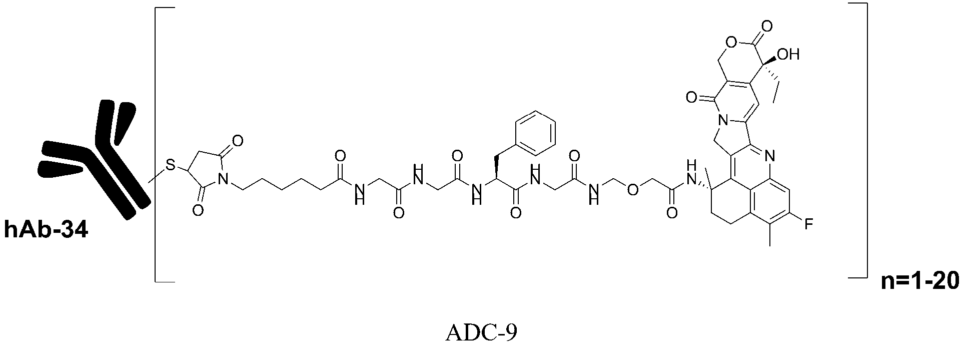

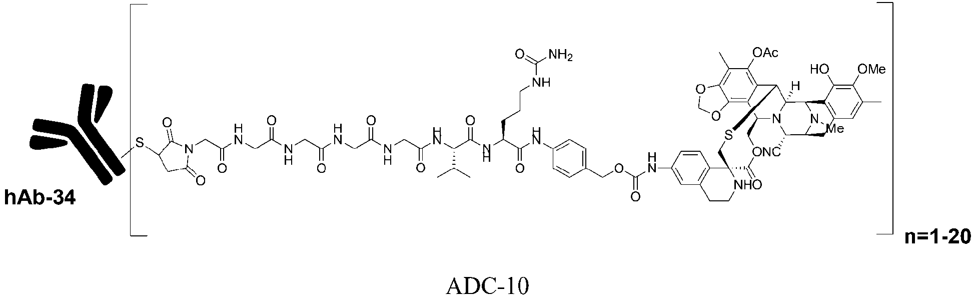

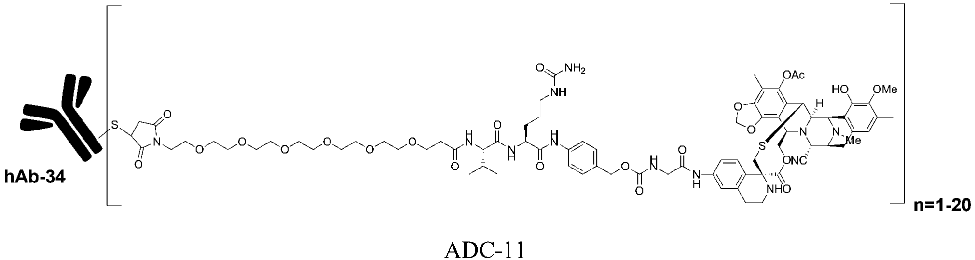

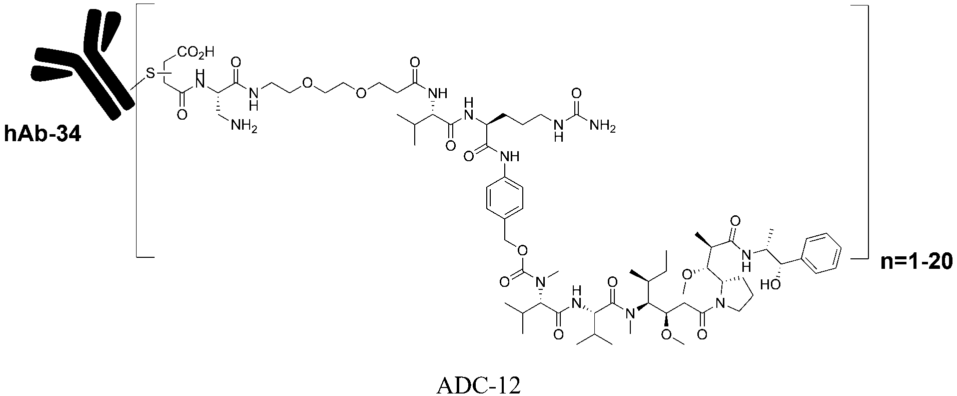

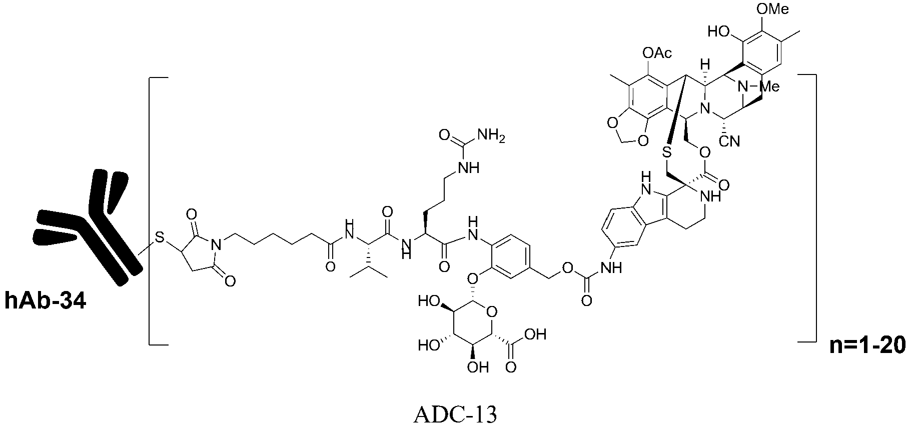

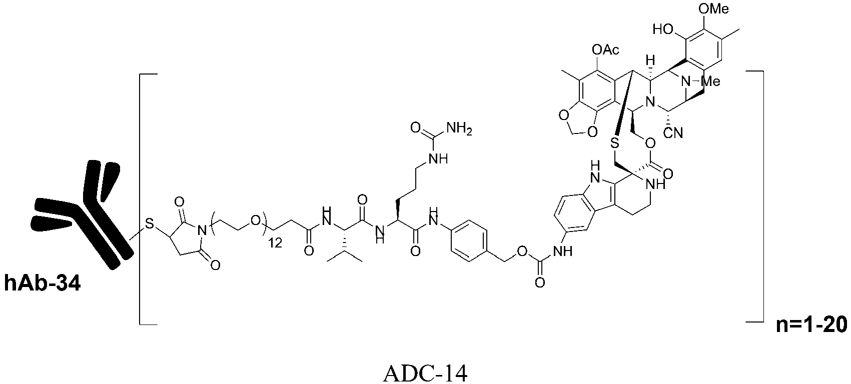

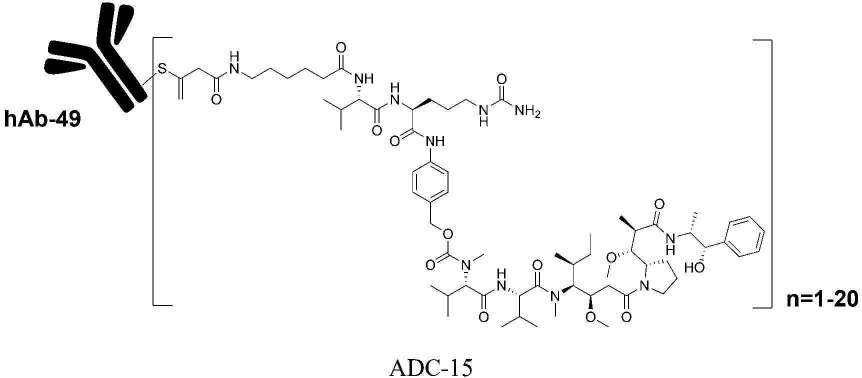

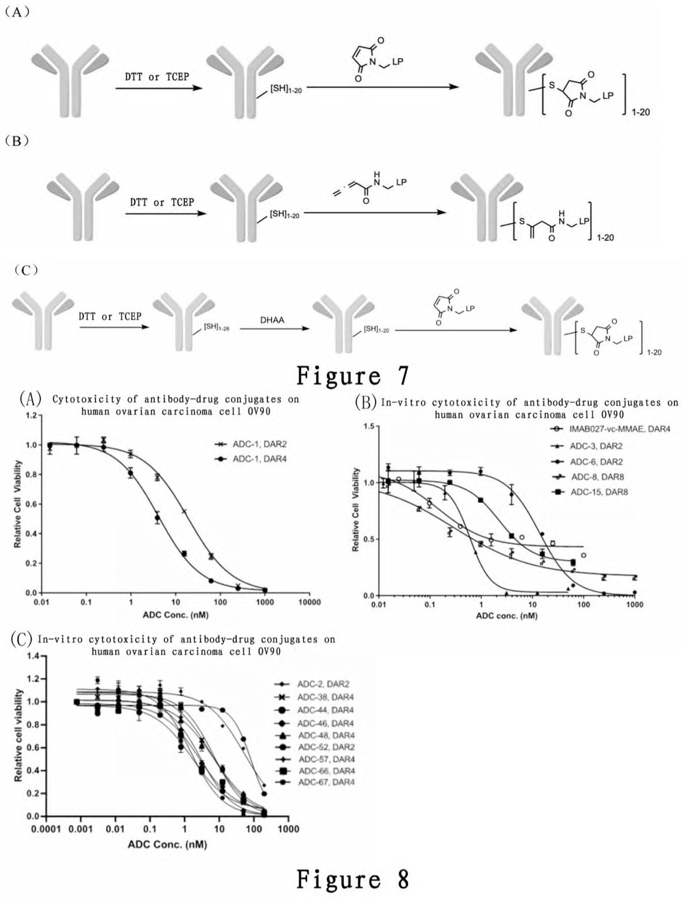

Figure 7(A) illustrated the overall method for preparing antibody-drug conjugates using maleimide connectors. The specific conjugation process was as follows: Adding 2 mg of antibody to a 0.5 ml centrifuge tube. Diluting the antibody to 5 mg/ml using 30 mM His-HAC buffer (pH5.5). Adding 100 mM EDTA aqueous solution to a final concentration of 5 mM and vortexing to mix thoroughly. Adding a volume of TCEP aqueous solution 2~15 times the molar mass of the antibody, vortexing to mix thoroughly, and incubating at 20°C in a thermostatic mixer for 1.5 hours. Adding a volume of toxin-linker solution 4-24 times the molar mass of the antibody, and supplementing with DMSO to 10% of the final reaction volume. Vortexing to mix thoroughly, and incubating at 20°C in a thermostatic mixer for 0.5-24 hours. After conjugation, removing free small molecules using 300 mg/mL dextran-coated activated charcoal (Sigma). Then, the residual activated carbon particles in the supernatant were filtered out with a hydrophilic filter membrane, and finally the ADC storage buffer was replaced with 50 mM PBS (pH6.0) using an ultrafiltration tube and stored at -80°C for future use.

Specifically, for the synthesis of ADCs-1~15, the amounts of TCEP used were 4, 4, 2, 3, and 1.5 times the molar mass of the antibody, respectively, and the amounts of toxin-linker used were 4, 4, 4, 5, and 4 times the molar mass of the antibody, respectively. For the synthesis of IMAB027-vcMMAE, the amounts of TCEP used was 3.4 times the molar mass of the antibody, and the amounts of vcMMAE used was 10 times the molar mass of the antibody, respectively.

Figure 7(B) illustrated the overall method for preparing antibody-drug conjugates using an olefin connector. 1-20 anti-tumor toxin molecules are conjugated to prepare the antibody-drug conjugate. 2 mg of antibody was added to a 0.5ml centrifuge tube. 25mM Na₂B₄O₇, 25mM NaCl, and 1mM DTPA (pH 7.4) buffer were added to dilute the antibody to 10 mg/ml. A volume of TCEP aqueous solution corresponding to 2-15 times the molar mass of the antibody was added and vortexed to mix thoroughly, and incubated in a thermostatic mixer at 20°C for 2-24 hours. A volume of toxin-linker solution corresponding to 4~24 times the molar mass of the antibody was added and incubated at 20°C in a thermostatic mixer for 0.5-24 hours after vortexed to mix thoroughly. After the coupling was completed, the reaction was stopped and the ADC was purified. Taking ADC15 as an example, the amount of TCEP used was 15 times the molar mass of the antibody, and the amount of toxin-linker used was 24 times the molar mass of the antibody.

Figures 7(A) and 7(C) illustrated the overall preparation method of antibody-drug conjugates, wherein 1-20 anti-tumor toxin molecules were conjugated via antibody interchain disulfide bonds and/or cysteine mutation sites to prepare the following ADCs-16~75:
Adding 2 mg of antibody to a 0.5 ml centrifuge tube. Diluting the antibody to 5 mg/ml with 30 mM His-HAC buffer (pH5.5). Adding 100 mM EDTA aqueous solution to a final concentration of 5 mM and vortexing to mix thoroughly. Adding 2 mg/ml TCEP at a 3:1 molar ratio of TCEP to antibody, Vortexing to mix, and incubating at 20°C in a thermostatic mixer for 1.5 hours. Alternatively, the antibody buffer was replaced with 2 mM EDTA, 100 mM Tris-HCl (pH 8.0) using a desalting column. 1 mg of antibody was added to a 1.5 ml microcentrifuge tube, and 100 mM DTT aqueous solution was added at a DTT to antibody molar ratio of 20:1-100:1, pipetted to mix, and incubated at 22°C in a thermostatic mixer overnight. 10 mg/mL linker-toxin DMSO solution was added at a compound to antibody molar ratio of 12:1, and DMSO was supplemented to 20% of the final reaction volume. Vortexing to mix thoroughly, and incubating at 20°C in a thermostatic mixer for 1.5 hours. After conjugation, removing free small molecules using 300 mg/mL dextran-coated activated charcoal (Sigma). Then, the residual activated carbon particles in the supernatant were filtered out with a hydrophilic filter membrane, and finally the ADC storage buffer was replaced with 50 mM PBS (pH7.2) using an ultrafiltration tube and stored at -80°C for future use.

Alternatively, the antibody buffer was replaced with 2 mM EDTA, 100 mM Tris-HCl (pH 8.0) using a desalting column. 1 mg of antibody was added to a 1.5 ml microcentrifuge tube, and 100 mM DTT was added at a DTT to antibody molar ratio of 100:1, pipetted to mix, and incubated at 22°C in a thermostatic mixer overnight. The antibody buffer was replaced with 2 mM EDTA, 150 mM NaCl and 50 mM Tris-HCl(pH 7.5) using a desalting column. A 100 mM DHAA DMA solution was added at a 20:1 molar ratio of DHAA to antibody. Mixing thoroughly by pipetting and incubating in a thermostatic mixer at 22°C for 2 hours. The DHAA was then removed using a desalting column and the antibody buffer was exchanged to 150 mM NaCl and 50 mM Tris-HCl (pH 7.5). A 10 mg/ml linker-toxin DMA solution was added at a compound to antibody molar ratio of 6:1, and DMA was supplemented to 10% of the final reaction volume. Vortexing to mix thoroughly, and incubating at 20°C in a thermostatic mixer for 4 hours. After conjugation, removing free small molecules using a desalting column.

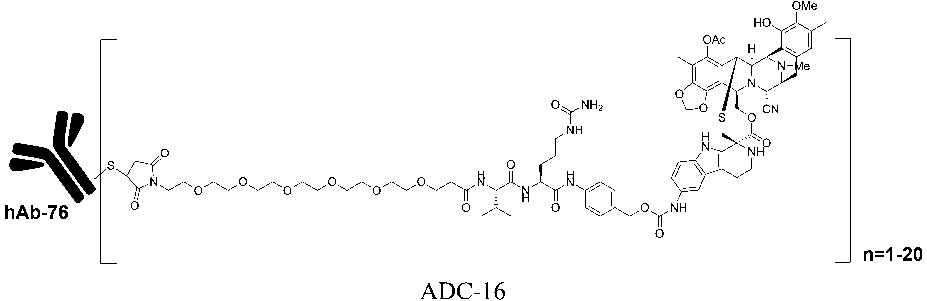

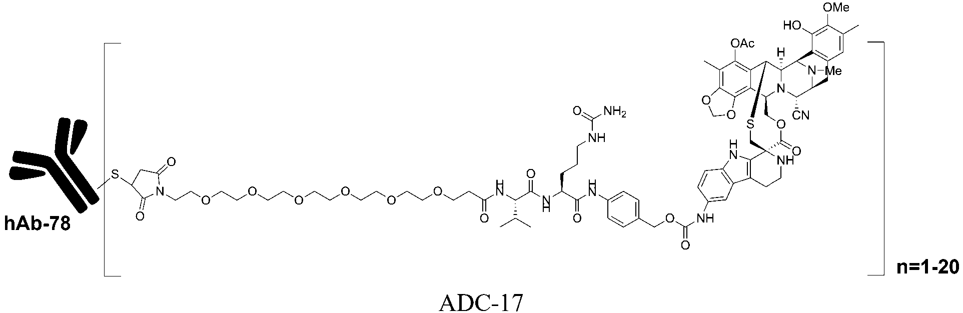

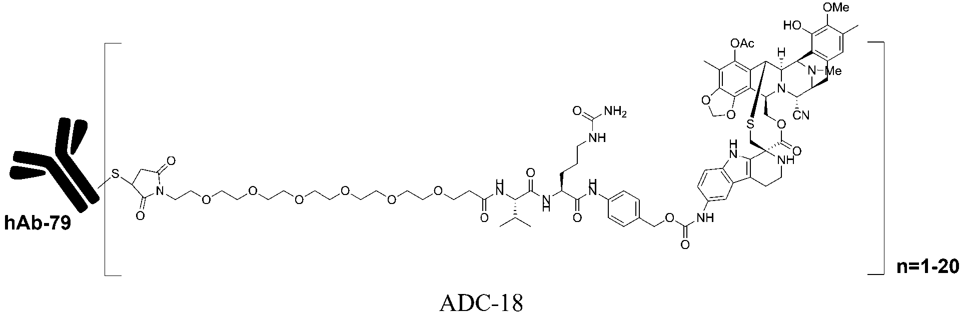

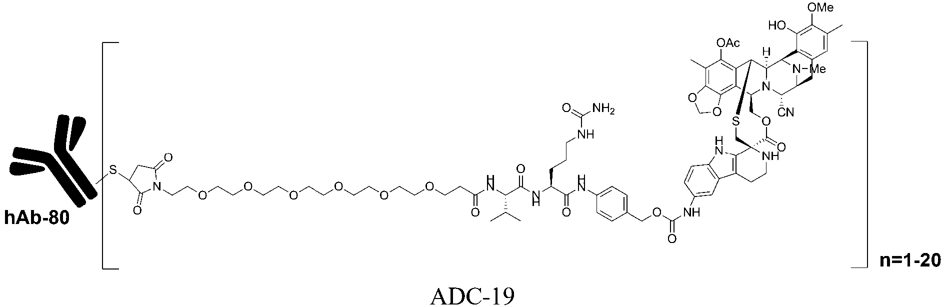

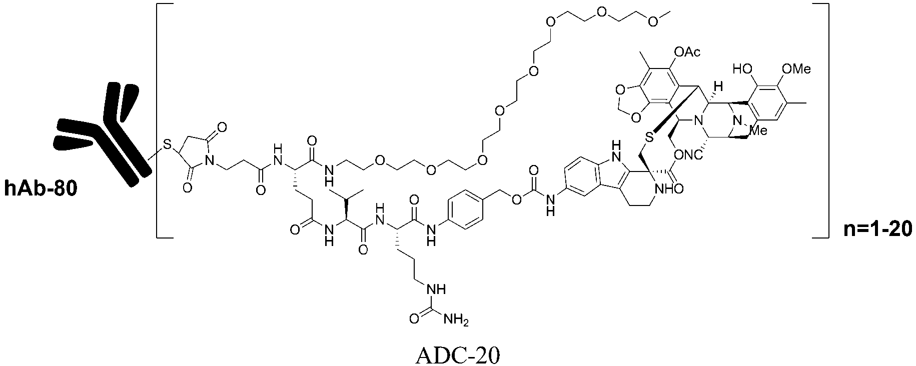

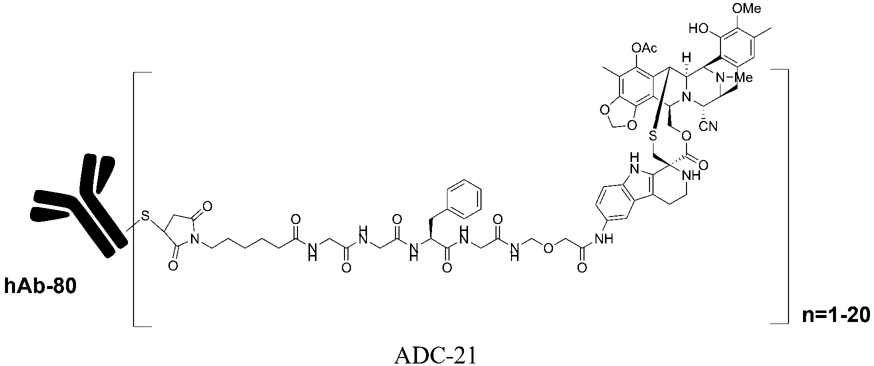

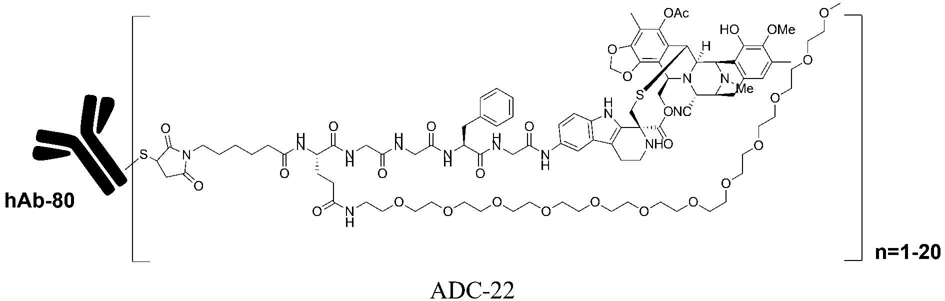

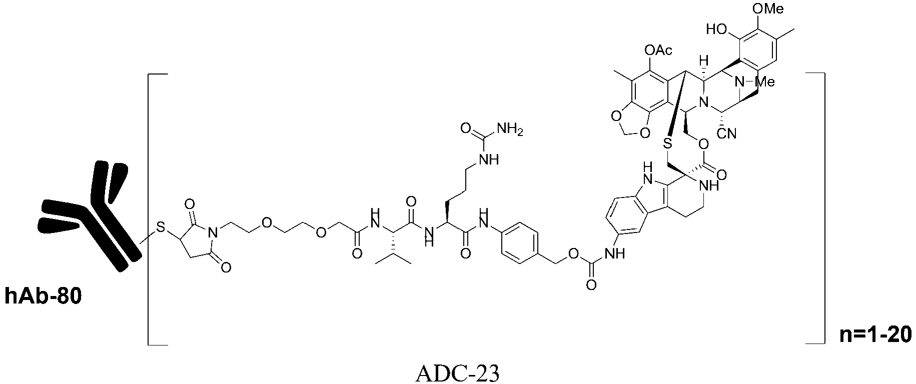

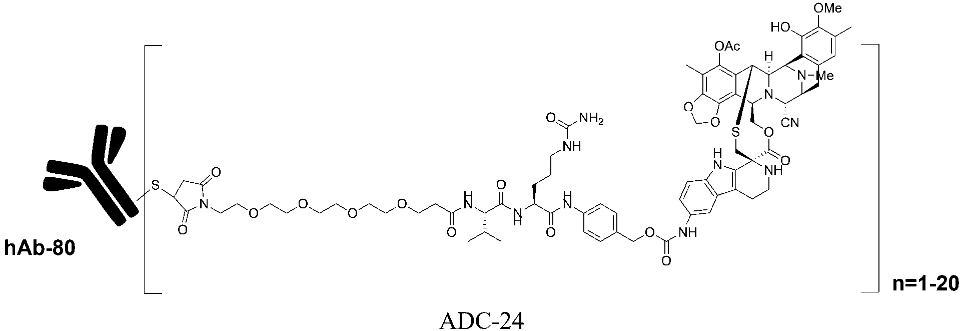

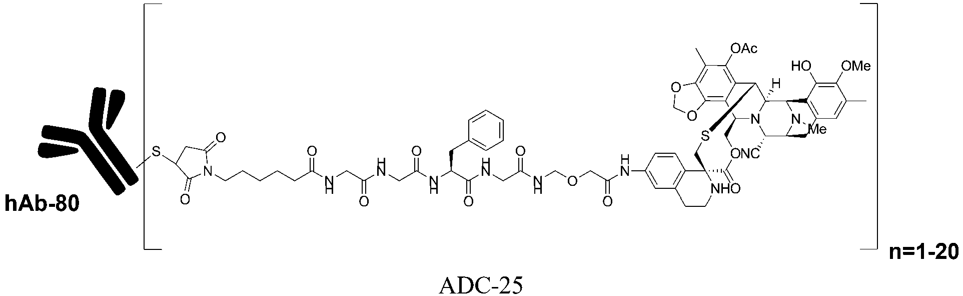

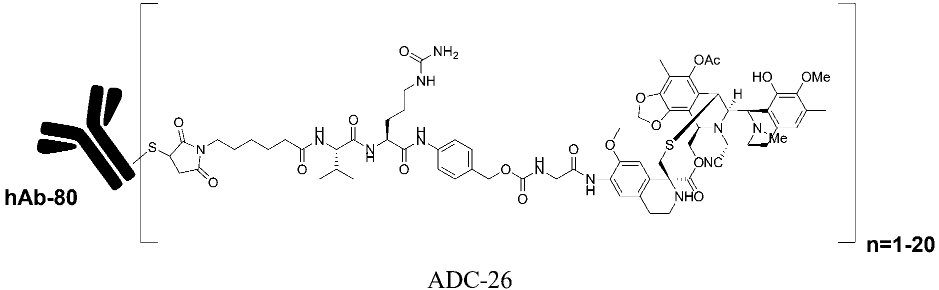

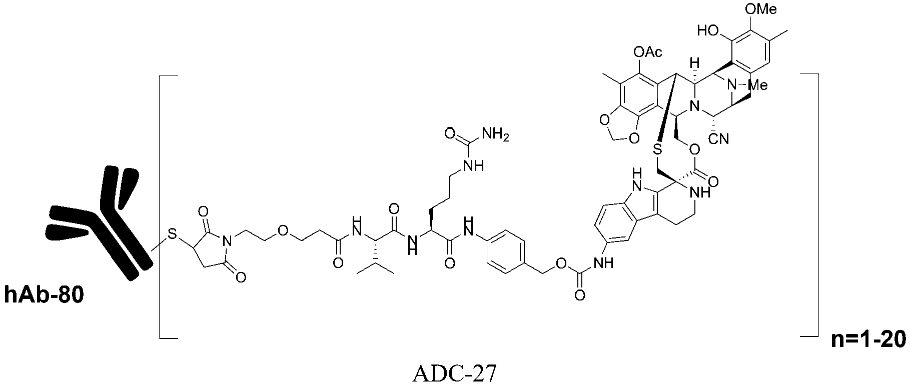

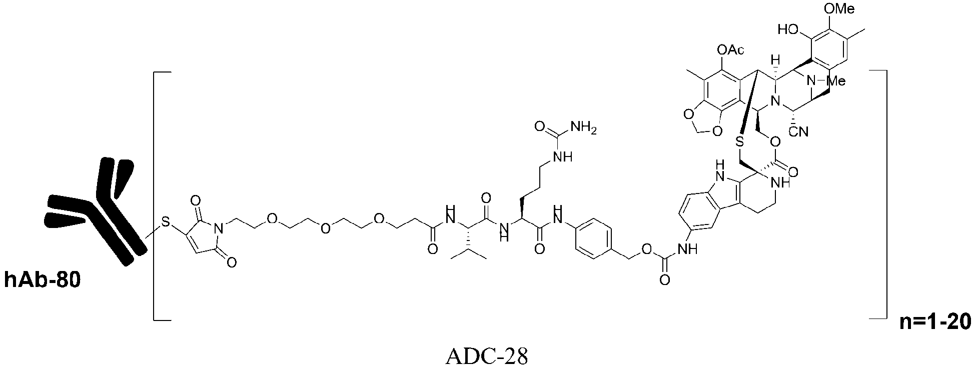

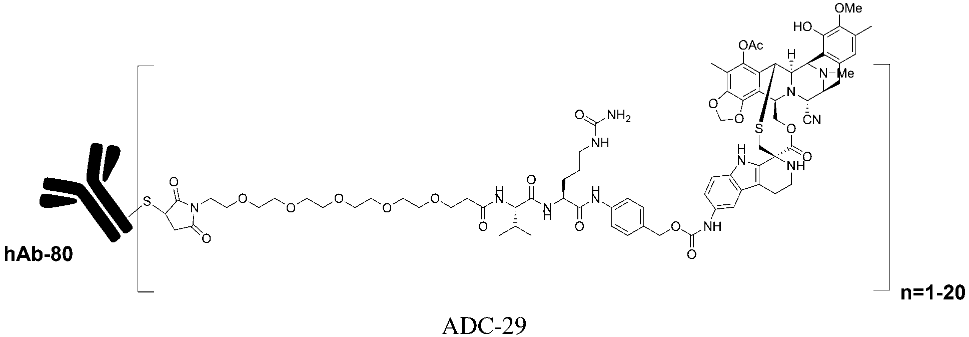

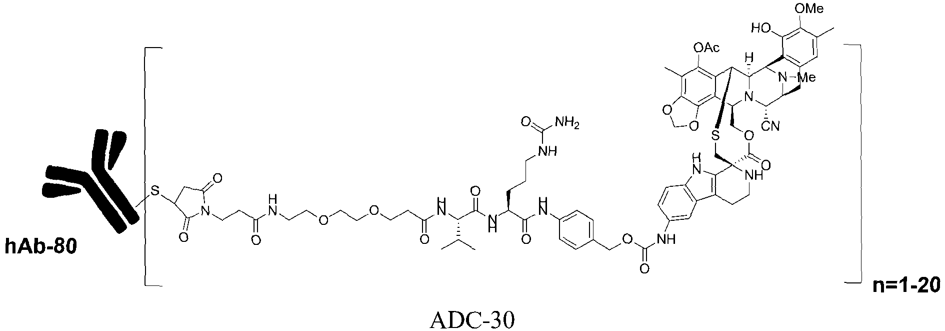

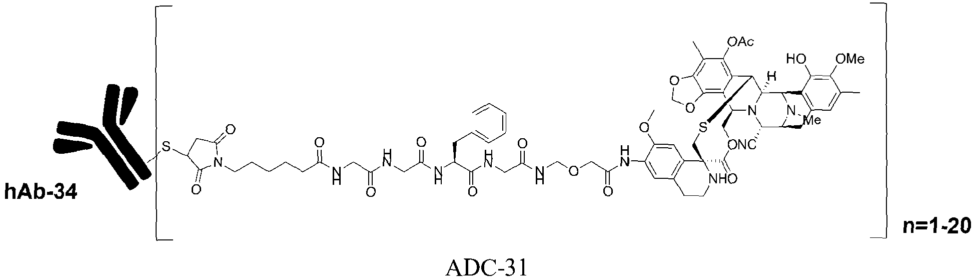

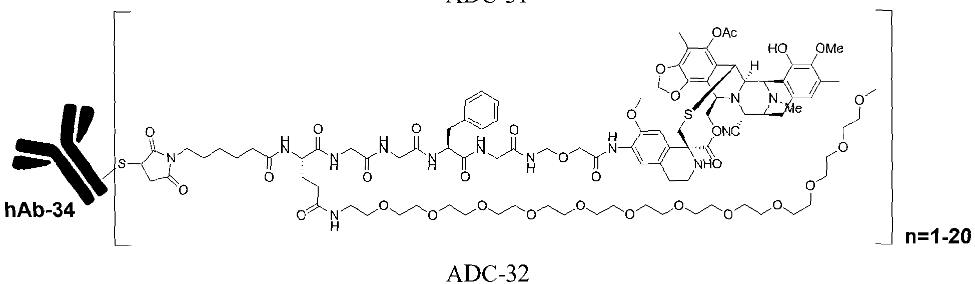

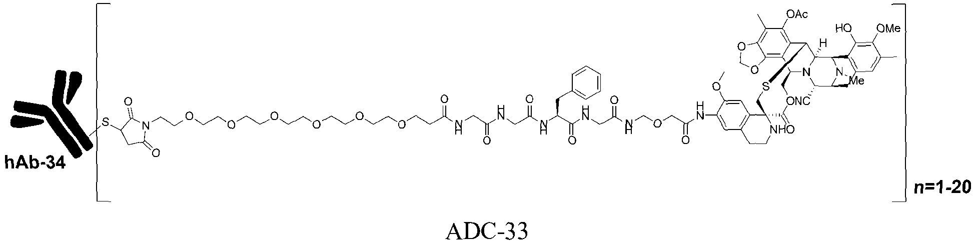

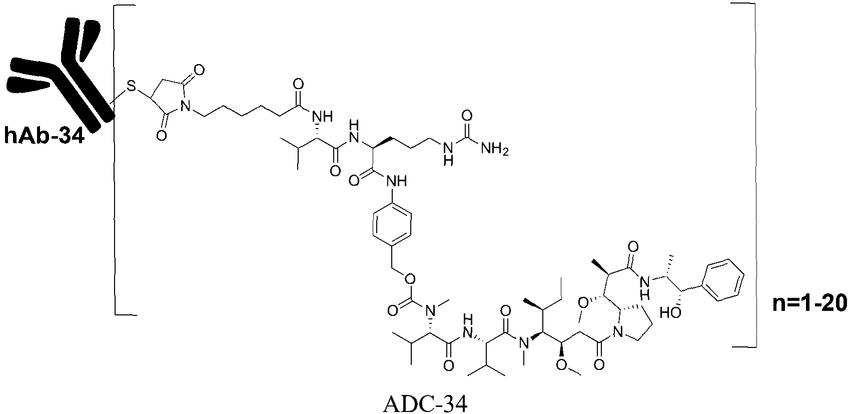

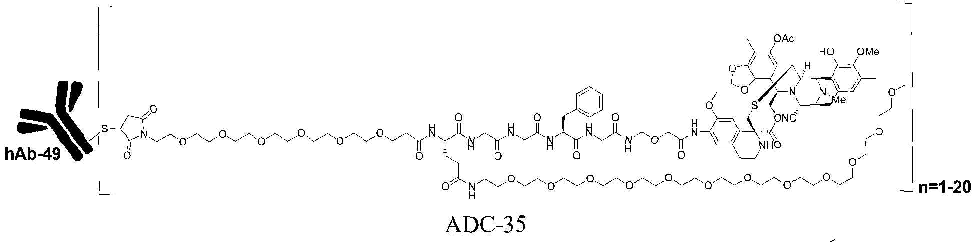

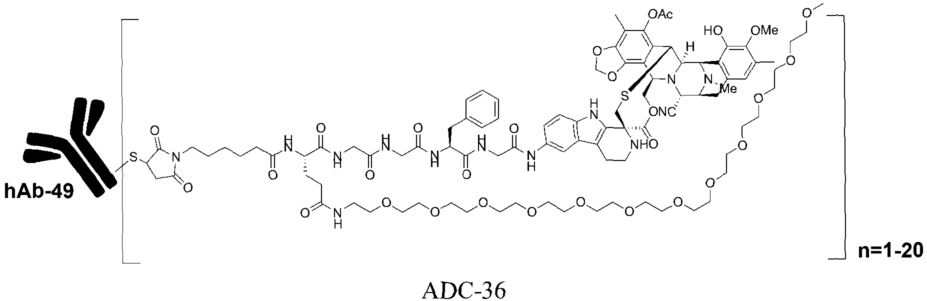

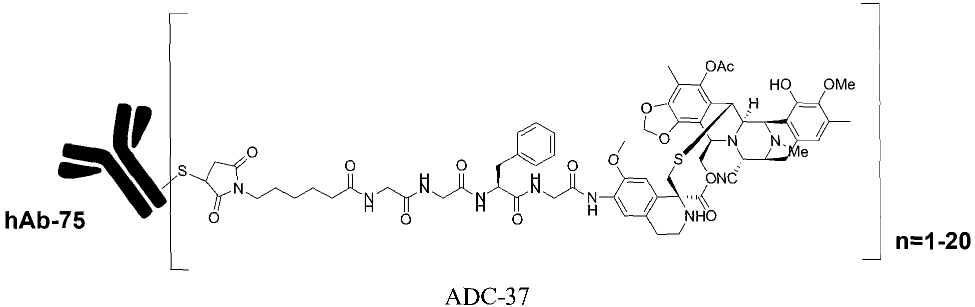

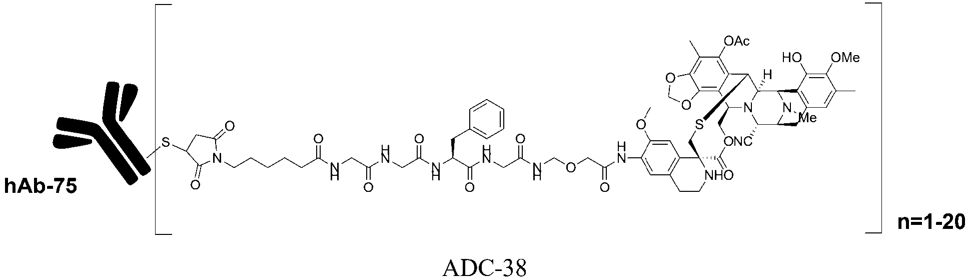

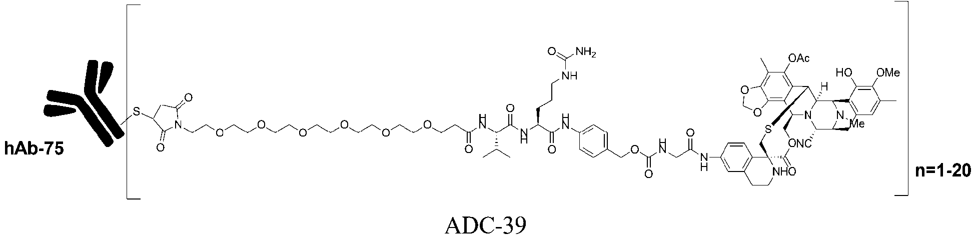

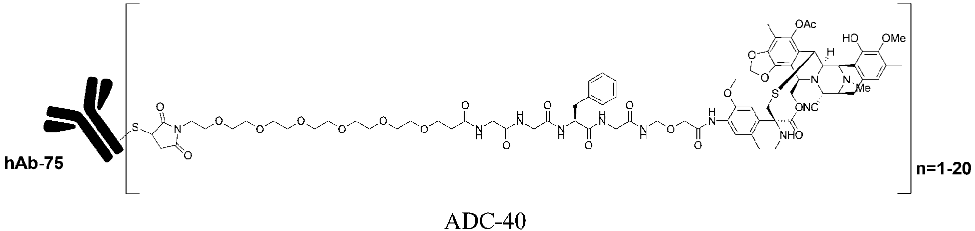

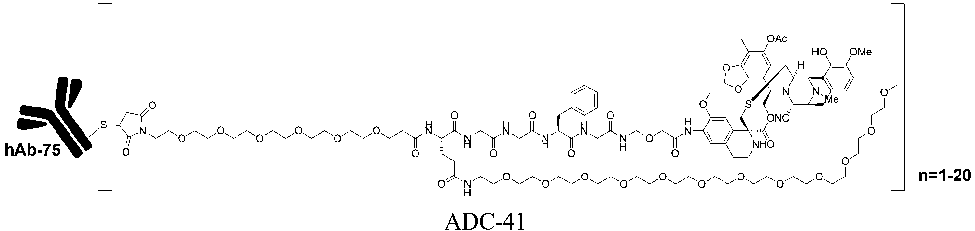

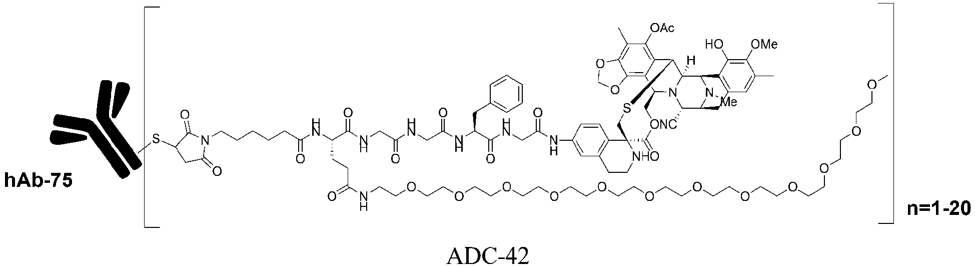

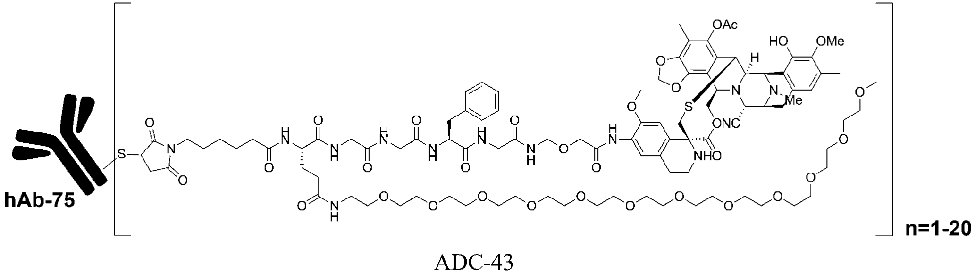

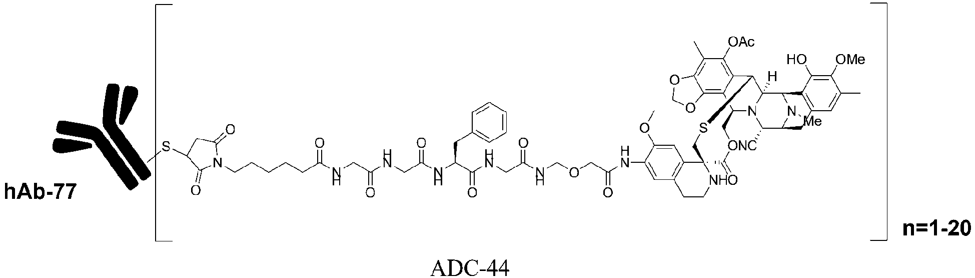

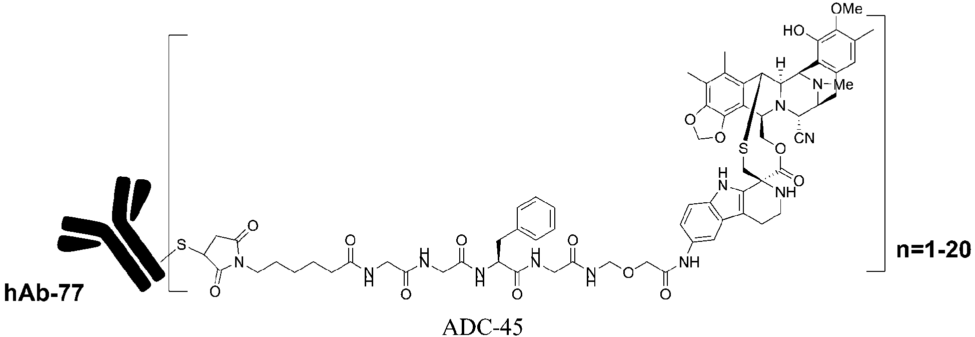

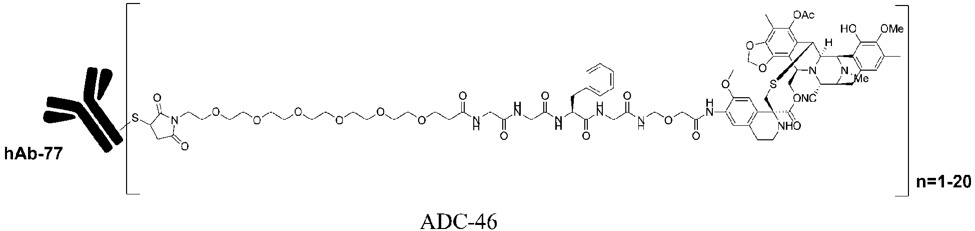

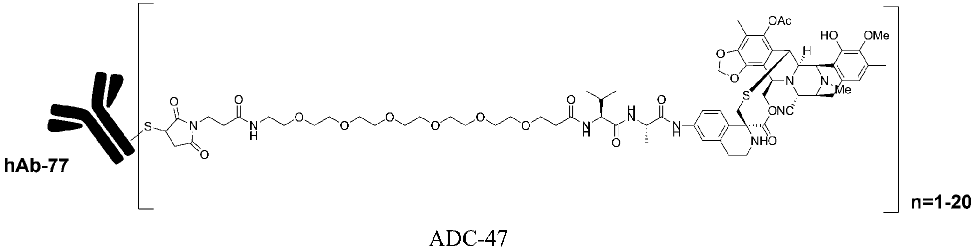

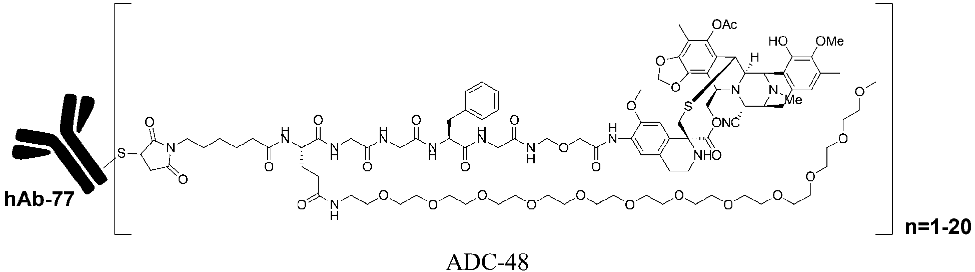

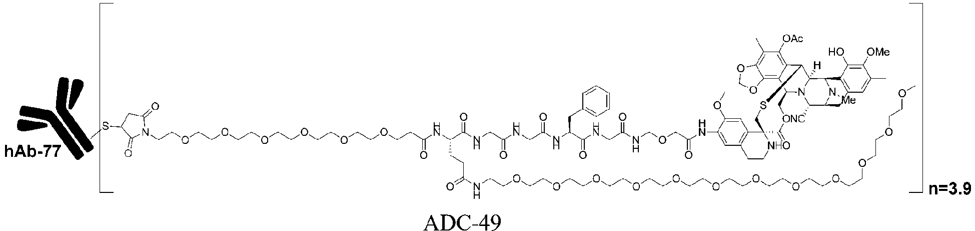

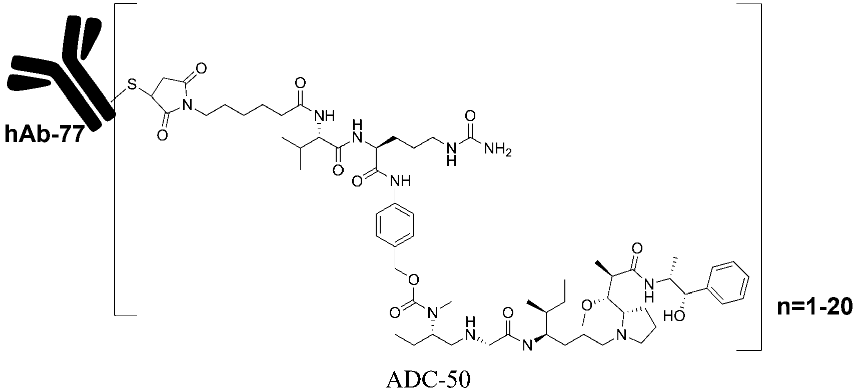

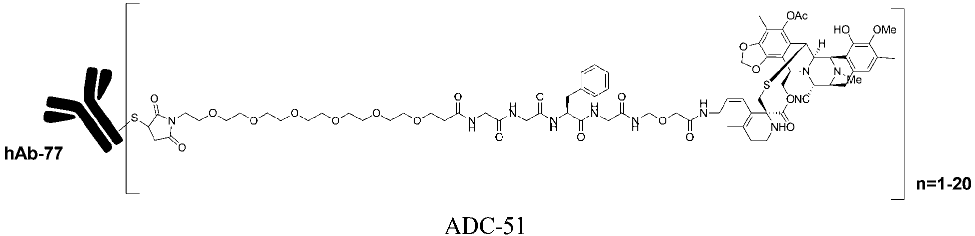

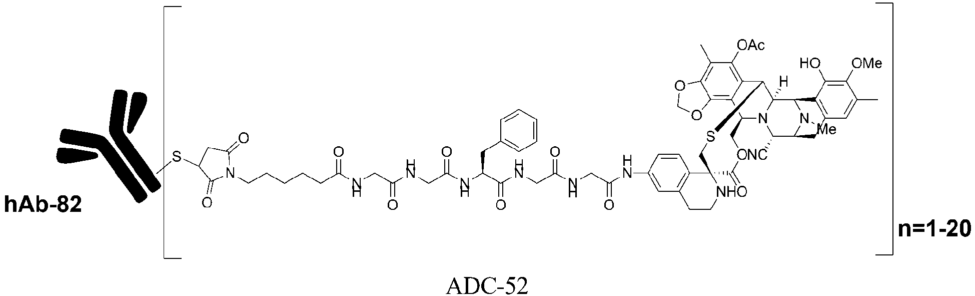

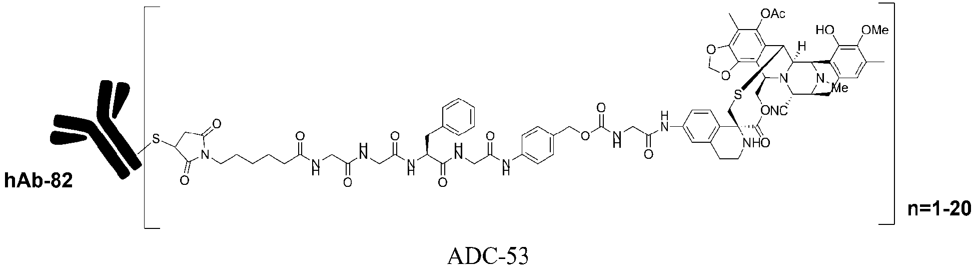

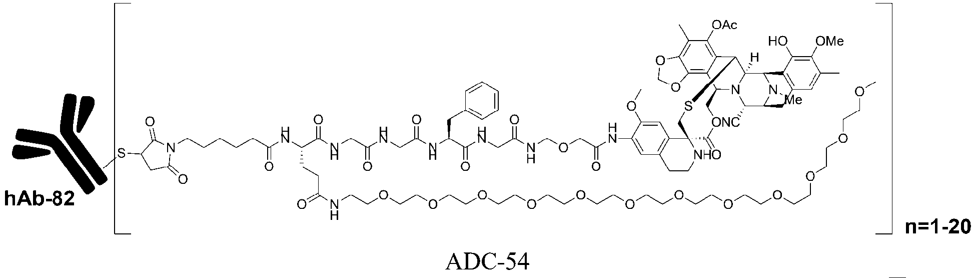

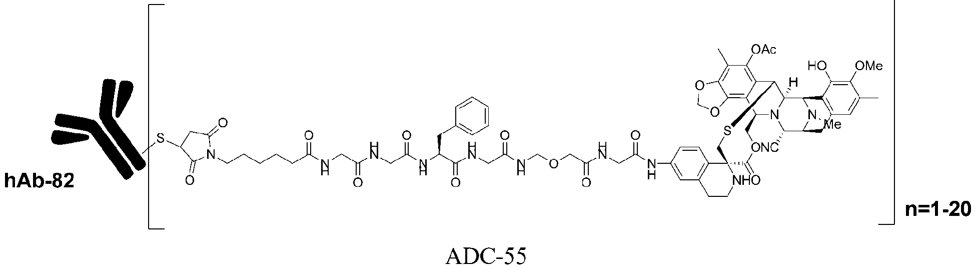

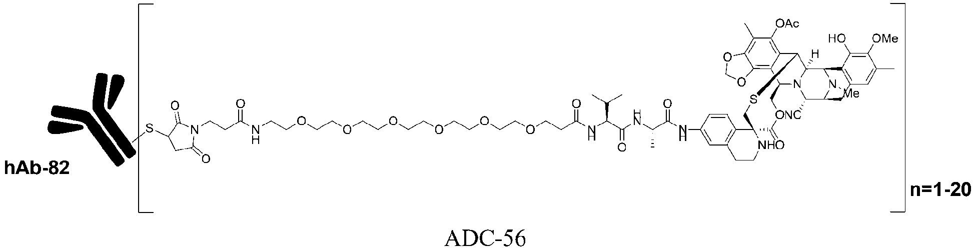

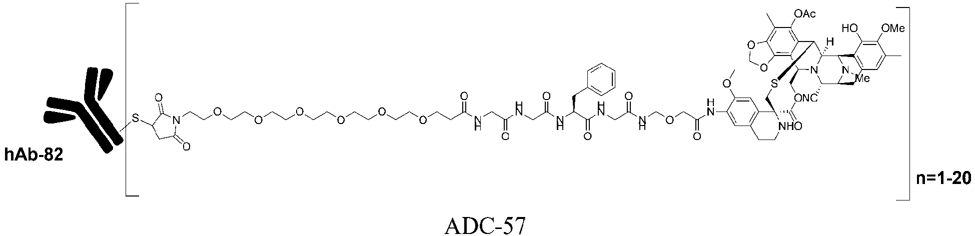

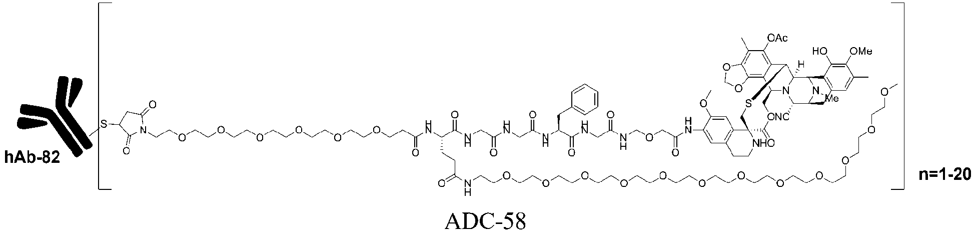

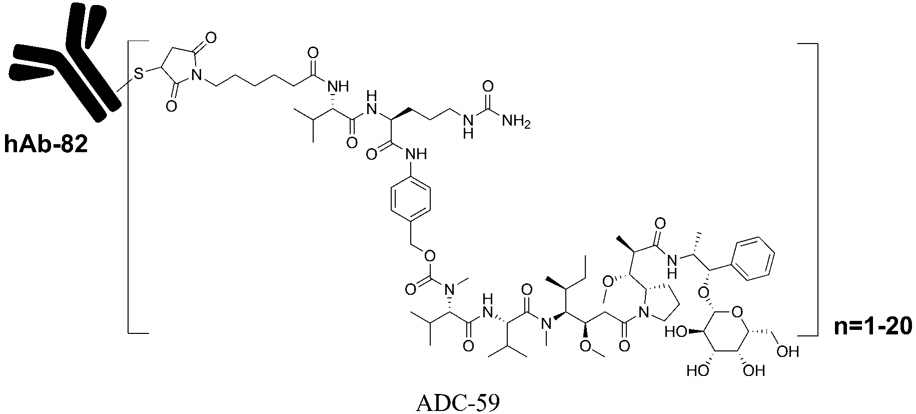

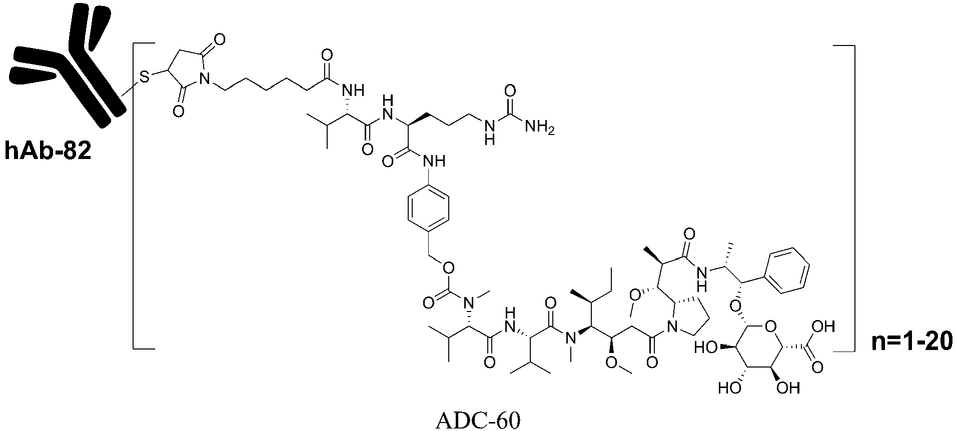

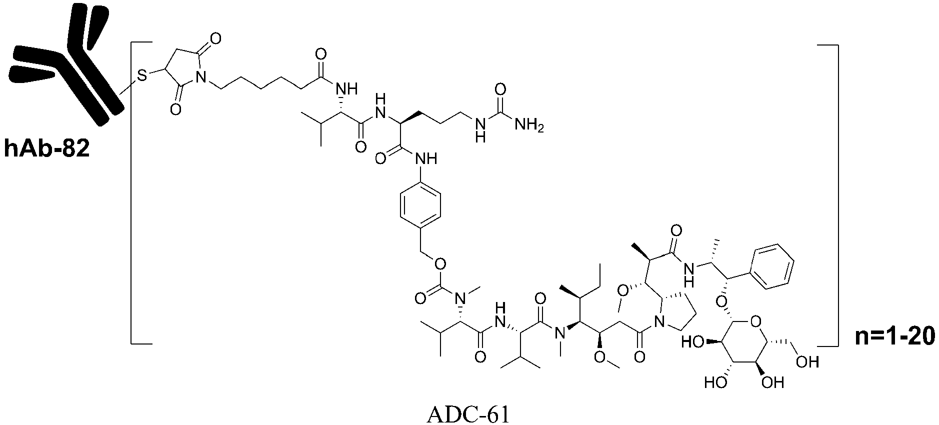

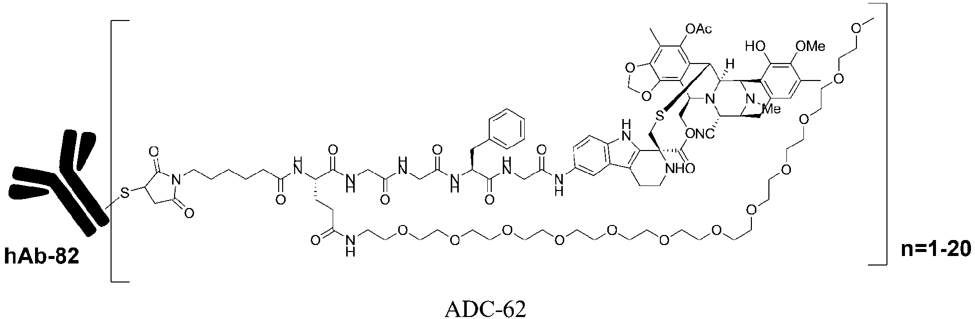

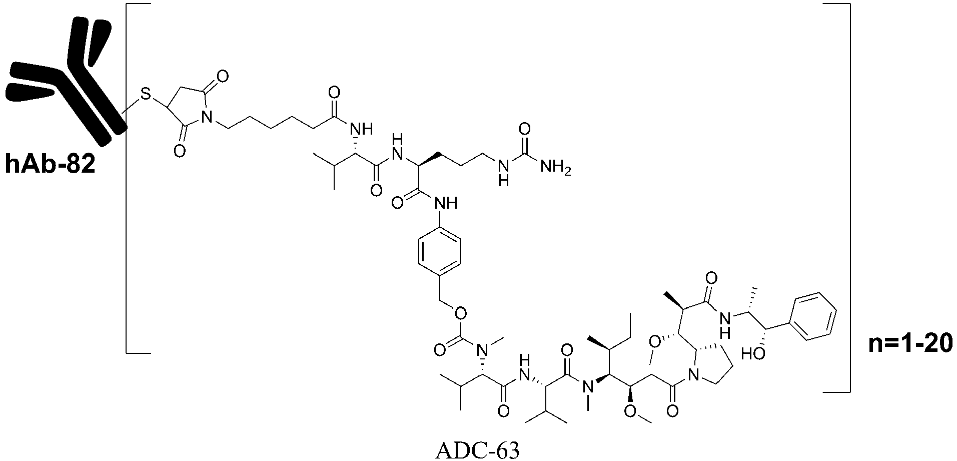

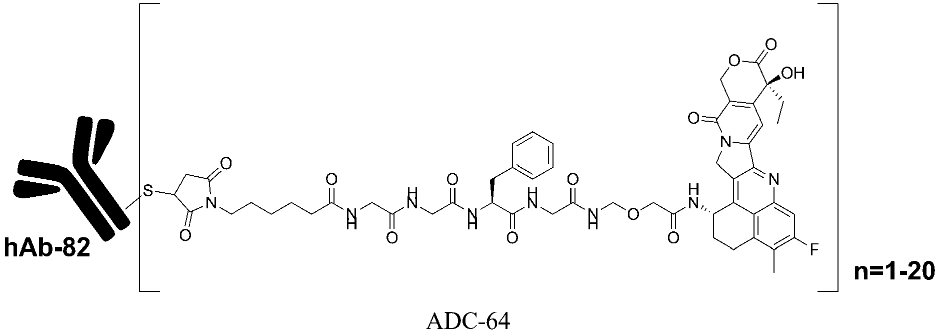

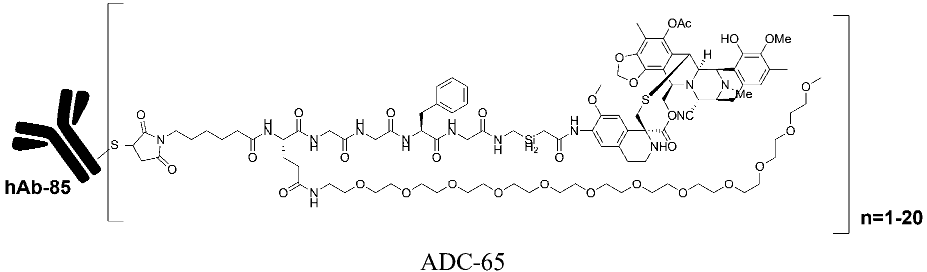

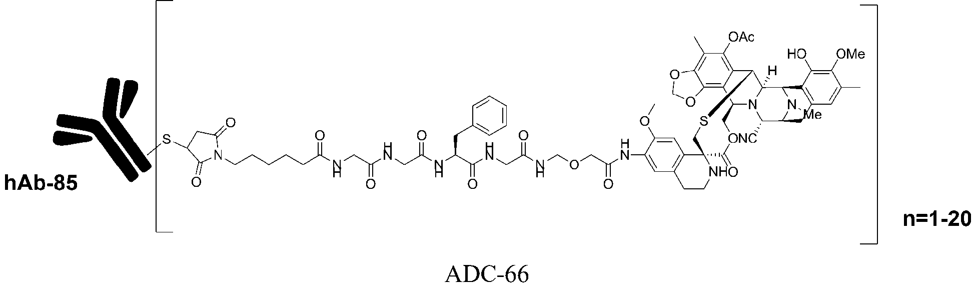

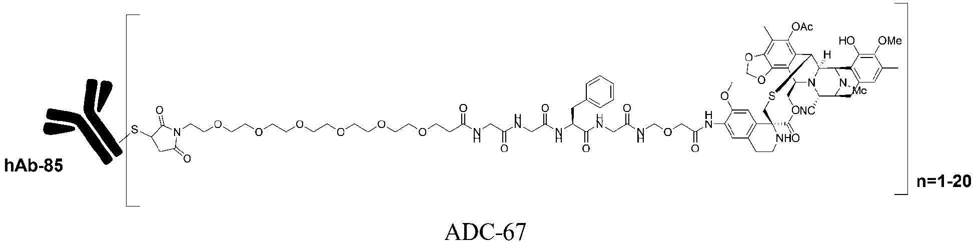

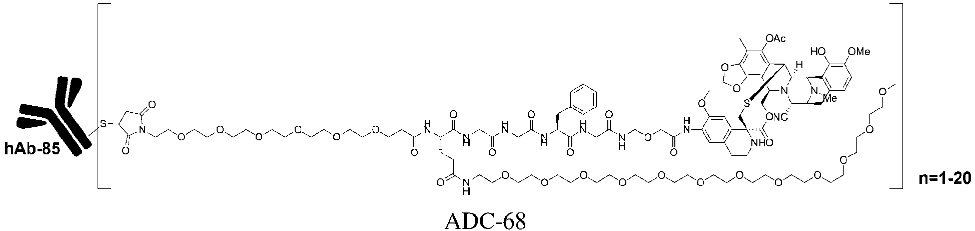

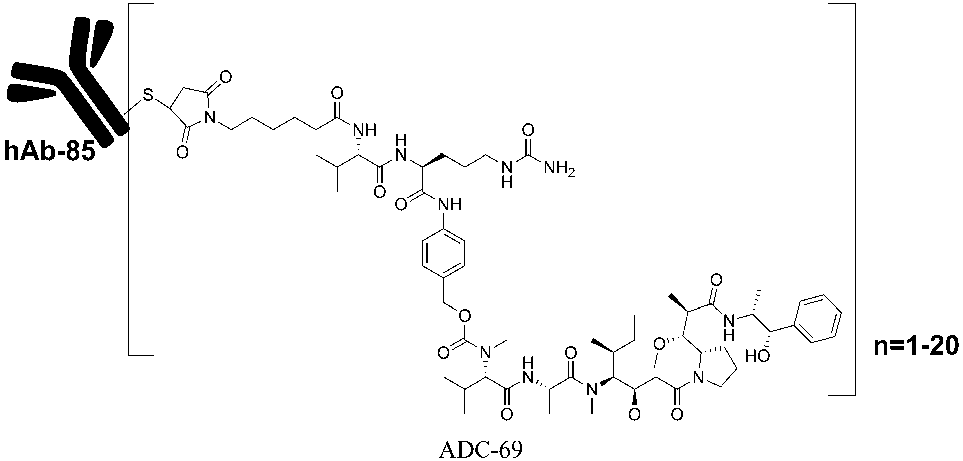

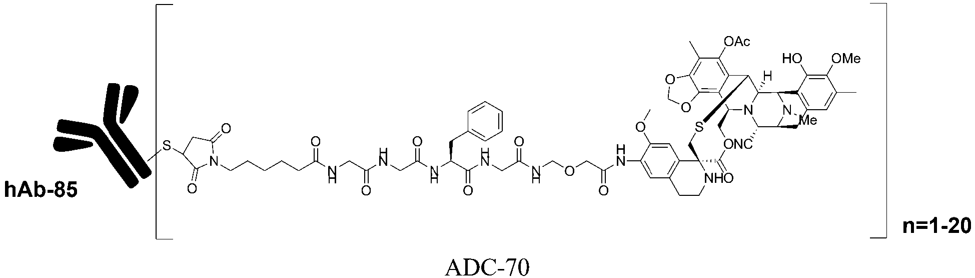

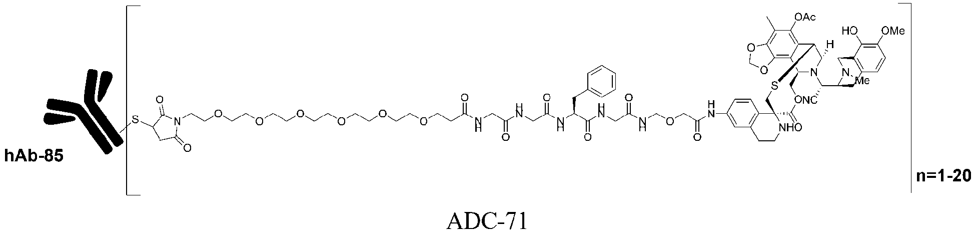

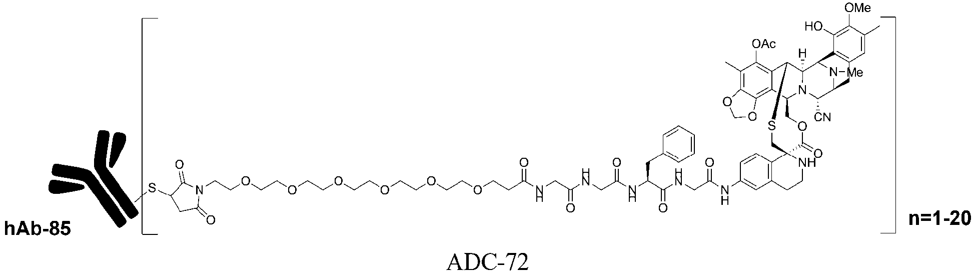

The ADC storage buffer was replaced with 50 mM His-HAC (pH 5.5) and stored at -80°C until use.

### Example 164 In Vitro Cytotoxicity of Antibody-Drug Conjugates Based on Anti-CLDN6 Antibody and Preferred Linker-Payload on CLDN6-Positive Human Ovarian Cancer OV90 Cells

1.5×10³ CLDN6-positive human ovarian cancer OV90 cells were seeded in a 96-well plate (excluding the edge wells filled with PBS). The cells were cultured in 80 µL of DMEM supplemented with 10% FBS (v/v), 100 U/mL penicillin, and 100 mg/mL streptomycin and cultured overnight in a humidified incubator at 37°C and 7.5%CO₂.

After one day of incubation in a thermostatic incubator, the selected ADC was added to each well (20 µL), resulting in a final ADC concentration range of 0.01 nM~1000 nM. After an additional five days of incubation, the 96-well plate was removed from the incubator and equilibrated to room temperature. Approximately 30 minutes later, 40 µL of CellTiter-Glo^{®} (Promega, G7572) was added to each well. The plate was shaken at 450 rpm for 5 minutes and allowed to equilibrate for 10 minutes without shaking before luminescence was measured on a SpectraMax i3x microplate reader. Graphpad Prism software was used to fit a curve of luminescence as a function of ADC concentration (nM). IC50 values were determined using Prism's built-in "log(Inhibitor) vs. Response - Variable Slope (4 Parameters)" function.

Figure 8A and the table below showed the in vitro cytotoxicity of antibody-drug conjugates

| Antibody-drug conjugate | IC50 (nM) |
|---|---|
| ADC-1, DAR2 | 17.86 |
| ADC-1, DAR4 | 4.12 |

| | |
|---|---|
| Note: DAR is the drug to antibody ratio | |

Figure 8B and Figure 8C showed the in vitro cytotoxicity of selected antibody-drug conjugates on OV90 cells.

### Example 165. In Vitro Cytotoxicity of Antibody-Drug Conjugates Based on Anti-CLDN6 Antibodies and Preferred Linker-Loaded on CLDN6-Positive Human Choriocarcinoma JEG-3 Cells

1.5×10³ CLDN6-positive human ovarian cancer JEG-3 cells were seeded in a 96-well plate (excluding the edge wells filled with PBS). The cells were cultured in 80 µL of MEM supplemented with 10% FBS (v/v), 100 U/mL penicillin, 100 mg/mL streptomycin, 1% nonessential amino acids, and 1 mM sodium pyruvate, and incubated overnight in a humidified incubator at 37°C and 7.5% CO₂.

After one day of incubation in a thermostatic incubator, each ADC was added to each well (20 µL), resulting in a final ADC concentration range of 0.01 nM~1000 nM. After an additional five days of incubation, the 96-well plate was removed from the incubator and equilibrated to room temperature. Approximately 30 minutes later, 40 µL of CellTiter Glo^{®} (Promega, G7572) was added to each well. The plate was shaken at 450 rpm for 5 minutes and allowed to equilibrate for 10 minutes without shaking before luminescence was measured on a SpectraMax i3x microplate reader. Graphpad Prism software was used to fit a curve of luminescence as a function of ADC concentration (nM). IC50 values were determined using Prism's built-in "log(Inhibitor) vs. Response - Variable Slope (4 Parameters)" function.

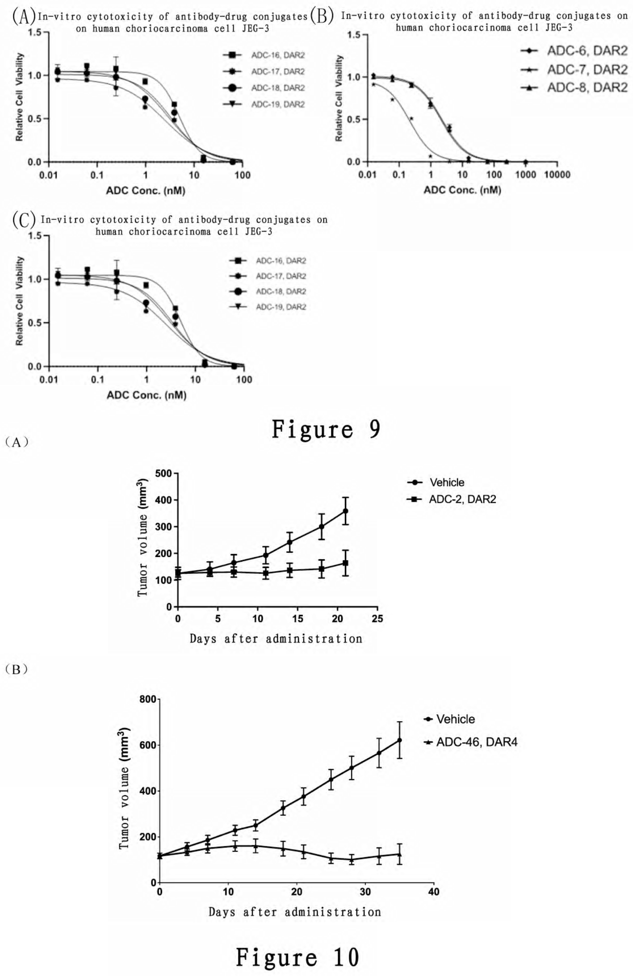

Figure 9 showed the in vitro cytotoxicity of the tested antibody-drug conjugates on JEG3 cells.

### Example 166. Evaluation of the In Vivo Efficacy of Anti-hCLDN6 ADC in an Ovarian Cancer Model Established with CLDN6-Positive Human Ovarian Cancer OV90 Cells

On study day 0, 8-10 week old mice weighing at least 20 g each were inoculated with OV90 tumor cells (5x10⁶ cells/animal in 200 µL PBS). ADCs formulated in PBS were administered (i.v.) at 10 mg/kg to groups of 5 mice on days 17 and 24 post-transplant. PBS served as a negative vehicle control. All treatment groups received weekly tail vein injections for two or three weeks. Tumor volume was measured to determine whether tumor growth was inhibited, delayed, or cured. Tumor size was measured twice weekly with calipers, and the average tumor volume for each group was plotted over time. Volume was expressed in mm³ using the following formula: V = 0.5 a x b², wherein a and b were the long and short diameters of the tumor, respectively. The use and welfare of experimental animals were conducted in accordance with the guidelines of the Association for Assessment and Accreditation of Laboratory Animal Care (AAALAC).

Figure 10 showed the efficacy of ADC-2, DAR2 administered for two weeks (A) and ADC-46, DAR 4 administered for three weeks (B) in a xenograft model in female BALB/C nude mice injected with hCLDN6-positive OV90 cells.

The Examples of the present invention are only used to illustrate the present invention and do not limit the scope of the claims. Other substantially equivalent alternatives that can be thought of by those skilled in the art are all within the scope of protection of the present invention.

## Claims

1. An isolated antigen-binding protein capable of binding to hCLDN6, **characterized by** comprising a heavy chain variable region, wherein the heavy chain variable region has:
HCDR1 with an amino acid sequence of X₁YTMS,
HCDR2 with an amino acid sequence of TISSGGGX₂TYYPDSVKG, X₂=R, N, Q, D or E,
HCDR3 with an amino acid sequence of GDX₄RYDX₃FAY, X₄=Y, N or Q,
wherein,
(1) X₁=Y, M, Q, D or E, X₃=G, or (2) X₁=S or D, X₃=A.

2. The antigen-binding protein according to claim 1, **characterized in that** the heavy chain variable region has a human heavy chain framework region, wherein the human heavy chain framework region has back mutation(s) at one or more sites relative to the murine heavy chain framework region, wherein the back mutation site in the heavy chain variable region includes R44, and the position numbering is determined by the Kabat numbering scheme.

3. The antigen-binding protein according to claim 2, **characterized in that** the heavy chain variable region is a sequence having 92.59% or more identity with HFR1 to HCDR3; having said back mutation site; and having said X₁, X₂, X₃ and/or X₄ sites.

4. The antigen-binding protein according to claim 3, **characterized in that** the heavy chain variable region has a sequence shown in any one of SEQ ID Nos. 22, 23, 24, 59, 60, 61, 62, 63, 66, 67, 133 to 153.

5. The antigen-binding protein according to claim 2, **characterized in that** the heavy chain framework region is a human IgG heavy chain framework region.

6. The antigen-binding protein according to claim 1, **characterized by** further comprising a light chain variable region, wherein the light chain variable region has:
LCDR1 with an amino acid sequence of RASENIDSX₅LA, X₅=Y, Q, or R,
LCDR2 with an amino acid sequence of ASTLLVD,
LCDR3 with an amino acid sequence of QHYYSX₆PYT, X₆=I or E.

7. The antigen-binding protein according to claim 6, **characterized in that** the light chain variable region has a human light chain framework region, and the light chain framework region has back mutation(s) at one or more sites relative to the murine light chain framework region, wherein the back mutation site in the light chain framework region include one or more of R66, Q70, and R85, and the position numbering is determined by the Kabat numbering scheme.

8. The antigen-binding protein according to claim 7, **characterized in that** the light chain variable region is a sequence having 95.88% or more identity with HFR1 to HCDR3; having said back mutation site; and having said X₅ and/or X₆ sites.

9. The antigen-binding protein according to claim 8, **characterized in that** the light chain variable region has a sequence shown in any one of SEQ ID Nos. 25, 28, 154, 155, and 156.

10. The antigen-binding protein according to claim 7, **characterized in that** the light chain framework region is a human IgG light chain framework region.

11. The antigen-binding protein according to claim 6, **characterized by** comprising a human heavy chain constant region and a human light chain constant region.

12. The antigen-binding protein according to claim 1, **characterized by** comprising a human heavy chain constant region.

13. The antigen-binding protein according to claim 11 or 12, **characterized in that** the heavy chain constant region is a human IgG1 heavy chain constant region and comprises one or more mutations selected from the group consisting of C220S, A121C, V205C, K149C, D265C, S239C, A330C, and S442C.

14. The antigen-binding protein according to claim 11 or 12, **characterized in that** the amino acid sequence of the heavy chain constant region is as shown in SEQ ID No.9.

15. The antigen-binding protein according to claim 11, **characterized in that** the amino acid sequence of the light chain constant region is as shown in SEQ ID No.10.

16. The antigen-binding protein according to claim 1, **characterized in that** the antigen-binding protein comprises an antibody or an antigen-binding fragment thereof.

17. The antigen-binding protein according to claim 2, **characterized by** having a heavy chain with the sequence as shown in any one of SEQ ID Nos. 157, 159 to 167.

18. The antigen-binding protein according to claim 2, **characterized by** having a heavy chain with the sequence as shown in any one of SEQ ID Nos. 158 and 171.

19. The antigen-binding protein according to claim 7, **characterized by** having a light chain with the sequence as shown in any one of SEQ ID Nos. 169 and 170.

20. The antigen-binding protein according to claim 7, **characterized by** having a light chain with the sequence as shown in SEQ ID No. 168.

21. The antigen-binding protein according to claim 1, **characterized in that** the antigen-binding protein is antibody hAb-13: comprising a heavy chain variable region with the sequence as shown in SEQ ID No. 18, and a light chain variable region as shown in SEQ ID No. 28.

22. The antigen-binding protein according to claim 1, **characterized in that** it is selected from:
Antibody hAb-26: comprising a heavy chain variable region with the sequence of SEQ ID No. 59, and a light chain variable region with the sequence of SEQ ID No. 28; or
Antibody hAb-27: comprising a heavy chain variable region with the sequence of SEQ ID No. 60, and a light chain variable region with the sequence of SEQ ID No. 28; or
Antibody hAb-28: comprising a heavy chain variable region with the sequence of SEQ ID No. 61, and a light chain variable region with the sequence of SEQ ID No. 28; or
Antibody hAb-29: comprising a heavy chain variable region with the sequence of SEQ ID No. 62, and a light chain variable region with the sequence of SEQ ID No. 28; or
Antibody hAb-30: comprising a heavy chain variable region with the sequence of SEQ ID No. 63, and a light chain variable region with the sequence of SEQ ID No. 28; or
Antibody hAb-33: comprising a heavy chain variable region with the sequence of SEQ ID No. 66, and a light chain variable region with the sequence of SEQ ID No. 28; or
Antibody hAb-34: comprising a heavy chain variable region with the sequence of SEQ ID No. 67, and a light chain variable region with the sequence of SEQ ID No. 28; or
Antibody hAb-44: comprising a heavy chain variable region with the sequence of SEQ ID No. 22, and a light chain variable region with the sequence of SEQ ID No. 25; or
Antibody hAb-45: comprising a heavy chain variable region with the sequence of SEQ ID No. 23, and a light chain variable region with the sequence of SEQ ID No. 25; or
Antibody hAb-46: comprising a heavy chain variable region with the sequence of SEQ ID No. 24, and a light chain variable region with the sequence of SEQ ID No. 28; or
Antibody hAb-47: comprising a heavy chain variable region with the sequence of SEQ ID No. 22, and a light chain variable region with the sequence of SEQ ID No. 28; or
Antibody hAb-48: comprising a heavy chain variable region with the sequence of SEQ ID No. 23, and a light chain variable region with the sequence of SEQ ID No. 28; or
Antibody hAb-49: comprising a heavy chain variable region with the sequence of SEQ ID No. 24, and a light chain variable region with the sequence of SEQ ID No. 28; or
Antibody hAb-50: comprising a heavy chain variable region with the sequence of SEQ ID No. 133, and a light chain variable region with the sequence of SEQ ID No. 28; or
Antibody hAb-51: comprising a heavy chain variable region with the sequence of SEQ ID No. 134, and a light chain variable region with the sequence of SEQ ID No. 28; or
Antibody hAb-52: comprising a heavy chain variable region with the sequence of SEQ ID No. 135, and a light chain variable region with the sequence of SEQ ID No. 28; or
Antibody hAb-53: comprising a heavy chain variable region with the sequence of SEQ ID No. 136, and a light chain variable region with the sequence of SEQ ID No. 28; or
Antibody hAb-54: comprising a heavy chain variable region with the sequence of SEQ ID No. 137, and a light chain variable region with the sequence of SEQ ID No. 28; or
Antibody hAb-55: comprising a heavy chain variable region with the sequence of SEQ ID No. 138, and a light chain variable region with the sequence of SEQ ID No. 28; or
Antibody hAb-56: comprising a heavy chain variable region with the sequence of SEQ ID No. 139, and a light chain variable region with the sequence of SEQ ID No. 28; or
Antibody hAb-57: comprising a heavy chain variable region with the sequence of SEQ ID No. 140, and a light chain variable region with the sequence of SEQ ID No. 28; or
Antibody hAb-58: comprising a heavy chain variable region with the sequence of SEQ ID No. 141, and a light chain variable region with the sequence of SEQ ID No. 28; or
Antibody hAb-59: comprising a heavy chain variable region with the sequence of SEQ ID No. 142, and a light chain variable region with the sequence of SEQ ID No. 28; or
Antibody hAb-60: comprising a heavy chain variable region with the sequence of SEQ ID No. 143, and a light chain variable region with the sequence of SEQ ID No. 28; or
Antibody hAb-61: comprising a heavy chain variable region with the sequence of SEQ ID No. 144, and a light chain variable region with the sequence of SEQ ID No. 28; or
Antibody hAb-62: comprising a heavy chain variable region with the sequence of SEQ ID No. 145, and a light chain variable region with the sequence of SEQ ID No. 28; or
Antibody hAb-63: comprising a heavy chain variable region with the sequence of SEQ ID No. 146, and a light chain variable region with the sequence of SEQ ID No. 28; or
Antibody hAb-64: comprising a heavy chain variable region with the sequence of SEQ ID No. 147, and a light chain variable region with the sequence of SEQ ID No. 28; or
Antibody hAb-65: comprising a heavy chain variable region with the sequence of SEQ ID No. 148, and a light chain variable region with the sequence of SEQ ID No. 28; or
Antibody hAb-66: comprising a heavy chain variable region with the sequence of SEQ ID No. 149, and a light chain variable region with the sequence of SEQ ID No. 28; or
Antibody hAb-67: comprising a heavy chain variable region with the sequence of SEQ ID No. 150, and a light chain variable region with the sequence of SEQ ID No. 28; or
Antibody hAb-68: comprising a heavy chain variable region with the sequence of SEQ ID No. 151, and a light chain variable region with the sequence of SEQ ID No. 28; or
Antibody hAb-69: comprising a heavy chain variable region with the sequence of SEQ ID No. 152, and a light chain variable region with the sequence of SEQ ID No. 28; or
Antibody hAb-70: comprising a heavy chain variable region with the sequence of SEQ ID No. 153, and a light chain variable region with the sequence of SEQ ID No. 28; or
Antibody hAb-71: comprising a heavy chain variable region with the sequence of SEQ ID No. 24, and a light chain variable region with the sequence of SEQ ID No. 154; or
Antibody hAb-72: comprising a heavy chain variable region with the sequence of SEQ ID No. 24, and a light chain variable region with the sequence of SEQ ID No. 155; or
Antibody hAb-73: comprising a heavy chain variable region with the sequence of SEQ ID No. 24, and a light chain variable region with the sequence of SEQ ID No. 156; or
Antibody hAb-74: comprising a heavy chain with the sequence of SEQ ID No. 157, and a light chain with the sequence of SEQ ID No. 168; or
Antibody hAb-75: comprising a heavy chain with the sequence of SEQ ID No. 158, and a light chain with the sequence of SEQ ID No. 169; or
Antibody hAb-76: comprising a heavy chain with the sequence of SEQ ID No. 159, and a light chain with the sequence of SEQ ID No. 170; or
Antibody hAb-77: comprising a heavy chain with the sequence of SEQ ID No. 160, and a light chain with the sequence of SEQ ID No. 169; or
Antibody hAb-78: comprising a heavy chain with the sequence of SEQ ID No. 161, and a light chain with the sequence of SEQ ID No. 169; or
Antibody hAb-79: comprising a heavy chain with the sequence of SEQ ID No. 162, and a light chain with the sequence of SEQ ID No. 169; or
Antibody hAb-80: comprising a heavy chain with the sequence of SEQ ID No. 163, and a light chain with the sequence of SEQ ID No. 169; or
Antibody hAb-81: comprising a heavy chain with the sequence of SEQ ID No. 164, and a light chain with the sequence of SEQ ID No. 169; or
Antibody hAb-82: comprising a heavy chain with the sequence of SEQ ID No. 165, and a light chain with the sequence of SEQ ID No. 169; or
Antibody hAb-83: comprising a heavy chain with the sequence of SEQ ID No. 166, and a light chain with the sequence of SEQ ID No. 169; or
Antibody hAb-84: comprising a heavy chain with the sequence of SEQ ID No. 167, and a light chain with the sequence of SEQ ID No. 169; or
Antibody hAb-85: comprising a heavy chain with the sequence of SEQ ID No. 171, and a light chain with the sequence of SEQ ID No. 169.

23. A nucleic acid molecule, **characterized in that** it is used to encode the antigen-binding protein according to any one of claims 1 to 22.

24. A vector, **characterized in that** it comprises the nucleic acid molecule according to claim 23.

25. A cell comprising the nucleic acid molecule according to claim 23, and/or the vector according to claim 24.

26. A pharmaceutical molecule **characterized by** comprising the antigen-binding protein according to any one of claims 1 to 22.

27. The pharmaceutical molecule according to claim 26, **characterized by** having a structure as shown in Formula I: 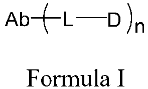 Wherein Ab is the antigen-binding protein, L is a linker comprising one or more connectors, D is a therapeutic agent or a detectable marker, and n is an integer selected from 1-20.

28. The pharmaceutical molecule according to claim 27, **characterized in that** n is an integer selected from 2-8.

29. The pharmaceutical molecule according to claim 27, **characterized in that** the therapeutic agent is selected from an immunomodulator, a cytotoxic agent, a cytostatic agent, a radioisotope, an anti-angiogenic agent or a liposome.

30. The pharmaceutical molecule according to claim 29, **characterized in that** the cytotoxic agent is selected from one or more of maytansine alkaloids, auristatins, eribulin, taxanes, calicheamicin, cemadotin, pyrrolobenzodiazepines, anthracyclines, camptothecin derivatives, α -amanitin and derivatives thereof, trabectedin and derivatives thereof, and lurbinectidin and derivatives thereof.

31. The pharmaceutical molecule according to claim 27, **characterized in that** the connector is selected from one or more of an oligopeptide connector, a hydrazine connector, a thiourea connector, a triggered self-immolative connector, a succinimidyl trans-4-(maleimidomethyl) cyclohexane-1-carboxylate connector, a maleimide connector, a disulphide connector, a thioether connector, and an olefin connector.

32. The pharmaceutical molecule according to claim 27, **characterized in that** it is selected from any one of ADCs 1~72:

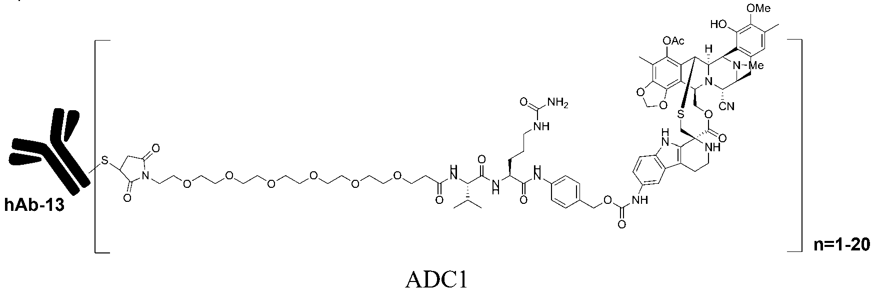

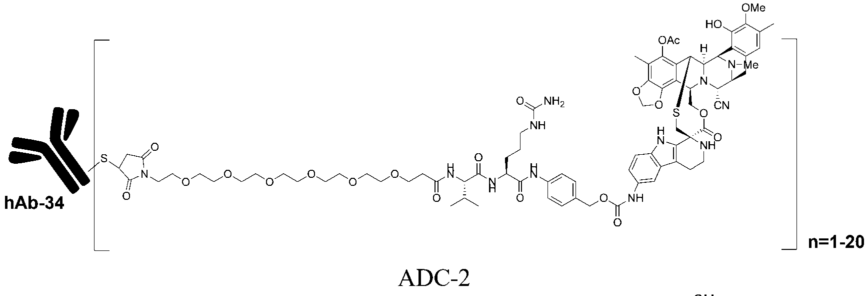

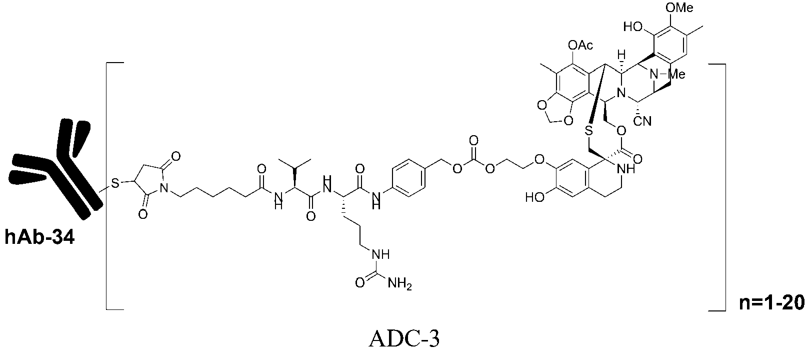

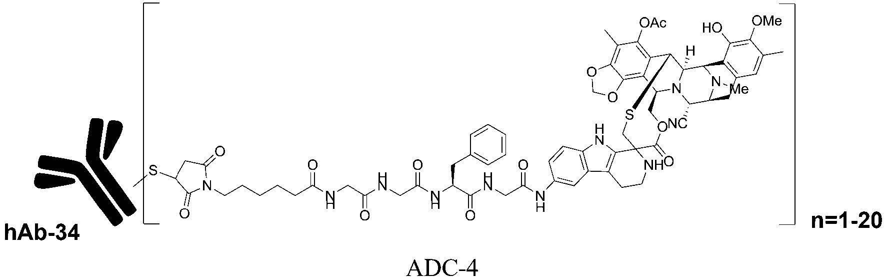

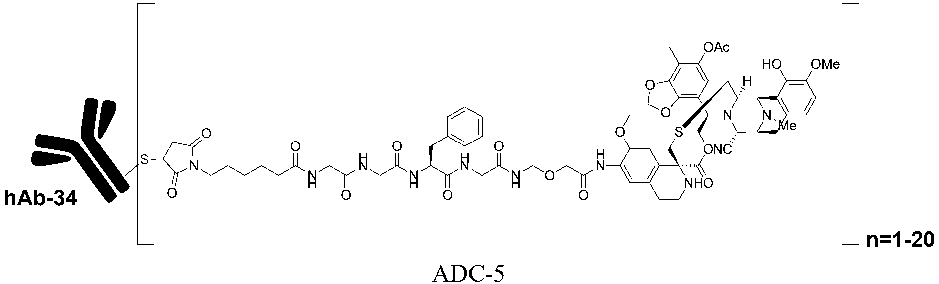

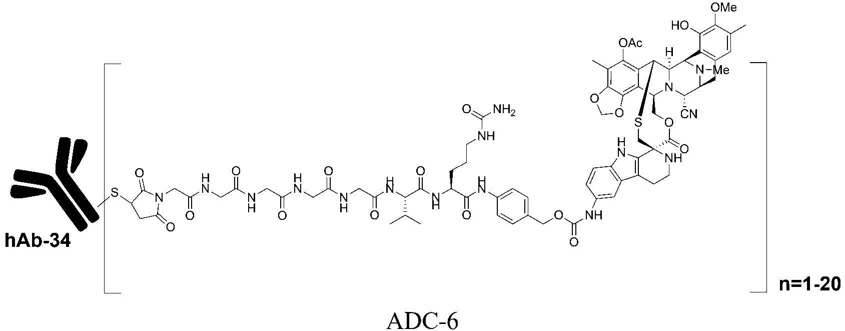

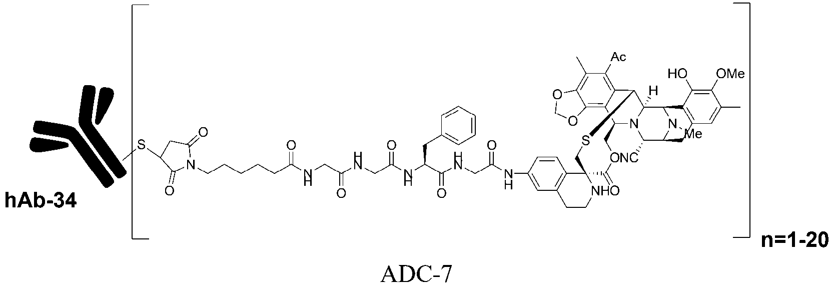

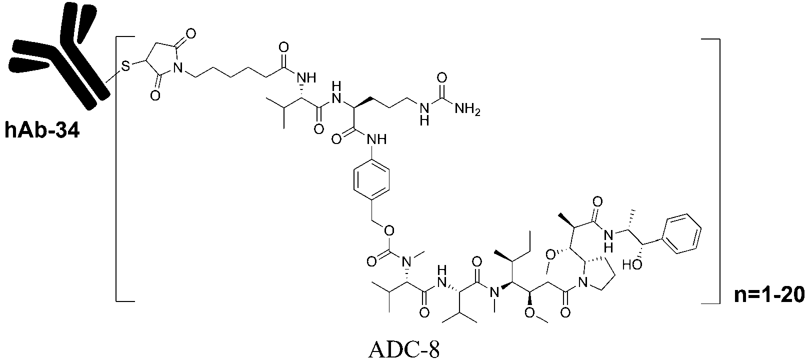

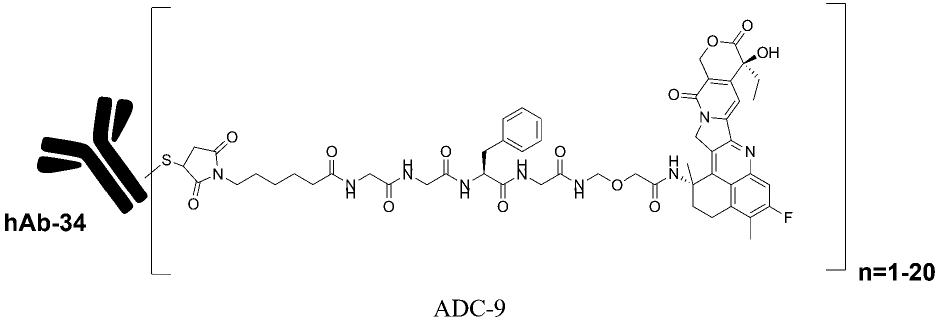

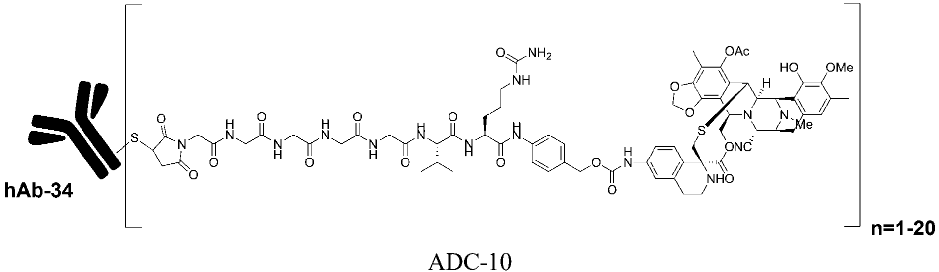

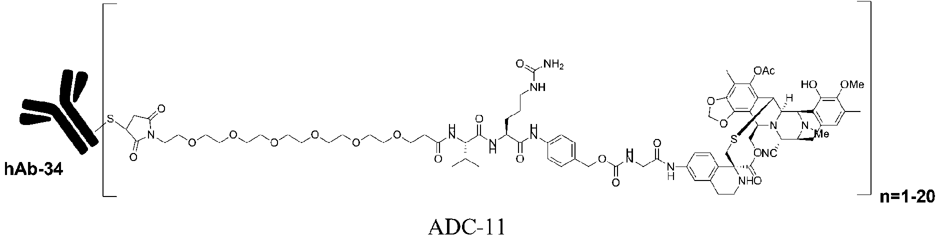

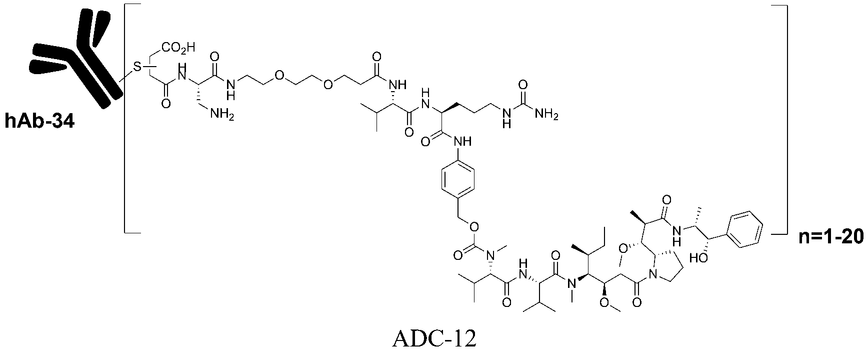

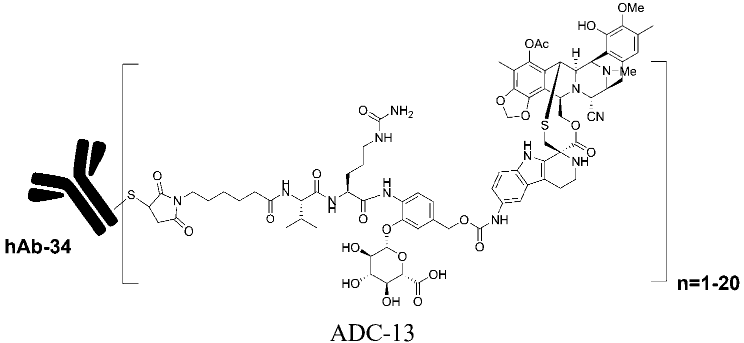

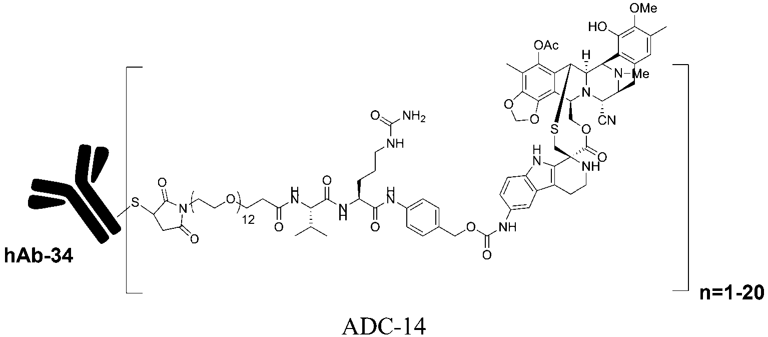

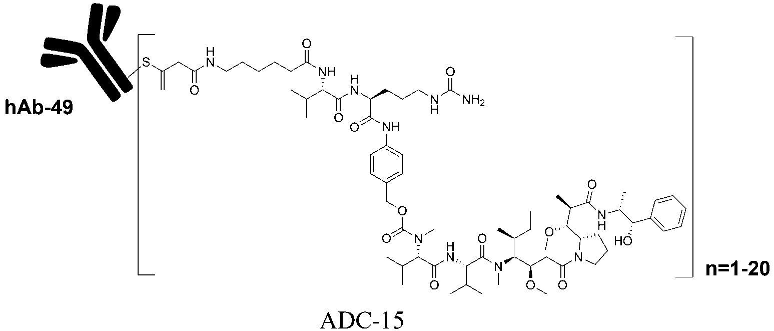

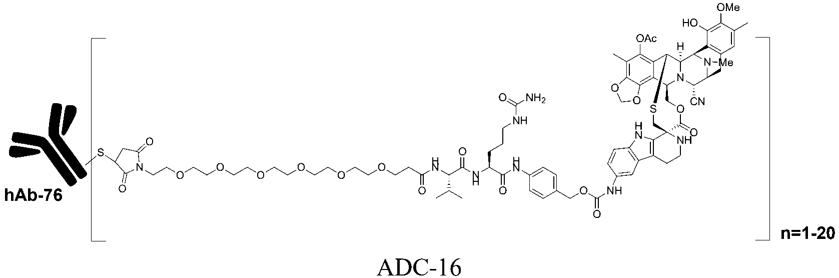

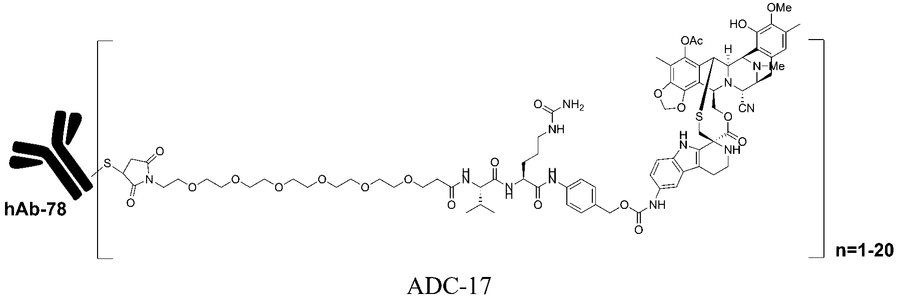

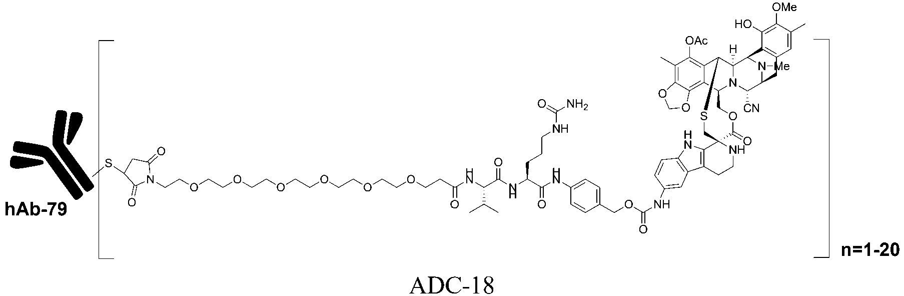

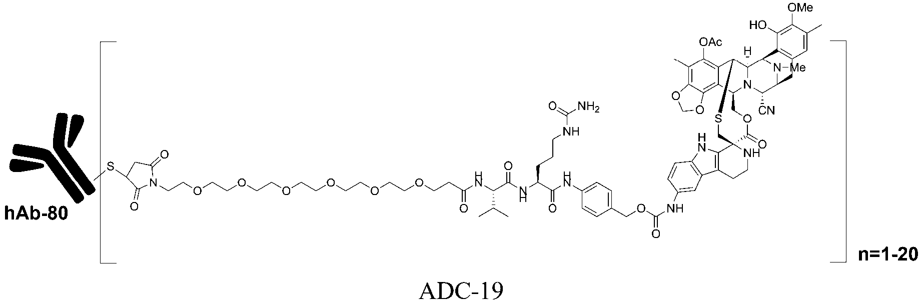

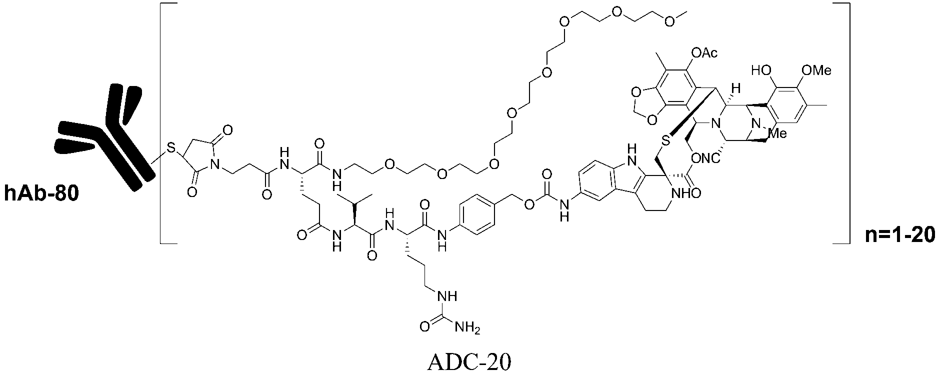

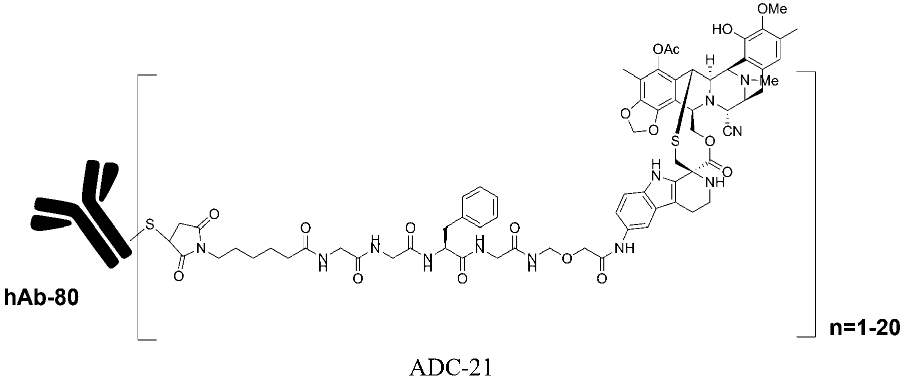

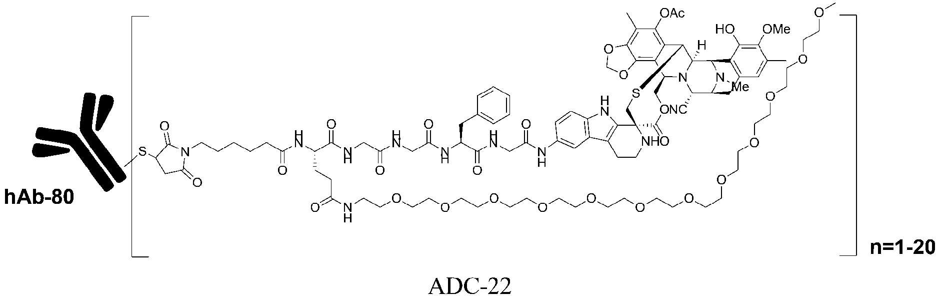

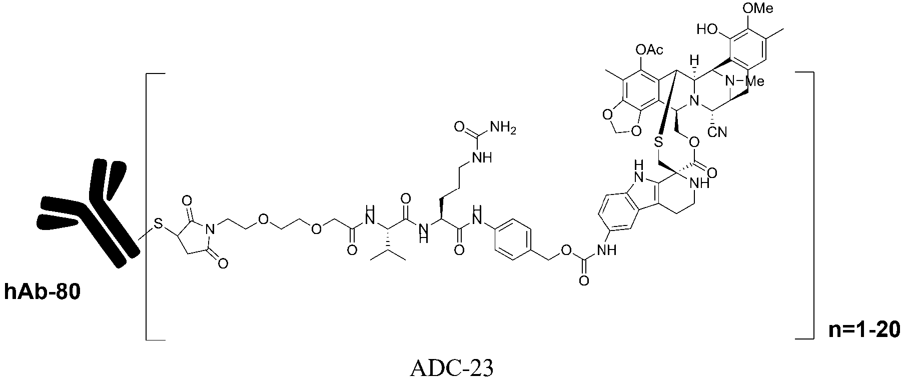

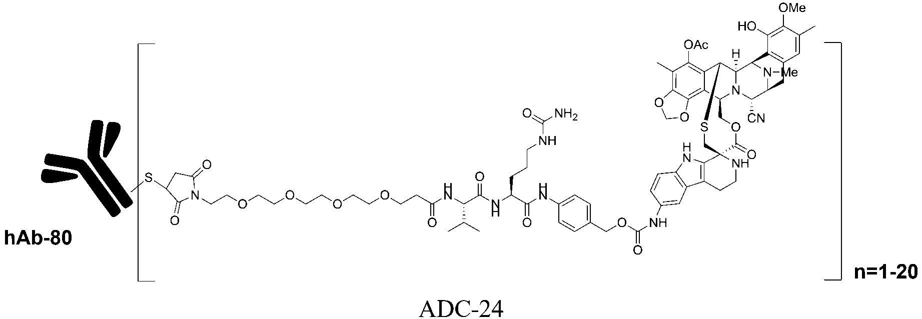

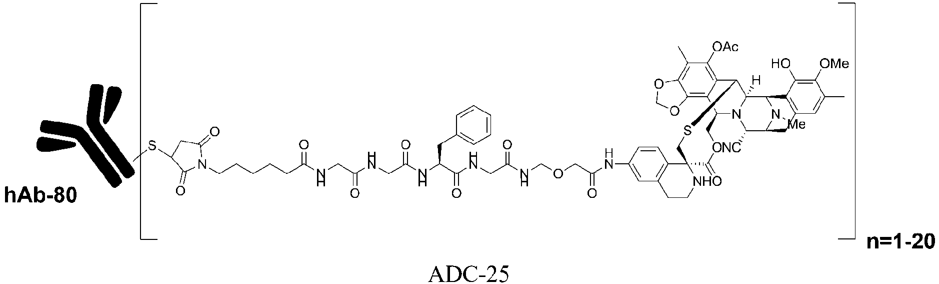

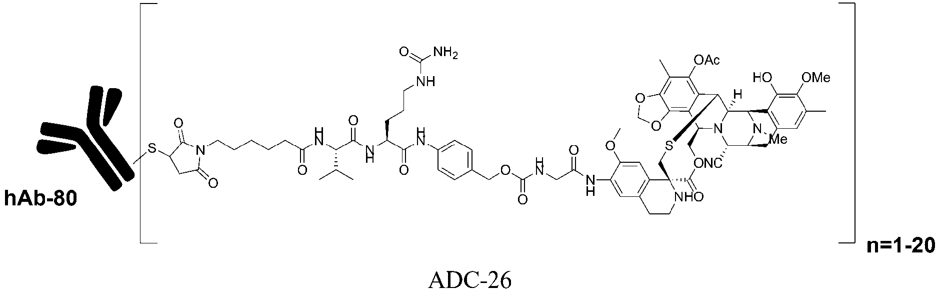

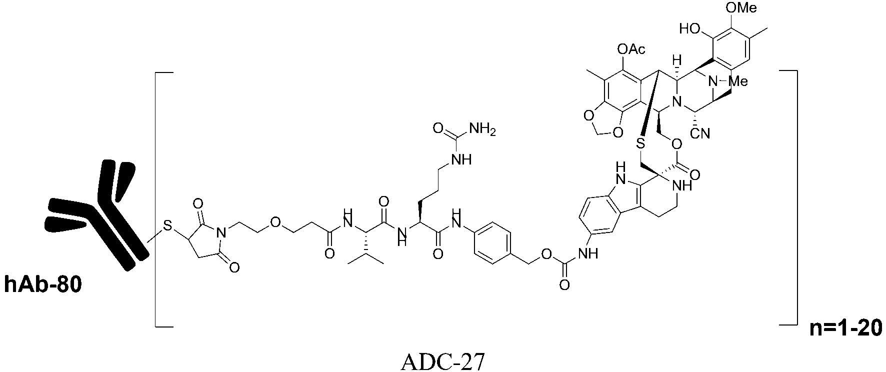

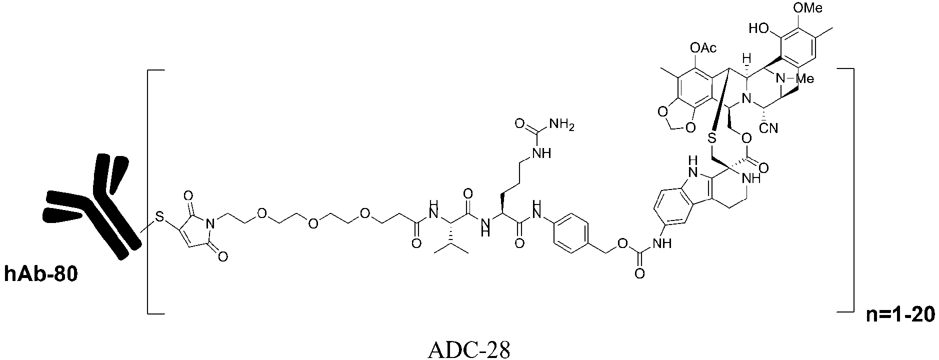

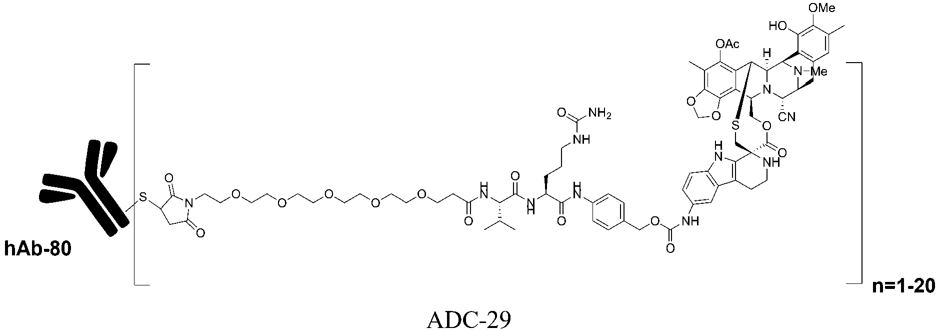

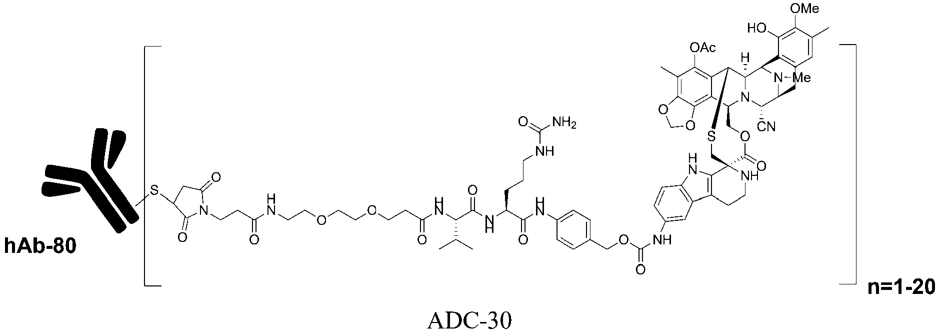

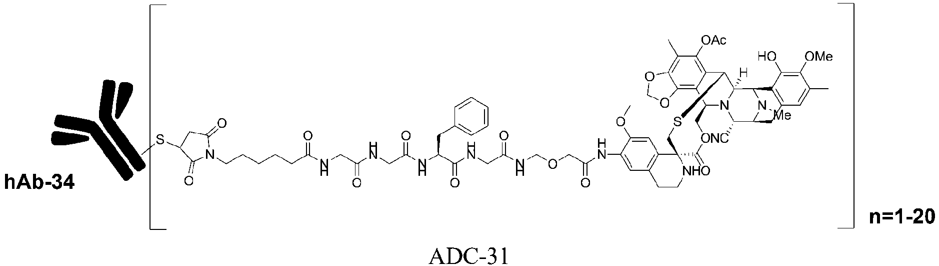

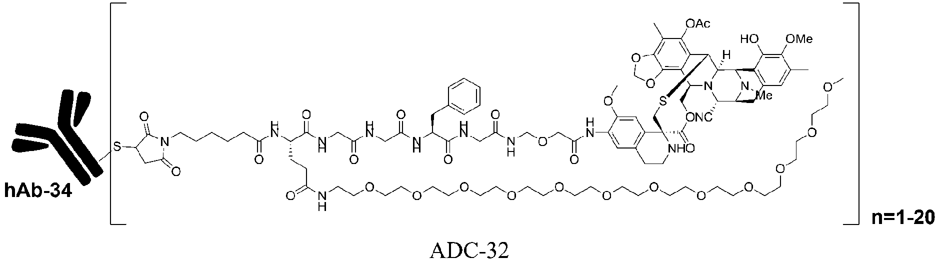

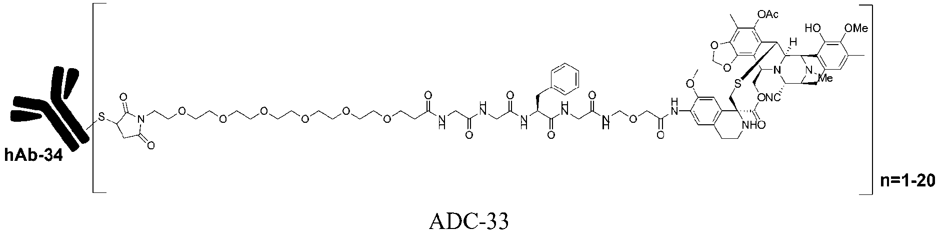

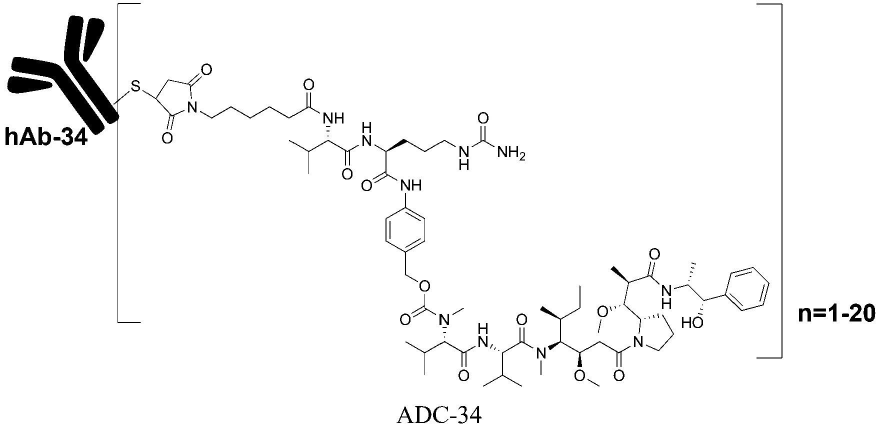

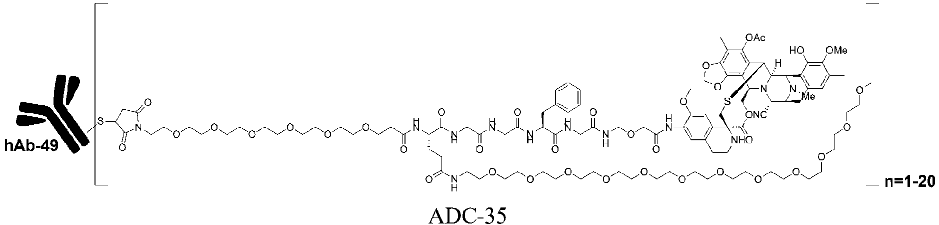

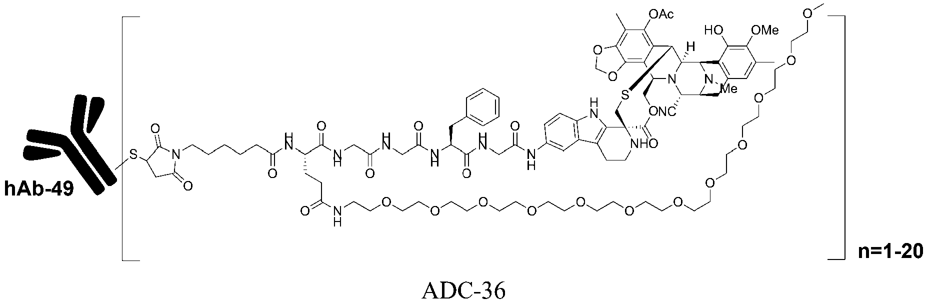

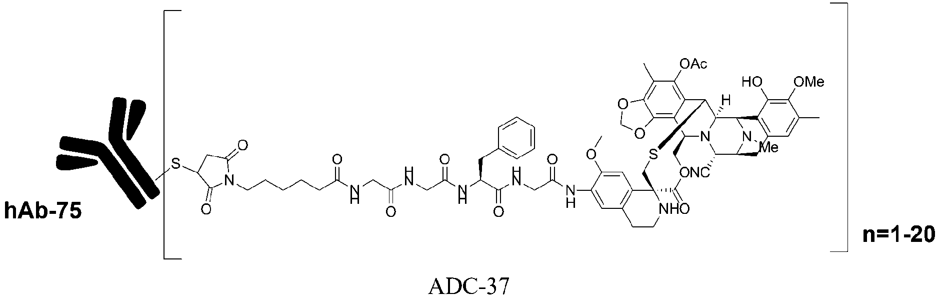

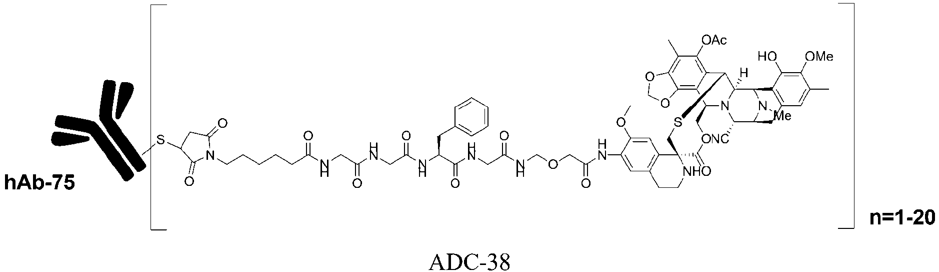

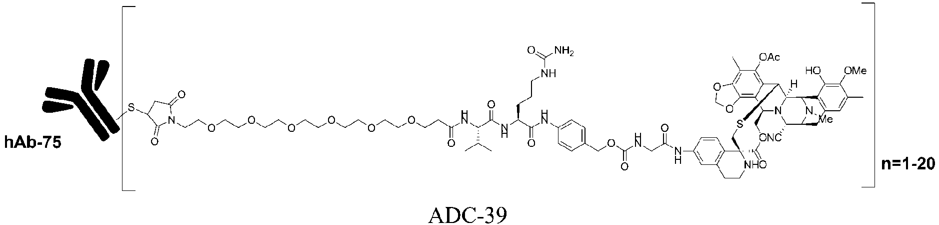

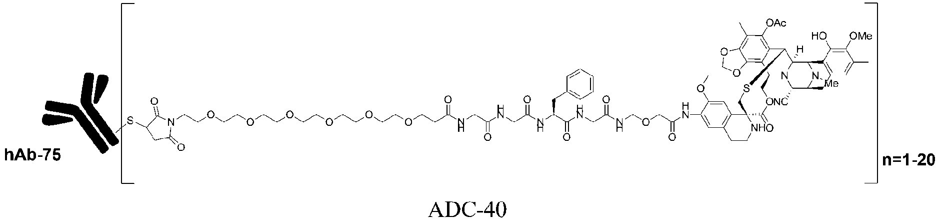

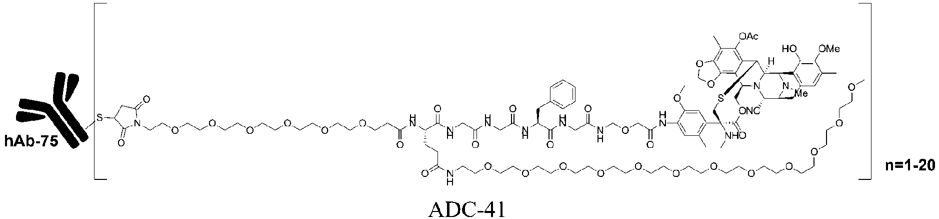

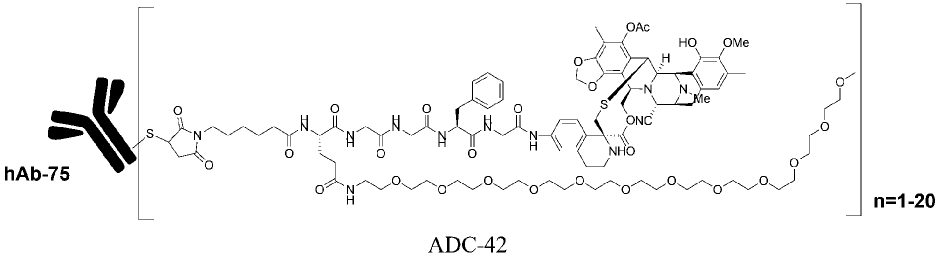

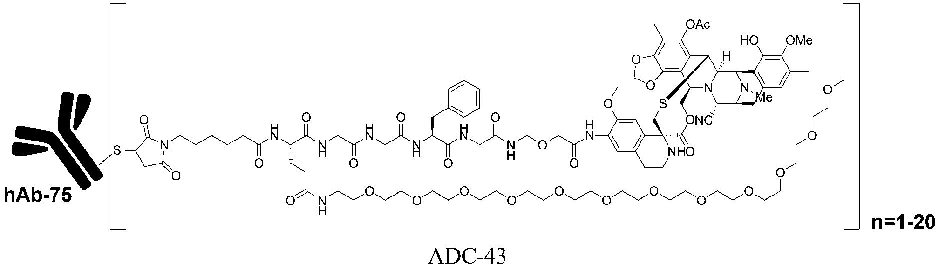

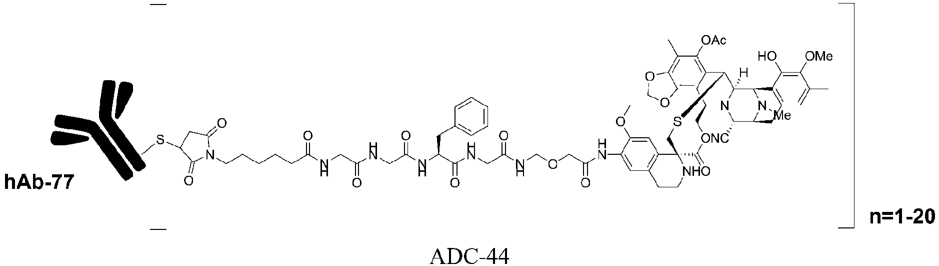

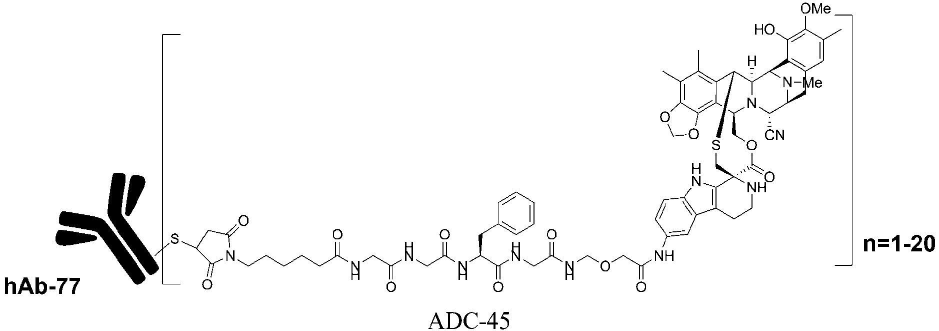

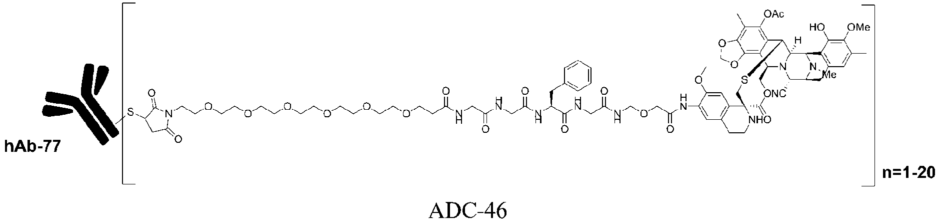

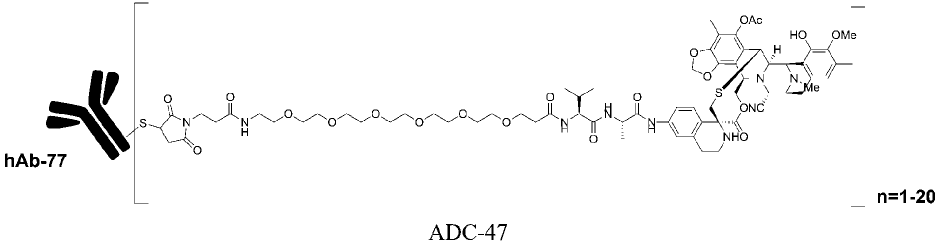

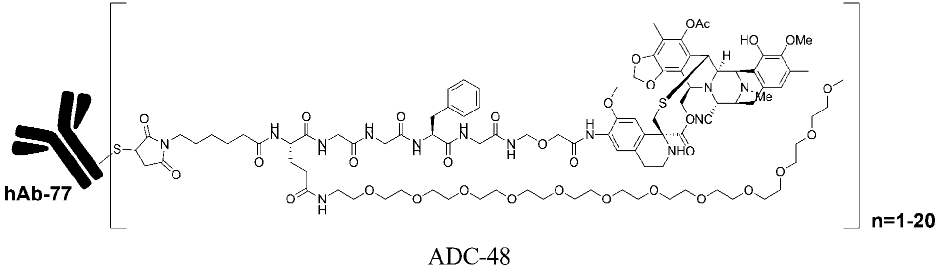

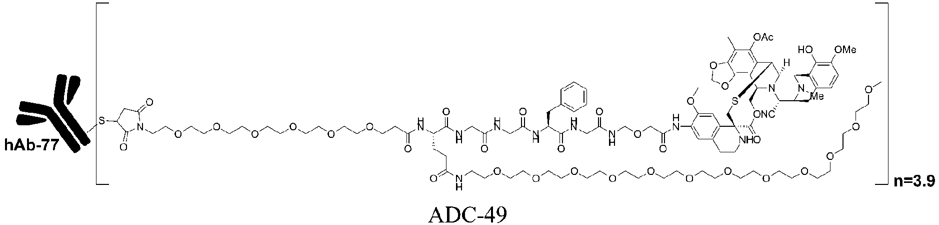

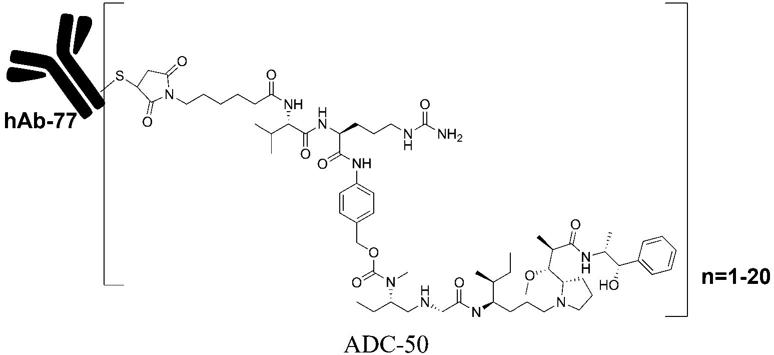

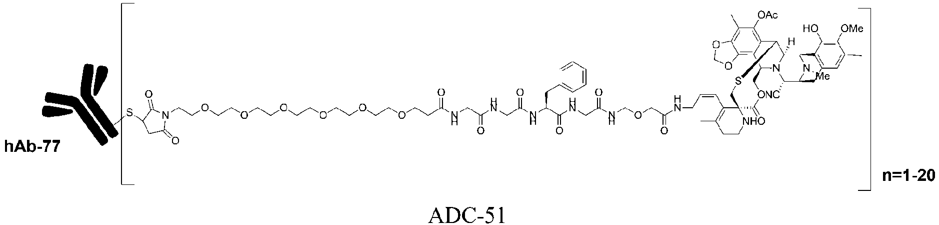

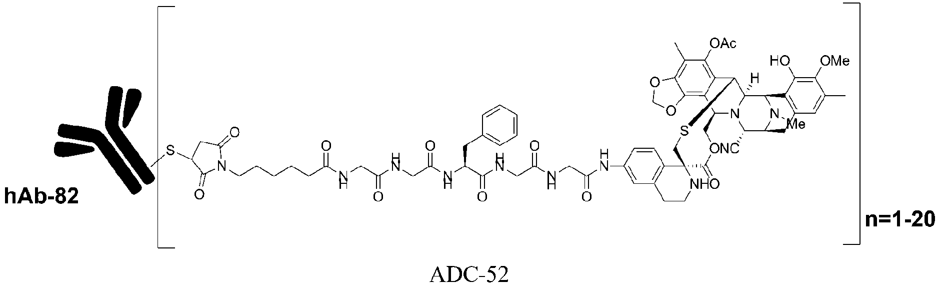

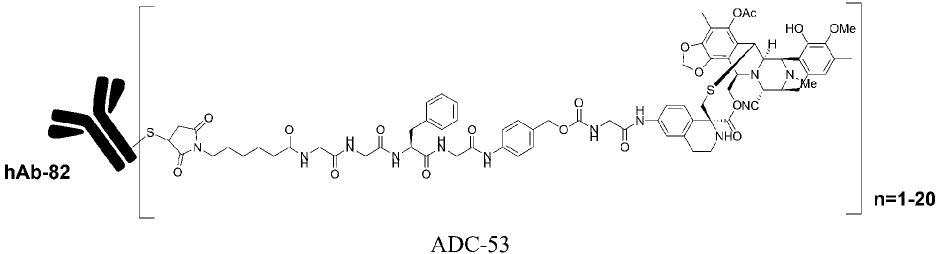

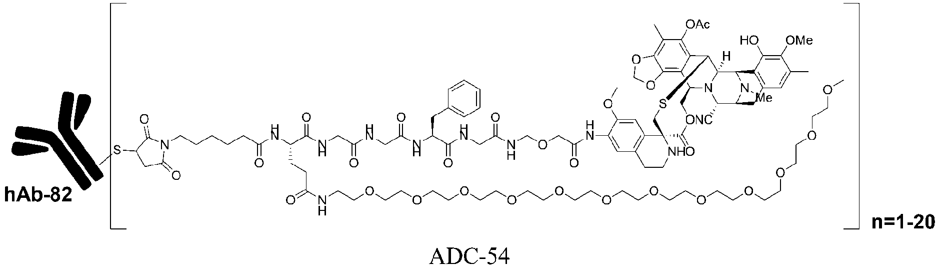

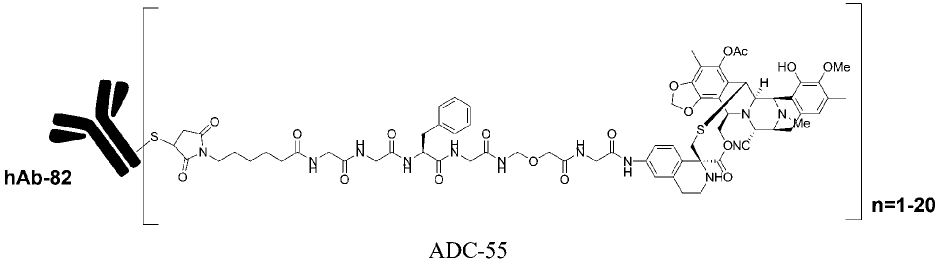

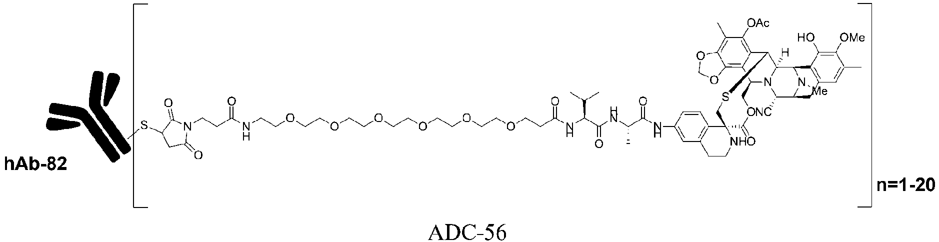

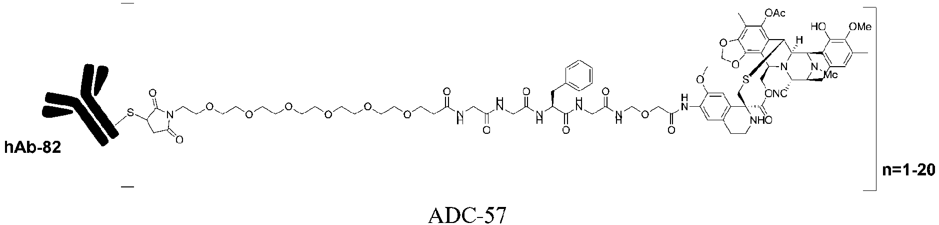

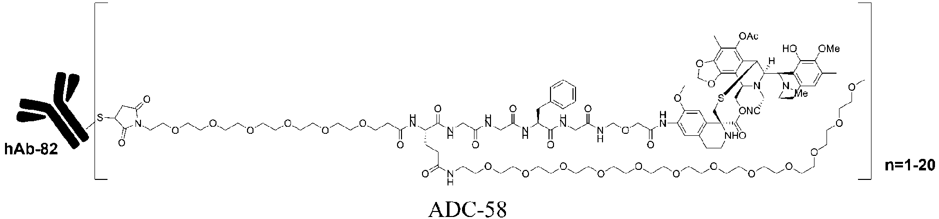

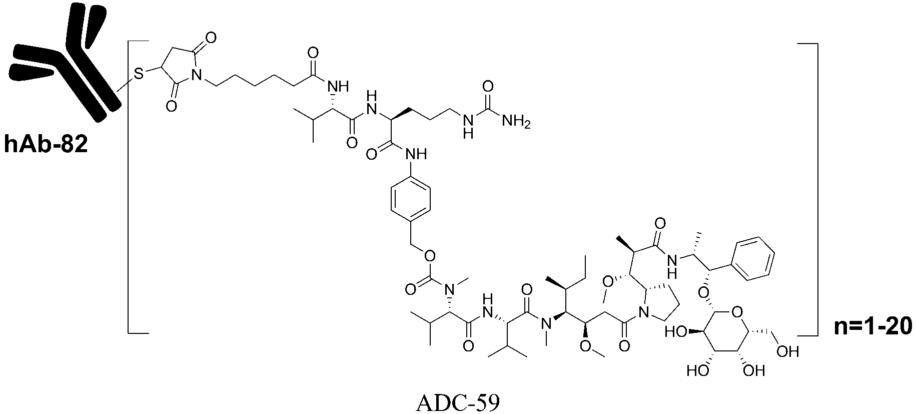

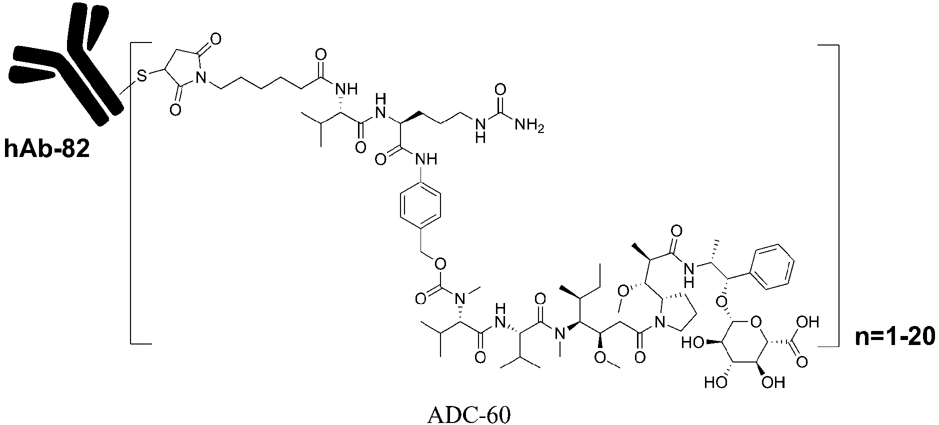

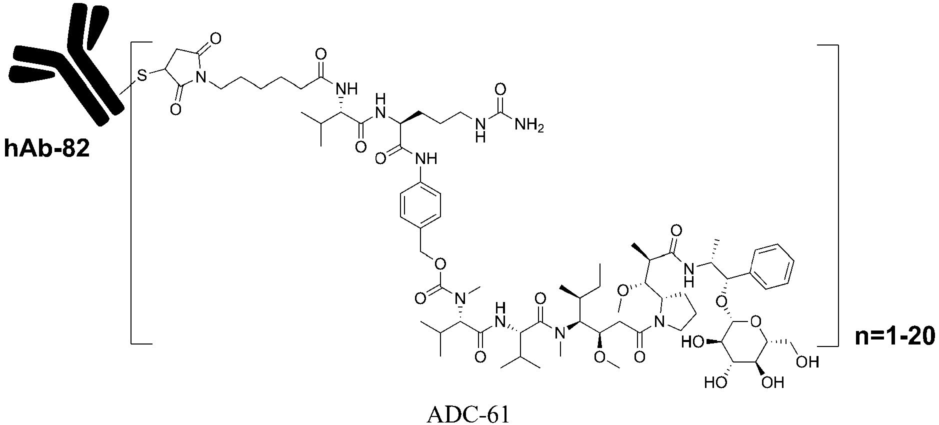

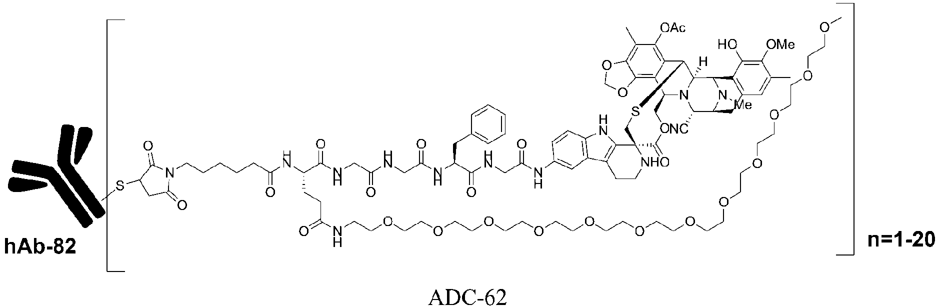

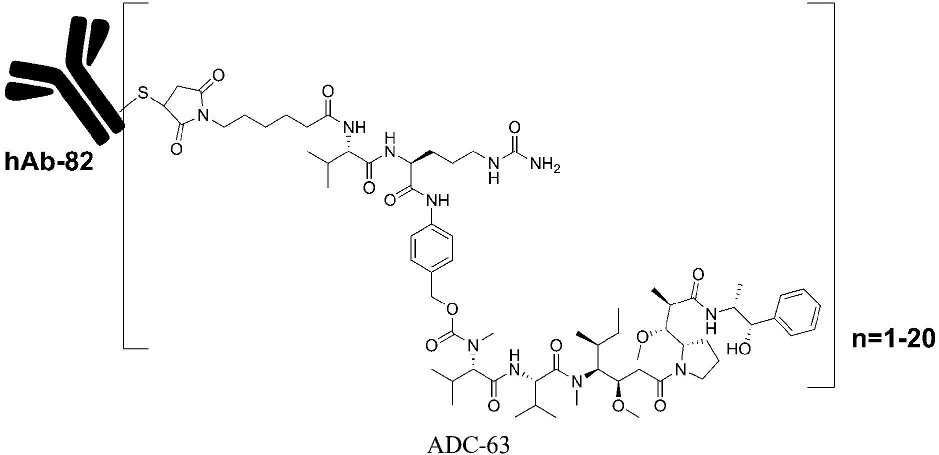

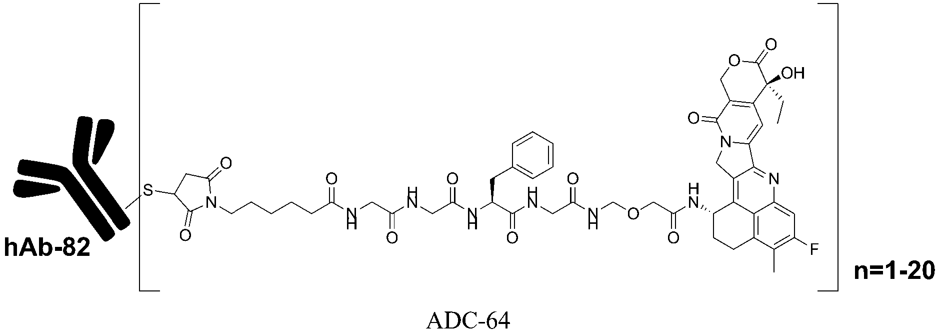

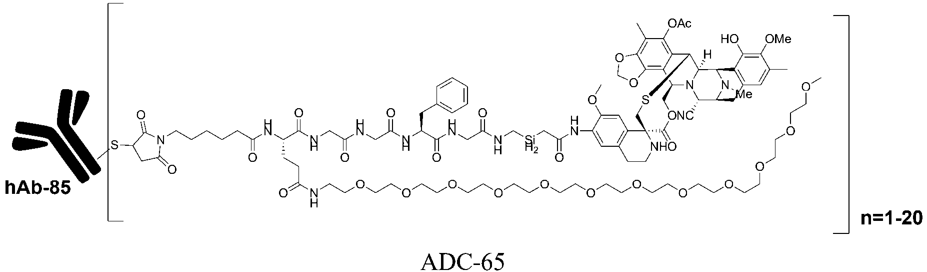

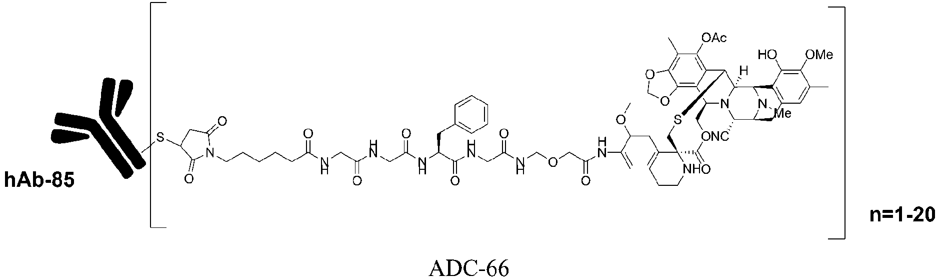

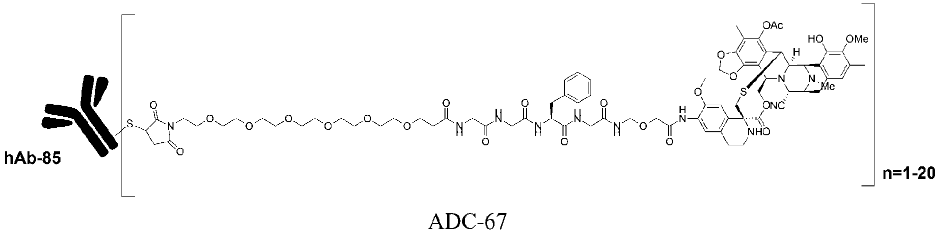

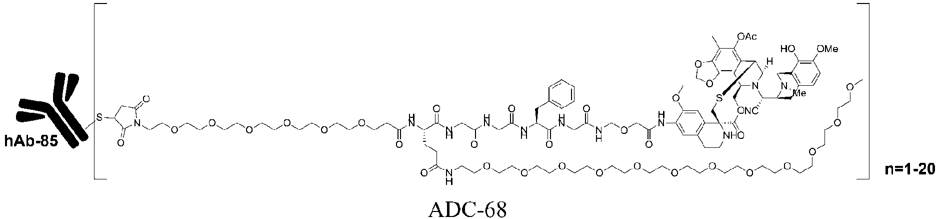

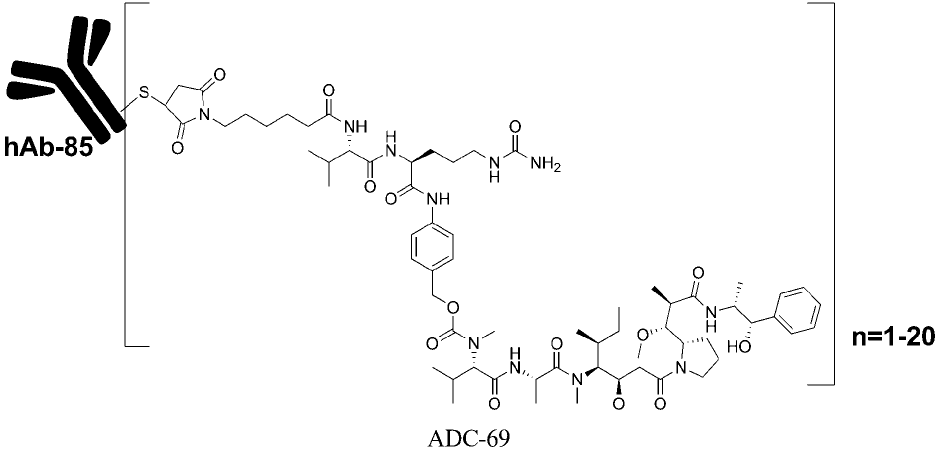

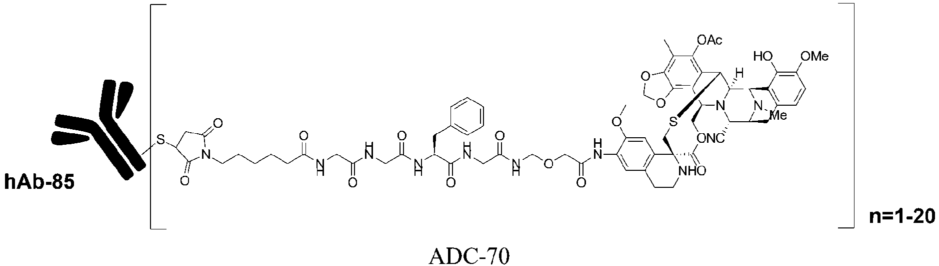

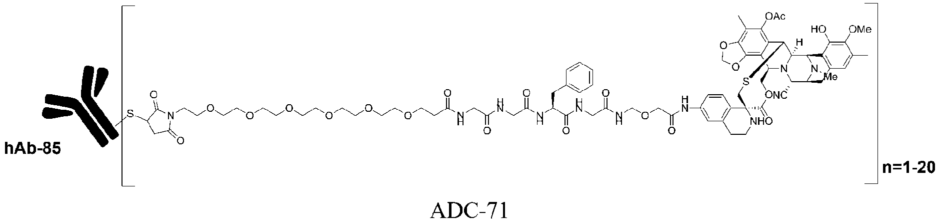

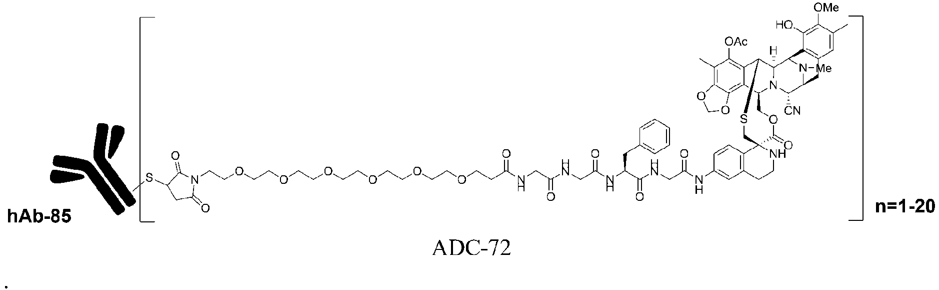

33. Use of the antigen-binding protein according to any one of claims 1 to 22, the nucleic acid molecule according to claim 23, the vector according to claim 24, the cell according to claim 25, the pharmaceutical molecule according to any one of claims 26 to 32 in the manufacture of a medicament for diagnosing, preventing and/or treating hCLDN6-related diseases and/or disorders.

34. The use according to claim 33, **characterized in that** the disease and/or disorder comprises a tumor, and the tumor is ovarian cancer, testicular cancer, non-small cell lung cancer, liver cancer, pancreatic cancer, choriocarcinoma or endometrial cancer.
